(19) Europäisches Patentamt
European Patent Office
Office européen des brevets

(11) **EP 4 372 086 A1**

(12) **EUROPEAN PATENT APPLICATION**
published in accordance with Art. 153(4) EPC


(43) Date of publication:
**22.05.2024 Bulletin 2024/21**

(21) Application number: **22841396.9**

(22) Date of filing: **13.07.2022**

(51) International Patent Classification (IPC):
***C12N 15/113*** $^{(2010.01)}$ ***A61K 48/00*** $^{(2006.01)}$
***A61P 31/00*** $^{(2006.01)}$ ***A61P 9/00*** $^{(2006.01)}$

(52) Cooperative Patent Classification (CPC):
**A61K 48/00; A61P 9/00; A61P 31/00; C12N 15/113**

(86) International application number:
**PCT/CN2022/105340**

(87) International publication number:
**WO 2023/284763 (19.01.2023 Gazette 2023/03)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **16.07.2021 CN 202110807463**



(71) Applicant: **Suzhou Ribo Life Science Co., Ltd.**
**Kunshan, Jiangsu 215300 (CN)**

(72) Inventors:
- **LIANG, Zicai**
  **Kunshan, Jiangsu 215300 (CN)**
- **ZHANG, Hongyan**
  **Kunshan, Jiangsu 215300 (CN)**
- **GAO, Shan**
  **Kunshan, Jiangsu 215300 (CN)**
- **LI, Haitao**
  **Kunshan, Jiangsu 215300 (CN)**

(74) Representative: **SSM Sandmair Patentanwälte Rechtsanwalt Partnerschaft mbB**
**Joseph-Wild-Straße 20**
**81829 München (DE)**


Remarks:
The complete document including Reference Table(s) and the Sequence Listing(s) can be downloaded from the EPO website

(54) **DOUBLE-STRANDED OLIGONUCLEOTIDE, COMPOSITION AND CONJUGATE CONTAINING DOUBLE-STRANDED OLIGONUCLEOTIDE, AND PREPARATION METHODS AND USES**


(57) Provided is a double-stranded oligonucleotide capable of regulating the expression level of a target gene, comprising a sense strand and an antisense strand, wherein the sense strand and the antisense strand each comprises nucleotide sequence I or nucleotide sequence II consisting of 19 modified or unmodified nucleotides, the nucleotide sequence I and the nucleotide sequence II are at least partially reverse complementary to form a double-stranded region, and the nucleotide sequence II is at least partially reverse complementary to a nucleotide sequence in an mRNA expressed by the target gene; and according to the direction from the 5' end to the 3' end, at least one of the 3rd to 6th nucleotides of the nucleotide sequence II is a stabilizing modified nucleotide, and none of the nucleotides other than the 3rd to 9th nucleotides in the nucleotide sequence II are stabilizing modified nucleotides. The provided double-stranded oligonucleotide and pharmaceutical composition and conjugate comprising the double-stranded oligonucleotide may effectively treat and/or prevent diseases or disorders associated with target gene expression, and have reduced off-target effects.


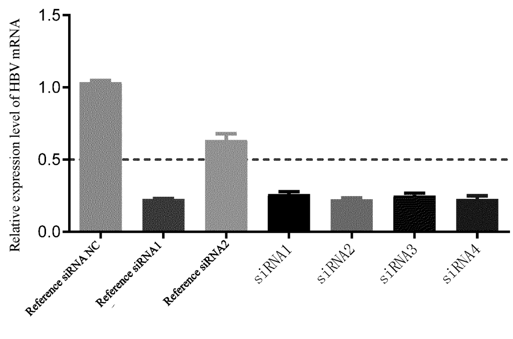


FIG. 1

EP 4 372 086 A1

## Description

## TECHNICAL FIELD

The present disclosure relates to a double-stranded oligonucleotide with reduced off-target effects, and a pharmaceutical composition and an oligonucleotide conjugate containing the double-stranded oligonucleotide. The present disclosure also relates to preparation methods for and uses of these double-stranded oligonucleotides, pharmaceutical compositions and oligonucleotide conjugates.

## BACKGROUND

Double-stranded oligonucleotides are known as pharmaceutical active ingredients. In recent years, considerable progress has been made in the formation of drugs from double-stranded oligonucleotides.

In the pharmaceutical research of double-stranded oligonucleotides, one of the important side effects and even toxicity-related effects is off-target effects. In one aspect, efforts have been made in the art to develop and synthesize double-stranded oligonucleotides with both good pharmaceutical activity and low off-target effects. Further in-depth exploration is still needed in the art to obtain double-stranded oligonucleotides that meet the requirements in these two aspects. In another aspect, double-stranded oligonucleotides that have shown excellent pharmaceutical activity in many preclinical pharmaceutical studies are difficult to use in actual drug development due to the toxicity caused by their off-target effects, so there is still a significant practical need to reduce the off-target effects of double-stranded oligonucleotides.

## SUMMARY

In order to develop a double-stranded oligonucleotide that exhibits reduced off-target effects while having good pharmaceutical activity, and to develop a method for reducing the off-target effects of double-stranded oligonucleotides, the inventors, after repeated studies and experiments, unexpectedly found that double-stranded oligonucleotides with stabilizing modified nucleotides at specific positions in the sequence have, while substantially retaining the pharmaceutical activity, significantly lower off-target effects than double-stranded oligonucleotides which are unmodified at corresponding positions. Therefore, the inventors made the following inventions:

In one aspect, the present disclosure provides a double-stranded oligonucleotide, comprising a sense strand and an antisense strand; the sense strand comprises a nucleotide sequence I, and the antisense strand comprises a nucleotide sequence II; both the nucleotide sequence I and the nucleotide sequence II consist of 19 nucleotides, and each of the nucleotides in the nucleotide sequence I and the nucleotide sequence II is a modified or unmodified nucleotide; the nucleotide sequence I and the nucleotide sequence II are at least partially reversely complementary to form a double-stranded region, and the nucleotide sequence II is at least partially reversely complementary to a first nucleotide sequence segment; the first nucleotide sequence segment is a nucleotide sequence of 19 nucleotides in an mRNA expressed by a target gene; in the direction from the 5’ end to the 3’ end, at least one of the 3rd-6th nucleotides in the nucleotide sequence II is a stabilizing modified nucleotide, and none of the nucleotides other than the 3rd-9th nucleotides in the nucleotide sequence II are stabilizing modified nucleotides; the stabilizing modified nucleotide refers to a nucleotide whose ribose hydroxyl group at the 2’ position is substituted with a stabilizing modification group; compared with a double-stranded oligonucleotide whose nucleotides at the corresponding positions are unmodified nucleotides, the double-stranded oligonucleotide comprising the stabilizing modified nucleotide has increased thermostability, and the steric hindrance of the stabilizing modification group is greater than that of 2’-O-methyl.

In another aspect, the present disclosure provides a double-stranded oligonucleotide, comprising a sense strand and an antisense strand, and each nucleotide of the sense strand and the antisense strand is a modified nucleotide, wherein the sense strand comprises a nucleotide sequence I, and the antisense strand comprises a nucleotide sequence II; both the nucleotide sequence I and the nucleotide sequence II consist of 19 nucleotides; the nucleotide sequence I and the nucleotide sequence II are at least partially reversely complementary to form a double-stranded region, and the nucleotide sequence II is at least partially reversely complementary to a first nucleotide sequence segment; the first nucleotide sequence segment is a nucleotide sequence of 19 nucleotides in an mRNA expressed by a target gene; in the direction from the 5’ end to the 3’ end, the 7th-9th nucleotides in the nucleotide sequence I are fluoro-modified nucleotides, and each of nucleotides at other positions in the nucleotide sequence I is independently one of non-fluoro-modified nucleotides; in the direction from the 5’ end to the 3’ end, the 2nd, 6th, 14th, and 16th nucleotides in the nucleotide sequence II are fluoro-modified nucleotides, and each of nucleotides at other positions in the nucleotide sequence II is independently one of non-fluoro-modified nucleotides; in the direction from the 5’ end to the 3’ end, the 3rd and/or 5th nucleotide(s) in the nucleotide sequence II are/is 2’-O-methoxyethyl-modified nucleotide(s).

In yet another aspect, the present disclosure also provides a pharmaceutical composition, comprising the

double-stranded oligonucleotide provided by the present disclosure and a pharmaceutically acceptable carrier.

In yet another aspect, the present disclosure also provides an oligonucleotide conjugate, comprising the double-stranded oligonucleotide provided by the present disclosure and a conjugated group conjugated to the double-stranded oligonucleotide; the conjugated group comprises a linker and pharmaceutically acceptable targeting groups and/or delivery auxiliary groups, and the double-stranded oligonucleotide, the linker and the targeting groups or the delivery auxiliary groups are sequentially linked covalently or non-covalently; each of the targeting groups is selected from a ligand capable of binding to a cell surface receptor, and each of the delivery auxiliary groups is selected from a group capable of increasing the biocompatibility of the oligonucleotide conjugate in a delivery target organ or tissue.

In yet another aspect, the present disclosure also provides use of the double-stranded oligonucleotide, the pharmaceutical composition and/or the oligonucleotide conjugate of the present disclosure in the preparation of a medicament for treating and/or preventing diseases or symptoms associated with the level of an mRNA expressed by a target gene.

In yet another aspect, the present disclosure also provides a method for treating and/or preventing diseases or symptoms associated with the level of an mRNA expressed by a target gene, the method comprising: administering to a subject in need thereof the double-stranded oligonucleotide, the pharmaceutical composition and/or the oligonucleotide conjugate of the present disclosure.

In yet another aspect, the present disclosure also provides a method for regulating the expression level of a target gene in a cell, the method comprising: contacting an effective amount of the double-stranded oligonucleotide, the pharmaceutical composition and/or the oligonucleotide conjugate of the present disclosure with the cell.

In addition, the present disclosure also provides a kit, comprising the double-stranded oligonucleotide, the pharmaceutical composition and/or the oligonucleotide conjugate of the present disclosure.

**Incorporation by Reference**

All publications, patents, and patent applications mentioned in this specification are incorporated herein by reference to the same extent as if each individual publication, patent, or patent application was specifically and individually indicated to be incorporated herein by reference.

**Beneficial Effects**

The double-stranded oligonucleotide, the pharmaceutical composition and/or the oligonucleotide conjugate of the present disclosure have good stability, high target gene expression regulation activity, and low off-target effects. The details are as follows.

First, the double-stranded oligonucleotide, the pharmaceutical composition and/or the oligonucleotide conjugate of the present disclosure can have lower off-target effects and/or toxicity caused by off-target effects, *in vitro* or *in vivo.* For example, the siRNA provided by the present disclosure has an inhibition rate of no more than 25% against off-target target sequences in an *in vitro* siCHECK system, showing significantly lower off-target effects than a reference siRNA containing no stabilizing modified nucleotides. In another example, in a rat administered the siRNA conjugate of the present disclosure at a dose of 30 mg/kg, the liver weight hardly increased, and the rat showed significantly lower toxicity than a reference siRNA conjugate in terms of hepatic steatosis and inflammation. In another example, the siRNA conjugate provided by the present disclosure has an inhibition rate of no more than 25% against off-target target sequences in the *in vitro* siCHECK system, showing significantly lower off-target effects than a reference siRNA conjugate containing no stabilizing modified nucleotides. In another example, mice administered the siRNA conjugate of the present disclosure at a dose of 30 mg/kg showed similar toxicity to the blank control group in terms of hepatic steatosis, and a significantly lower toxicity than mice administered a reference conjugate containing no stabilizing modified nucleotides in terms of hepatic steatosis. In another example, in mice administered the siRNA conjugate of the present disclosure at a dose of 100 mg/kg, their blood biochemical indicators were significantly reduced, and there was no significant abnormality compared with the blank control group. In addition, compared with the reference siRNA conjugate, the mice administered the siRNA conjugate of the present disclosure did not show moderate or severer inflammatory cell infiltration and necrosis, and showed significantly lower toxicity in histopathology. In another example, even at a high dose of 300 mg/kg, there was no significant difference in serum ALT compared with the blank control group. Compared with the 6 of the mice administered the reference conjugate that showed inflammatory cell infiltration in their histopathological sections, only 3 of the mice administered the conjugate of the present disclosure showed inflammatory cell infiltration: the number of mice showing inflammatory cell infiltration was significantly reduced. In another example, the siRNA conjugate of the present disclosure has low off-target effects. In the *in vitro* siCHECK system, the siRNA conjugate of the present disclosure exhibited excellent on-target inhibition activity against the target sequence, with an $IC_{50}$ value of 4.50 pM-11.3 pM. Meanwhile, the inhibition rate against off-target target sequences in all tested siRNA concentration ranges was less than 50%, indicating low off-target effects. In another example, in mice administered the siRNA conjugate of the present

disclosure weekly at a high dose of 300 mg/kg for three consecutive weeks, their serum ALT and AST concentrations were comparable to those of the blank control group. Further, the pathological sections of the mice administered the siRNA conjugate of the present disclosure also showed similar responses to the blank control group in terms of hepatic steatosis and inflammation: there was no significant abnormality, indicating that the siRNA conjugate of the present disclosure has very low hepatotoxicity. In another example, compared with a reference siRNA conjugate containing no stabilizing modified nucleotides, the siRNA conjugate of the present disclosure not only has comparable or significantly higher inhibition activity against the on-target target sequence, but also showed significantly lower off-target effects, with the ratio of off-target $IC_{25}$/on-target $IC_{25}$ being at least 499, even as high as 2100. In another example, compared with mice administered reference siRNA conjugates, the blood biochemical indicators of the mice administered the siRNA conjugate of the present disclosure at a dose of 100 mg/kg were significantly reduced, and remained close to those of the blank control group. Further, the mice showed significantly lower toxicity in histopathology: no abnormality was found in most of the mice, and only some of them showed mild hepatocyte degeneration.

Second, the double-stranded oligonucleotide, the pharmaceutical composition and/or the oligonucleotide conjugate of the present disclosure exhibited excellent target gene expression regulation activity in *in vitro* cell experiments. For example, when the double-stranded oligonucleotide of the present disclosure is a siRNA, the siRNA provided by the present disclosure has very high target sequence inhibition activity in the *in vitro* siCHECK system, with $IC_{50}$ of 0.0022-0.0086 nM. Meanwhile, the siRNA has a similar target sequence inhibition activity to the reference siRNA containing no stabilizing modified nucleotides. In another example, the siRNAs provided by the present disclosure showed excellent HBV mRNA inhibition activity in the primary hepatocytes of 44Bri mice. At a siRNA concentration of 50 nM, the HBV mRNA inhibition rate was at least 73.92% and could reach up to 77.58%, and the siRNAs showed similar HBV mRNA inhibition activity to the corresponding reference siRNAs containing no stabilizing modified nucleotides. In another example, the siRNA conjugate of the present disclosure exhibited excellent HBV mRNA inhibition activity in the primary hepatocytes of 44Bri mice. At a siRNA concentration of 10 nM, the HBV mRNA inhibition rate was at least 81.29% and could reach up to 93.06%, and the siRNA conjugate showed similar HBV mRNA inhibition activity to the corresponding reference conjugate 4, which contains no stabilizing modified nucleotides. In another example, the siRNA conjugate of the present disclosure exhibited excellent HBV mRNA inhibition activity in the primary hepatocytes of 44Bri mice. At a siRNA concentration of 10 nM, the HBV mRNA inhibition rate was at least 70.14% and could reach up to 85.97%. The siRNA conjugate showed similar HBV mRNA inhibition activity to the corresponding reference siRNA conjugate containing no stabilizing modified nucleotides. Further, compared with the reference siRNA conjugate whose corresponding position is an unmodified nucleotide, the mRNA inhibition activity was surprisingly significantly increased, and the highest increase could reach up to 22.99%. In another example, the siRNA conjugate provided by the present disclosure has very high target sequence inhibition activity in the *in vitro* siCHECK system. At a low concentration of 0.01 nM, the target sequence expression inhibition rate was at least 38.92 nM and could reach up to 67.54%. At a concentration of 0.1 nM, the target sequence expression inhibition rate was at least 84.73% and could reach up to 89.35%. Meanwhile, the siRNA conjugate has similar target sequence inhibition activity to the reference siRNA conjugate containing no stabilizing modified nucleotides. In another example, the siRNA conjugate of the present disclosure has very high target sequence inhibition activity in the *in vitro* siCHECK system, with $IC_{50}$ of 6.89-8.55 pM. Meanwhile, the siRNA conjugate has similar target sequence inhibition activity to the other reference conjugates that have the same sequence but do not contain stabilizing modified nucleotides. In another example, a siRNA conjugate of the present disclosure showed high target sequence inhibition activity in the *in vitro* siCHECK system, with $IC_{50}$ of 49.8 pM. In another example, the siRNA conjugate provided by the present disclosure has very high target sequence inhibition activity in the *in vitro* siCHECK system. At a low concentration of 0.01 nM, the target sequence expression inhibition rate was at least 51.56 nM and could reach up to 58.76%. At a concentration of 0.1 nM, the target sequence expression inhibition rate may be as high as 87.92-88.84%. Meanwhile, the siRNA conjugate has similar target sequence inhibition activity to the reference siRNA conjugate containing no stabilizing modified nucleotides.

Third, the double-stranded oligonucleotide, the pharmaceutical composition and/or the oligonucleotide conjugate of the present disclosure can have higher stability and/or higher activity *in vivo*. For example, when the double-stranded oligonucleotide of the present disclosure is a siRNA, the siRNA conjugate of the present disclosure showed an excellent HBV mRNA inhibition effect in mice. At a dose of 0.1 mg/kg, the HBV mRNA inhibition rate was at least 63.18%. At a dose of 1 mg/kg, the HBV mRNA inhibition rate may even be as high as 96.31%. In addition, the siRNA conjugate showed similar HBV mRNA inhibition activity to the corresponding reference siRNA conjugate containing no stabilizing modified nucleotides. In another example, the siRNA conjugate of the present disclosure showed an excellent HBV mRNA inhibition effect in mice. At a dose of 0.1 mg/kg, the HBV mRNA inhibition rate was at least 44.88%. At a dose of 1 mg/kg, the HBV mRNA inhibition rate may even be as high as 84.25%. In addition, at the same concentration, the siRNA conjugate showed similar HBV mRNA inhibition activity to the corresponding reference siRNA conjugate containing no stabilizing modified nucleotides. In another example, at different time points after administration, the siRNA conjugate of the present disclosure can significantly reduce the levels of TG and CHO in mouse serum, and showed a similar blood lipid level lowering effect to the corresponding reference siRNA conjugate containing no stabilizing modified

nucleotides. In particular, at a dose of 3 mg/kg, the siRNA conjugate of the present disclosure has always shown a very high blood lipid TG lowering effect throughout the administration period of up to 50 days, and the highest inhibition rate can reach up to 90.2%. In another example, at different time points after administration, the siRNA conjugate of the present disclosure can significantly reduce the levels of TG and CHO in mouse serum, and showed a similar blood lipid level lowering effect to the corresponding reference siRNA conjugate containing no stabilizing modified nucleotides. In particular, at doses of 3 mg/kg and 1 mg/kg, the siRNA conjugate of the present disclosure has always shown a very high blood lipid TG lowering effect throughout the administration period of up to 50 days, and the highest inhibition rate can reach up to 92.0%. In another example, at different time points after administration, different concentrations of the siRNA conjugate of the present disclosure can all reduce the TG level in mouse serum. Especially, at a dose of 9 mg/kg, the siRNA conjugate of the present disclosure, after only one administration, can maintain a TG level inhibition rate of greater than 50% for as long as 64 days, and the inhibition rate can reach up to 89.5%, showing an excellent blood lipid inhibition ability. In another example, at different time points after administration, the siRNA conjugate of the present disclosure can significantly reduce the levels of TG and CHO in mouse serum. In addition, throughout the 43 days of the experiment, the inhibition effect was always kept high. Especially, the siRNA conjugates of the present disclosure in a dose of 3 mg/kg all showed excellent blood lipid inhibition effects in mice: the maximum serum TG inhibition rates were all higher than 88%, and the maximum serum CHO inhibition rates were 51.18%-57.41%. In another example, at different time points after administration, the siRNA conjugate of the present disclosure can significantly reduce the levels of TG and CHO in mouse serum, and maintain a high inhibition effect throughout the 22 days of the experiment. In addition, the conjugate showed a similar blood lipid level lowering effect to the corresponding reference siRNA conjugate containing no stabilizing modified nucleotides. In another example, the siRNA conjugate of the present disclosure showed an excellent mRNA inhibition effect on target gene expression in mice. At a dose of 3 mg/kg, the mRNA inhibition rate of target gene expression is at least 70% and could even reach up to 95%. In addition, the siRNA conjugate showed similar or even higher mRNA inhibition activity on target gene expression than the corresponding reference siRNA conjugate containing no stabilizing modified nucleotides.

Therefore, the double-stranded oligonucleotide, the pharmaceutical composition and the oligonucleotide conjugate provided by the present disclosure can have significantly lower off-target effects and toxicity caused by off-target effects, and can also effectively regulate the expression level of a target gene *in vivo* and *in vitro,* and thus can effectively treat and/or prevent symptoms of diseases associated with the level of an mRNA expressed by the target gene while having significantly higher safety, exhibiting good application prospects.

## BRIEF DESCRIPTION OF THE DRAWINGS

FIG. 1 is a bar chart showing the relative expression levels of HBV mRNA in primary hepatocytes of 44Bri mice after transfection with siRNAs of the present disclosure and reference siRNAs.

FIG. 2 is a bar chart showing the relative expression levels of HBV mRNA in primary hepatocytes of 44Bri mice after free intake of siRNA conjugates of the present disclosure or a reference siRNA conjugate and a reference siRNA NC.

FIGs. 3A and 3B are scatter plots showing the relative expression levels of HBV mRNA in the livers of 44Bri mice after administration of different concentrations of siRNA conjugates of the present disclosure or a reference siRNA conjugate and PBS.

FIG. 4 is a bar chart showing the relative expression levels of HBV mRNA in primary hepatocytes of 44Bri mice after free intake of siRNA conjugates of the present disclosure or reference siRNA conjugates and a reference siRNA NC.

FIG. 5 is a scatter plot showing the relative expression levels of HBV mRNA in the livers of 44Bri mice after administration of a siRNA conjugate of the present disclosure or a reference siRNA conjugate and PBS.

FIGs. 6A and 6B are scatter plots showing ALT and AST concentrations in mouse serum after administration of siRNA conjugates of the present disclosure or reference siRNA conjugates and PBS.

FIG. 7 is a bar chart showing the relative expression levels of a target sequence in the *in vitro* siCHECK system after co-transfection with plasmids containing the target sequence and siRNA conjugates or reference siRNA NC.

FIGs. 8A and 8B are line graphs showing changes in the level of serum TG or serum CHO over time after administration of a siRNA conjugate of the present disclosure, a reference siRNA conjugate or PBS.

FIGs. 9A and 9B are line graphs showing changes in the level of serum TG or serum CHO over time after administration of siRNA conjugates of the present disclosure or PBS.

FIG. 10 is a line graph showing changes in the serum TG level over time after administration of different concentrations of a siRNA conjugate of the present disclosure or PBS.

FIGs. 11A and 11B are line graphs showing changes in the level of serum TG or serum CHO over time after administration of siRNA conjugates of the present disclosure or PBS.

FIGs. 12A and 12B are scatter plots showing ALT and AST concentrations in mouse serum after weekly administration of 300 mg/kg of siRNA conjugates of the present disclosure or PBS for three consecutive weeks.

FIGs. 13A and 13B are line graphs showing changes in the level of serum TG or serum CHO over time after administration of siRNA conjugates of the present disclosure, a reference siRNA conjugate or PBS.

FIG. 14 is a bar chart showing the relative expression levels of a target sequence in the *in vitro* siCHECK system after co-transfection with plasmids containing the target sequence and siRNA conjugates or reference siRNA NC.

FIG. 15 is a scatter plot showing the relative expression levels of mANGPTL3 mRNA in the livers of C57BL/6 mice after administration of siRNA conjugates of the present disclosure or a reference siRNA conjugate and PBS.

## DETAILED DESCRIPTION

Specific embodiments of the present disclosure are described in detail below. It will be appreciated that the specific embodiments described herein are intended to illustrate and explain the present disclosure only rather than limit the present disclosure.

In the present disclosure, unless otherwise stated, the HBV gene refers to the viral gene of the hepatitis B virus (HBV), such as the gene with the sequence set forth under Genbank registration number NC_003977.2, and HBV mRNArefers to the mRNAtranscribed from the HBV gene; APOC3 mRNA refers to the mRNA with the sequence set forth under Genbank registration number NM_000040.3; the APOC3 gene refers to the gene that transcribes the above APOC3 mRNA; ANGPTL3 mRNA refers to the mRNA with the sequence set forth under Genbank registration number NM_014495.4; and the ANGPTL3 gene refers to the gene that transcribes the above ANGPTL3 mRNA.

### Definitions

In the text above and below, unless otherwise specified, the uppercase letters C, G, U, and A represent the base composition of nucleotides; the lowercase letter m indicates that the nucleotide adjacent to the letter m on the left side is a methoxy-modified nucleotide; the lowercase letter f indicates that the nucleotide adjacent to the letter f on the left side is a fluoro-modified nucleotide; the lowercase letter s indicates that the two nucleotides adjacent to the letter s on the left and right sides are linked by a phosphorothioate group; P1 indicates that the nucleotide adjacent to the P1 on the right side is a 5'-phosphate nucleotide or 5'-phosphate analog-modified nucleotide. In some embodiments, P1 is VP, Ps or P that indicates a specific modification, wherein the letter combination VP indicates that the nucleotide adjacent to the letter combination VP on the right side is a vinylphosphate (5'-(E)-vinylphosphonate, E-VP)-modified nucleotide, the letter combination Ps indicates that the nucleotide adjacent to the letter combination Ps on the right side is a phosphorothioate-modified nucleotide, and the uppercase letter P indicates that the nucleotide adjacent to the letter P on the right side is a 5'-phosphate nucleotide.

In the text above and below, the "fluoro-modified nucleotide" refers to a nucleotide in which the hydroxy group at the 2' position of the ribosyl group of the nucleotide is substituted with fluorine, and "non-fluoro-modified nucleotide" refers to a nucleotide or a nucleotide analog in which the hydroxy group at the 2' position of the ribosyl group of the nucleotide is substituted with a non-fluorine group. "Nucleotide analog" refers to a group that can replace a nucleotide in a nucleic acid but has a structure different from adenine ribonucleotide, guanine ribonucleotide, cytosine ribonucleotide, uracil ribonucleotide, or thymine deoxyribonucleotide, such as an isonucleotide, a bridged nucleic acid (BNA for short) or an acyclic nucleotide. The "methoxy-modified nucleotide" refers to a nucleotide in which the 2'-hydroxy group of the ribosyl group is substituted with a methoxy group.

In the context of the present disclosure, the expressions "complementary" and "reversely complementary" can

be used interchangeably and have the meaning well known to those skilled in the art, that is, in a double-stranded nucleic acid molecule, the bases of one strand are paired with the bases of the other strand in a complementary manner. In DNA, the purine base adenine (A) is always paired with the pyrimidine base thymine (T) (or uracil (U) in RNA), and the purine base guanine (C) is always paired with the pyrimidine base cytosine (G). Each base pair comprises a purine and a pyrimidine. When adenines of one strand are always paired with thymines (or uracils) of another strand and guanines are always paired with cytosines, the two strands are considered complementary to each other, and the sequences of the strands can be deduced from the sequences of their complementary strands. Accordingly, "mismatch" in the art means that in a double-stranded nucleic acid, the bases in the corresponding positions are not paired in a complementary manner.

In the text above and below, unless otherwise specified, "substantially reversely complementary" means that there are no more than 3 base mismatches between two nucleotide sequence segments involved; "virtually reversely complementary " means that there is no more than one base mismatch between two nucleotide sequence segments; "completely reversely complementary" means that there is no base mismatch between two nucleotide sequence segments.

In the text above and below, particularly in the description of the method for preparing the double-stranded oligonucleotide, the pharmaceutical composition or the oligonucleotide conjugate of the present disclosure, unless otherwise specified, the nucleoside monomer refers to, according to the kind and order of the nucleotides in the double-stranded oligonucleotide or the oligonucleotide conjugate to be prepared, an unmodified or modified nucleoside phosphoramidite monomer used in solid-phase phosphoramidite synthesis (unmodified or modified RNA phosphoramidite; RNA phosphoramidites are also referred to as nucleoside phosphoramidites sometimes). Solid-phase phosphoramidite synthesis is a method used in RNA synthesis well known to those skilled in the art. The nucleoside monomers used in the present disclosure are all commercially available.

Those skilled in the art will appreciate that for any group containing one or more substituents, these groups are not intended to introduce any substitution or substitution pattern that is sterically impractical, synthetically infeasible, and/or inherently unstable.

As used herein, "alkyl" refers to straight and branched chains having a specified number of carbon atoms, typically 1 to 20 carbon atoms, such as 1 to 10 carbon atoms, for example, 1 to 8 or 1 to 6 carbon atoms. For example, $C_1$-$C_6$ alkyl includes straight and branched chain alkyl groups of 1 to 6 carbon atoms. When reference is made to an alkyl residue having a specific number of carbons, all branched and straight chain forms having that number of carbons are intended to be encompassed. Therefore, for example, "butyl" is meant to include n-butyl, sec-butyl, isobutyl and tert-butyl; and "propyl" includes n-propyl and isopropyl. Alkylene is a subset of alkyl and refers to residues which are identical to alkyl but have two points of attachment.

As used herein, "alkenyl" refers to an unsaturated branched or straight chain alkyl group having at least one carbon-carbon double bond obtained by removing a molecule of hydrogen from adjacent carbon atoms of the parent alkyl group. The group may be in the cis or trans configuration of the double bond. Typical alkenyl groups include, but are not limited to: vinyl; propenyl, such as prop-1-en-1-yl, prop-1-en-2-yl, prop-2-en-1-yl (allyl), and prop-2-en-2-yl; and butenyl, such as but-1-en-1-yl, but-1-en-2-yl, 2-methylprop-1-en-1-yl, but-2-en-1-yl, but-2-en-2-yl, but-1,3-dien-1-yl and but-1,3-dien-2-yl. In certain embodiments, alkenyl groups have 2 to 20 carbon atoms, while in other embodiments, alkenyl groups have 2 to 10, 2 to 8, or 2 to 6 carbon atoms. Alkenylene is a subset of alkenyl and refers to residues which are identical to alkenyl but have two points of attachment.

As used herein, "alkynyl" refers to an unsaturated branched or straight chain alkyl group having at least one carbon-carbon triple bond obtained by removing two molecules of hydrogen from adjacent carbon atoms of the parent alkyl group. Typical alkynyl groups include, but are not limited to: ethynyl; propynyl, such as prop-1-yn-1-yl and prop-2-yn-1-yl; and butynyl, such as but-1-yn-1-yl, but-1-yn-3-yl and but-3-yn-1-yl. In certain embodiments, alkynyl groups have 2 to 20 carbon atoms, while in other embodiments, alkynyl groups have 2 to 10, 2 to 8, or 2 to 6 carbon atoms. Alkynylene is a subset of alkynyl and refers to residues which are identical to alkynyl but have two points of attachment.

As used herein, "alkoxy" refers to an alkyl group of a specified number of carbon atoms linked by an oxygen bridge, for example, methoxy, ethoxy, propoxy, isopropoxy, n-butoxy, sec-butoxy, tert-butoxy, pentyloxy, 2-pentyloxy, isopentyloxy, neopentyloxy, hexyloxy, 2-hexyloxy, 3-hexyloxy and 3-methylpentyloxy. An alkoxy group typically has 1 to 10, 1 to 8, 1 to 6, or 1 to 4 carbon atoms linked by an oxygen bridge.

As used herein, "aryl" refers to a group derived from an aromatic monocyclic or polycyclic hydrocarbon ring system by removing a hydrogen atom from a ring carbon atom. The aromatic monocyclic or polycyclic hydrocarbon ring system contains only hydrogen and carbons of 6 to 18 carbon atoms, wherein at least one ring in the ring system is fully unsaturated, i.e., a cyclic delocalized $(4n+2)\pi$-electron system according to the Hückel theory is included. Aryl groups include, but are not limited to, groups such as phenyl, fluorenyl, and naphthyl. Arylene is a subset of aryl and refers to residues which are identical to aryl but have two points of attachment.

"Heteroaryl" refers to a group derived from a 3- to 18-membered aromatic ring and contains 2 to 17 carbon atoms and 1 to 6 heteroatoms selected from nitrogen, oxygen and sulfur. As used herein, a heteroaryl group may be a

monocyclic, bicyclic, tricyclic or tetracyclic ring system, wherein at least one ring in the ring system is fully unsaturated, i.e., a cyclic delocalized (4n+2) π-electron system according to the Hückel theory is included. Heteroaryl groups include fused or bridged ring systems. In some embodiments, the heteroatoms in the heteroaryl group are oxidized heteroatoms. In some embodiments, one or more nitrogen atoms are included in the heteroaryl group. In some embodiments, one or more of the nitrogen atoms in the heteroaryl group are quaternized nitrogen atoms. A heteroaryl group is attached to the rest of the molecule via any ring atom. Examples of heteroaryl groups include, but are not limited to: azacycloheptatriene, acridinyl, benzimidazolyl, benzindolyl, 1,3-benzobisoxazolyl, benzofuranyl, benzooxazolyl, benzo[d]thiazolyl, benzothiadiazolyl, benzo[*b*][1,4]dioxepinyl, benzo[b][1,4]oxazinyl, 1,4-benzodioxanyl, benzonaphthofuranyl, benzooxazolyl, benzodioxolyl, benzodioxinyl, benzopyranyl, benzopyranonyl, benzofuranyl, benzofuranonyl, benzothienyl, benzothieno[3,2-d]pyrimidinyl, benzotriazolyl, benzo[4,6]imidazo[1,2-a]pyridinyl, carbazolyl, cinnolinyl, cyclopenta[d]pyrimidinyl, 6,7-dihydro-5H-cyclopenta[4,5]thieno[2,3-d]pyrimidinyl, 5,6-dihydrobenzo[h]quinazolinyl, 5,6-dihydrobenzo[h]cinnolinyl, 6,7-dihydro-5H-benzo[6,7]cyclohepta[1,2-c]pyridazinyl, dibenzofuranyl, dibenzothienyl, furanyl, furanonyl, furo[3,2-c]pyridinyl, 5,6,7,8,9,10-hexahydrocycloocta[d]pyrimidinyl, 5,6,7,8,9,10-hexahydrocycloocta[d]pyridazinyl, 5,6,7,8,9,10-hexahydrocycloocta[d]pyridinyl, isothiazolyl, imidazolyl, indazolyl, indolyl, isoindolyl, indolinyl, isoindolinyl, isoquinolyl, indolizinyl, isoxazolyl, 5,8-methano-5,6,7,8-tetrahydroquinazolinyl, naphthyridinyl, 1,6-naphthyridinonyl, oxadiazolyl, 2-oxoazepinyl, oxazolyl, oxiranyl, 5,6,6a,7,8,9,10,10a-octahydrobenzo[H]quinazolinyl, 1-phenyl-1*H*-pyrrolyl, phenazinyl, phenothiazinyl, phenoxazinyl, phthalazinyl, pteridinyl, purinyl, pyrrolyl, pyrazolyl, pyrazolo[3,4-d]pyrimidinyl, pyridinyl, pyrido[3,2-d] pyrimidinyl, pyrido[3,4-d]pyrimidinyl, pyrazinyl, pyrimidinyl, pyridazinyl, pyrrolyl, quinazolinyl, quinoxalinyl, quinolinyl, tetrahydroquinolinyl, 5,6,7,8-tetrahydroquinazolinyl, 5,6,7,8-tetrahydrobenzo[4,5]thieno[2,3-d]pyrimidinyl, 6,7,8,9-tetrahydro-5H-cyclohepta[4,5]thieno[2,3-d]pyrimidinyl, 5,6,7,8-tetrahydropyrido[4,5-c]pyridazinyl, thiazolyl, thiadiazolyl, triazolyl, tetrazolyl, triazinyl, thieno[2,3-d]pyrimidinyl, thieno[3,2-d]pyrimidinyl, thieno[2,3-c]pridinyl and thiophenyl/thienyl.

A variety of hydroxy protecting groups may be used in the present disclosure. In general, protecting groups render a chemical functionality insensitive to specific reaction conditions and may be added and removed at that functionality in a molecule without substantially damaging the rest of the molecule. Representative hydroxyl protecting groups are disclosed in Beaucage et al., Tetrahedron 1992, 48, 2223-2311, and Greene and Wuts, Protective Groups in Organic Synthesis, Chapter 2, 2d ed, John Wiley & Sons, New York, 1991, each of which is hereby incorporated by reference in its entirety. In some embodiments, the protecting groups are stable under basic conditions and can be removed under acidic conditions. In some embodiments, non-exclusive examples of hydroxyl protecting groups used herein include dimethoxytrityl (DMT), monomethoxytrityl, 9-phenyloxanthene-9-yl (Pixyl) and 9-(p-methoxyphenyl)xanth-9-yl (Mox). In some embodiments, non-exclusive examples of hydroxyl protecting groups used herein include Tr (trityl), MMTr (4-methoxytrityl), DMTr (4,4'-dimethoxy Trityl) and TMTr (4,4',4"-trimethoxytrityl).

The term "subject", as used herein, refers to any animal, such as a mammal or a marsupial. Subjects of the present disclosure include, but are not limited to, humans, non-human primates (e.g., rhesus or other types of macaques), mice, pigs, horses, donkeys, cows, rabbits, sheep, rats, and any species of poultry.

As used herein, "treatment" refers to means of obtaining a beneficial or desired result, including but not limited to therapeutic benefit. The term "treatment benefit" means eradicating or ameliorating the underlying disorder being treated. Furthermore, therapeutic benefit is obtained by eradicating or ameliorating one or more physiological symptoms associated with the underlying disorder, whereby improvement is observed in the subject, although the subject may still be afflicted by the underlying disorder.

"Prevention" as used herein refers to the means of obtaining a beneficial or desired result, including but not limited to prophylactic benefit. For "prophylactic benefit", a double-stranded oligonucleotide, a pharmaceutical composition, or an oligonucleotide conjugate may be administered to a subject at risk for a particular disease, or a subject with one or more physiological symptoms of a reported disease, even though a diagnosis of the disease may not have been made.

## First double-stranded oligonucleotide

In one aspect, the present disclosure provides a first double-stranded oligonucleotide capable of regulating gene expression and having low off-target effects.

The first double-stranded oligonucleotide of the present disclosure contains a nucleotide group as a basic structural unit. It is well known to those skilled in the art that the nucleotide group contains a phosphate group, a ribosyl group and a base, which are not detailed here.

CN102140458B discloses a siRNA that specifically inhibits HBV genes, and studies various chemical modification strategies of the siRNA. It is found in the study that different modification strategies will have completely different effects on the stability, biological activity and cytotoxicity of the siRNA. In this study, 7 effective modification methods were confirmed. Compared with unmodified siRNA, the siRNA obtained by one of the modification methods improved the blood stability while maintaining an inhibition activity comparable with the unmodified siRNA. However, the issue of

off-target effects was not mentioned in this study.

The double-stranded oligonucleotide of the present disclosure comprises a sense strand and an antisense strand; the sense strand comprises a nucleotide sequence I, and the antisense strand comprises a nucleotide sequence II; both the nucleotide sequence I and the nucleotide sequence II consist of 19 nucleotides, and each of the nucleotides in the nucleotide sequence I and the nucleotide sequence II is a modified or unmodified nucleotide; the nucleotide sequence I and the nucleotide sequence II are at least partially reversely complementary to form a double-stranded region, and the nucleotide sequence II is at least partially reversely complementary to a first nucleotide sequence segment; the first nucleotide sequence segment is a nucleotide sequence of 19 nucleotides in an mRNA expressed by a target gene; in the direction from the 5' end to the 3' end, at least one of the 3rd-6th nucleotides in the nucleotide sequence II is a stabilizing modified nucleotide, and none of the nucleotides other than the 3rd-9th nucleotides in the nucleotide sequence II are stabilizing modified nucleotides; the stabilizing modified nucleotide refers to a nucleotide whose ribose hydroxyl group at the 2' position is substituted with a stabilizing modification group; compared with a double-stranded oligonucleotide whose nucleotides at the corresponding positions are unmodified nucleotides, the double-stranded oligonucleotide comprising the stabilizing modified nucleotide has increased thermostability, and the steric hindrance of the stabilizing modification group is greater than that of 2'-O-methyl.

In some embodiments, in the direction from the 5' end to the 3' end, the 3rd or 5th nucleotide in the nucleotide sequence II is the stabilizing modified nucleotide. In some embodiments, in the direction from the 5' end to the 3' end, no more than 2 nucleotides of the 3rd-9th nucleotides in the nucleotide sequence II are the stabilizing modified nucleotides. By limiting the number of stabilizing modified nucleotides at a specific position, the double-stranded oligonucleotide of the present disclosure may achieve an optimal balance between pharmaceutical activity and low off-target effects, and also has excellent stability. In some embodiments, in the direction from the 5' end to the 3' end, the 3rd and/or 5th nucleotide(s) in the nucleotide sequence II are/is the stabilizing modified nucleotide(s). In some embodiments, in the direction from the 5' end to the 3' end, the 3rd and/or 5th nucleotide(s) in the nucleotide sequence II are/is the stabilizing modified nucleotide(s), and one of the 4th, 7th, and 9th nucleotides is also the stabilizing modified nucleotide.

In some embodiments, in the double-stranded oligonucleotide of the present disclosure, in the direction from the 5' end to the 3' end, the 3rd and 9th nucleotides in the nucleotide sequence II are the stabilizing modified nucleotides; or, in the direction from the 5' end to the 3' end, the 5th and 7th nucleotides in the nucleotide sequence II are the stabilizing modified nucleotides; or, in the direction from the 5' end to the 3' end, the 5th and 9th nucleotides in the nucleotide sequence II are the stabilizing modified nucleotides.

In the double-stranded oligonucleotide of the present disclosure, in the direction from the 5' end to the 3' end, the nucleotides other than the 3rd-9th nucleotides in the nucleotide sequence II are not the stabilizing modified nucleotides. If at least one of the 3rd-6th nucleotides in the nucleotide sequence II is the stabilizing modified nucleotide, and the sequence further comprises a stabilizing modified nucleotide other than the 3rd-9th nucleotides, the ability to regulate the expression level of the target sequence of the double-stranded oligonucleotide may be significantly affected.

In some embodiments, "double-stranded oligonucleotide has increased thermostability" means that the melting temperature Tm of the double-stranded oligonucleotide is increased. In some embodiments, "double-stranded oligonucleotide has increased thermostability" means that the melting temperature Tm of the double-stranded oligonucleotide is increased by at least 0.05 °C. In some embodiments, the expression means an increase of 0.1-6 °C. In some embodiments, the expression means an increase of 0.5-4 °C. Without being limited by theoretical explanation, by including stabilizing modified nucleotides at specific positions, the ability of the antisense strand in the double-stranded oligonucleotide of the present disclosure to bind to the mRNA expressed by the target gene is substantially unaffected, while the binding to an off-target target mRNA is significantly reduced, thereby reducing or even eliminating off-target effects.

In some embodiments, each of the stabilizing modification groups independently has a structure represented by -X-R, wherein X is O, NR', S or $SiR'_2$; R is one of $C_2$-$C_6$ alkyl, substituted $C_2$-$C_6$ alkyl, $C_6$-$C_8$ aryl and substituted $C_6$-$C_8$ aryl; each R' is independently one of H, $C_1$-$C_6$ alkyl, substituted Ci-$C_6$ alkyl, $C_6$-$C_8$ aryl and substituted $C_6$-$C_8$ aryl; the substituted $C_2$-$C_6$ alkyl or substituted $C_6$-$C_8$ aryl refers to a group formed by substituting one or more hydrogen atoms on $C_2$-$C_6$ alkyl or $C_6$-$C_8$ aryl with a substituent, and the substituent is each independently selected from one or more of the following substituents: $C_1$-$C_3$ alkyl, $C_6$-$C_8$ aryl, $C_1$-$C_3$ alkoxy, halogen, oxo and sulfanylidene. It should be noted that the present disclosure is not intended to cover all modified groups conforming to the structure, but only involves those stabilizing modification groups capable of increasing the thermostability of double-stranded oligonucleotides. In some embodiments, each of the stabilizing modification groups is independently selected from one of 2'-O-methoxyethyl, 2'-O-allyl, 2'-C-allyl, 2'-O-2-N-methylamino-2-oxoethyl, 2'-O-2-N,N-dimethylaminoethyl, 2'-O-3-aminopropyl and 2'-O-2,4-dinitrophenyl. In some embodiments, each of the stabilizing modification groups is 2'-O-methoxyethyl.

In some embodiments, in the double-stranded oligonucleotide of the present disclosure, the nucleotide sequence II is substantially reversely complementary, virtually reversely complementary or completely reversely complementary to the first nucleotide sequence segment. In some embodiments, in the direction from the 5' end to the 3' end, the nucleotides at the 2nd-19th positions of the nucleotide sequence II are completely reversely complementary to the nucleotides at the 1st-18th positions of the first nucleotide sequence segment.

In some embodiments, in the double-stranded oligonucleotide of the present disclosure, the nucleotide sequence II is substantially reversely complementary, virtually reversely complementary or completely reversely complementary to the nucleotide sequence I. In some embodiments, the nucleotide sequence II is completely reversely complementary to the nucleotide sequence I, or there is a base mismatch between the 2nd nucleotide in the nucleotide sequence II in the direction from the 5' end to the 3' end and the 2nd nucleotide in the nucleotide sequence I in the direction from the 3' end to the 5' end. By including such base mismatch, the target gene expression regulation activity of the double-stranded oligonucleotide of the present disclosure may be further improved while maintaining a low off-target effect.

In some embodiments, in the double-stranded oligonucleotide of the present disclosure, the sense strand further comprises a nucleotide sequence III, and the antisense strand further comprises a nucleotide sequence IV; each nucleotide in the nucleotide sequence III and the nucleotide sequence IV is independently one of the non-fluoro-modified nucleotides and is not the stabilizing modified nucleotide; the length of the nucleotide sequence III is 1, 2, 3 or 4 nucleotides; the nucleotide sequence IV and the nucleotide sequence III are equal in length, and the nucleotide sequence IV and the nucleotide sequence III are virtually reversely complementary or completely reversely complementary; the nucleotide sequence III is linked to the 5' end of the nucleotide sequence I, and the nucleotide sequence IV is linked to the 3' end of the nucleotide sequence II; the nucleotide sequence IV is virtually reversely complementary or completely reversely complementary to the second nucleotide sequence segment, and the second nucleotide sequence segment refers to a nucleotide sequence that is adjacent to the first nucleotide sequence segment in an mRNA expressed by the target gene and is the same length as the nucleotide sequence IV Thus, the double-stranded oligonucleotide of the present disclosure may have a double-stranded complementary region of 19-23 nucleotides in length.

In some embodiments, the double-stranded oligonucleotide of the present disclosure further comprises a nucleotide sequence V, each nucleoside in the nucleotide sequence V is independently one of the non-fluoro-modified nucleotides and is not the stabilizing modified nucleotide, the length of the nucleotide sequence V is 1 to 3 nucleotides, and the nucleotide sequence V is linked to the 3' end of the antisense strand, thereby constituting the 3' overhang of the antisense strand. In this way, the length ratio of the sense strand to the antisense strand of the double-stranded oligonucleotide provided by the present disclosure may be 19/19, 19/20, 19/21, 19/22, 20/20, 20/21, 20 /22, 20/23, 21/21, 21/22, 21/23, 21/24, 22/22, 22/23, 22/24, 22/25, 23/23, 23/24, 23/25 or 23/26. In some embodiments, the length of the nucleotide sequence V is 2 nucleotides, and in the direction from the 5' end to the 3' end, the nucleotide sequence V is 2 consecutive thymine deoxyribonucleotides (dTdT), 2 consecutive uracil ribonucleotides (LTU), or is completely reversely complementary to a third nucleotide sequence segment; the third nucleotide sequence segment refers to a nucleotide sequence that is adjacent to the first nucleotide sequence segment or the second nucleotide sequence segment in the mRNA expressed by the target gene at the 5' end and is the same length as the nucleotide sequence V. Therefore, in some embodiments, the sense strand and the antisense strand of the double-stranded oligonucleotide of the present disclosure are each 19/21 nucleotides or 21/23 nucleotides in length, and at this time, the double-stranded oligonucleotide of the present disclosure has better target gene expression regulation activity.

As mentioned above, in the double-stranded oligonucleotide of the present disclosure, each nucleotide is a modified or unmodified nucleotide. In the context of the present disclosure, the term "modified nucleotide" as used refers to a nucleotide or nucleotide analog formed by substituting the 2' hydroxyl of the ribosyl group of a nucleotide with other groups, or the base group on the nucleotide is a modified base group. The modified nucleotides will not cause significant weakening or loss of the function of the double-stranded oligonucleotide to regulate gene expression. For example, modified nucleotides disclosed in J. K. Watts et al., Chemically modified siRNA: tools and applications. Drug Discov Today, 2008, 13(19-20):842-55 may be selected. In some embodiments, in the direction from the 5' end to the 3' end, the 2nd, 6th, 14th, and 16th nucleotides in the nucleotide sequence II are 2'-fluoro-modified nucleotides, or the 2nd, 14th, and 16th nucleotides in the nucleotide sequence II are 2'-fluoro-modified nucleotides, and the 6th nucleotide in the nucleotide sequence II is a stabilizing modified nucleotide. In some embodiments, all nucleotides in the nucleotide sequence II are modified nucleotides; in the direction from the 5' end to the 3' end, the 2nd, 6th, 14th, and 16th nucleotides in the nucleotide sequence II are 2'-fluoro-modified nucleotides, and the other nucleotides in the nucleotide sequence II are each independently one of non-fluoro-modified nucleotides. In some embodiments, in the direction from the 5' end to the 3' end, the 7th-9th nucleotides in the nucleotide sequence I are 2'-fluoro-modified nucleotides. In some embodiments, all nucleotides in the nucleotide sequence I are modified nucleotides; in the direction from the 5' end to the 3' end, the 7th-9th nucleotides in the nucleotide sequence I are 2'-fluoro-modified nucleotides, and the other nucleotides in the nucleotide sequence I are each independently one of non-fluoro-modified nucleotides. With the modification, the double-stranded oligonucleotide of the present disclosure may achieve a good balance between gene expression regulation activity and *in vivo* stability.

In the context of the present disclosure, "fluoro-modified nucleotide" refers to a nucleotide formed by substituting the hydroxyl group at the 2' position of the ribosyl group of a nucleotide with fluorine, which has the structure represented by the following formula (7). "Non-fluoro-modified nucleotide" refers to a nucleotide or nucleotide analog formed by substituting the hydroxyl group at the 2' position of the ribosyl group of a nucleotide with a non-fluorine group. In some

embodiments, each of the non-fluoro-modified nucleotides is independently selected from one of a nucleotide or nucleotide analog formed by substituting the hydroxyl group at the 2' position of the ribosyl group of a nucleotide with a non-fluorine group.

Nucleotides in which the hydroxyl at the 2' position of the ribosyl group is substituted with a non-fluorine group are well known to those skilled in the art. These nucleotides may be selected from 2'-alkoxy-modified nucleotides, 2'-alkyl-modified nucleotides, 2'-substituted alkyl-modified nucleotides, 2'-amino-modified nucleotides, 2'-substituted amino-modified nucleotides and 2'-deoxynucleotides.

In some embodiments, the 2'-alkoxy-modified nucleotides are methoxy-modified nucleotides (2'-OMe), as represented by formula (8). In some embodiments, the 2'-amino modified nucleotide (2'-$NH_2$) is represented by formula (9). In some embodiments, the 2'-deoxynucleotide (DNA) is represented by formula (10):

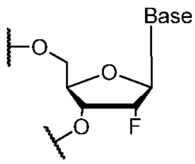 , 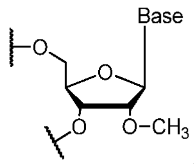 , 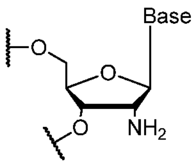 , 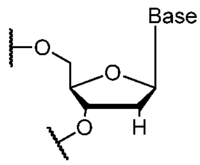 .

formula (7) formula (8) formula (9) formula (10)

The word "nucleotide analog" refers to a group capable of substituting the nucleotide in nucleic acid, but structurally different from adenine ribonucleotides, guanine ribonucleotides, cytosine ribonucleotides, uracil ribonucleotides, or thymus pyrimidine deoxyribonucleotides. In some embodiments, the nucleotide analog may be isonucleotide, bridged nucleic acid (BNA for short) or acyclic nucleotide.

A BNA refers to a constrained or inaccessible nucleotide. A BNA may contain five-membered, six-membered, or seven-membered ring bridge structures with "fixed" C3'-endosugar constrictions. Typically the bridge is incorporated at the 2',4'-position of the ribose to provide a 2',4'-BNA nucleotide. In some embodiments, BNA may be LNA, ENA, cET BNA, etc., wherein LNA is represented by formula (12), ENA is represented by formula (13), and cET BNA is represented by formula (14):

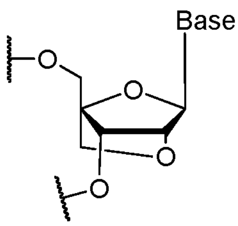 , 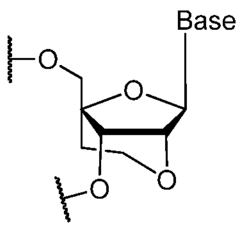 , 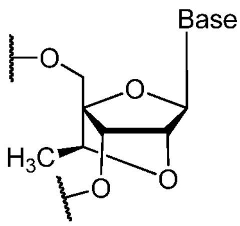 .

formula (12) formula (13) formula (14)

Acyclic nucleotide is a type of nucleotide formed by opening the sugar ring of the nucleotide. In some embodiments, the acyclic nucleotide may be unlocked nucleic acid (UNA) or glycerol nucleic acid (GNA), wherein UNA is represented by formula (15), and GNA is represented by formula (16):

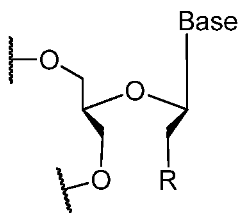 , 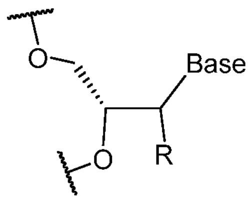 .

formula (15) formula (16)

In formula (15) and formula (16), R is selected from H, OH or alkoxy (O-alkyl).

Isonucleotide refers to a compound formed by changing the position of the base in the nucleotide on the ribose

ring. In some embodiments, the isonucleotide may be a compound formed by moving a base from the 1'-position to the 2'-position or 3'-position of the ribose ring, as represented by formula (17) or (18).

In the compounds of formulas (17)-(18), base represents a nucleic acid base, such as A, U, G, C or T; and R is selected from H, OH, F or non-fluorine groups as described above.

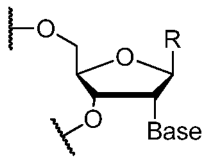

formula (17)

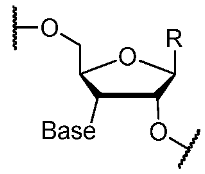

formula (18)

In some embodiments, the nucleotide analog is selected from one of isonucleotides, LNA, ENA, cET, UNA and GNA. In some embodiments, each of the non-fluoro-modified nucleotides is a methoxy-modified nucleotide. In the text above and below, the methoxy-modified nucleotide refers to a nucleotide formed by substituting the 2'-hydroxyl of the ribosyl group with methoxy.

In the text above and below, "fluoro-modified nucleotide", "2'-fluorine-modified nucleotide", "nucleotide in which the 2'-hydroxyl of the ribosyl group is substituted with fluorine" and "nucleotide with 2'-fluororibosyl group" have the same meaning, all of which refer to a compound which is formed by substituting the 2'-hydroxyl of the nucleotide with fluorine and having a structure represented by formula (7). The expressions "methoxy-modified nucleotide", "2'-methoxy-modified nucleotide", "nucleotide in which the 2'-hydroxyl of the ribosyl group is substituted with a methoxy group" and "nucleoside with 2'-methoxyribosyl group" have the same meaning, all of which refer to a compound which is formed by substituting the 2'-hydroxyl of the nucleotide with methoxy group and having a structure represented by formula (8).

In some embodiments, the double-stranded oligonucleotide of the present disclosure is a double-stranded oligonucleotide with the following modifications: in the direction from the 5' end to the 3' end, in the sense strand, the nucleotides at the 7th, 8th, and 9th or 5th, 7th, 8th, and 9th positions of the nucleotide sequence I are fluoro-modified nucleotides, and the nucleotides at the remaining positions in the sense strand are methoxy-modified nucleotides; in the antisense strand, the nucleotides at the 2nd, 6th, 14th, 16th or 2nd, 6th, 8th, 9th, 14th, 16th positions of the nucleotide sequence II are fluoro-modified nucleotides, the nucleotide at the 3rd or 5th position in the antisense strand is a stabilizing modified nucleotide, and the nucleotides at the remaining positions in the antisense strand are methoxy-modified nucleotides.

The double-stranded oligonucleotide with the above modification is not only low in cost, but also makes it difficult for ribonuclease in the blood to cut the double-stranded oligonucleotide, thereby increasing the stability of the double-stranded oligonucleotide and making it have stronger resistance to nuclease hydrolysis. Meanwhile, the modified double-stranded oligonucleotide has a higher target gene expression regulation activity.

In some embodiments, at least one of the phosphate groups in the phosphate-sugar backbone of at least one single strand of the sense strand and the antisense strand of the double-stranded oligonucleotide provided by the present disclosure is a phosphate with a modified group. In some embodiments, the phosphate group with the modified group is a phosphorothioate group formed by substituting at least one oxygen atom in a phosphodiester bond in the phosphate group with a sulfur atom. In some embodiments, the phosphate group with the modified group is a phosphorothioate group with a structure represented by formula (121):

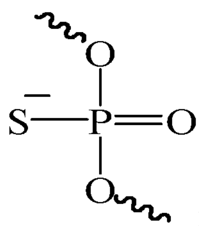

formula (121).

This modification may stabilize the double-stranded structure of double-stranded oligonucleotides and maintain high specificity and high affinity of base pairing.

In some embodiments, in the double-stranded oligonucleotide, the phosphate group having a modified group is present in at least one from the group consisting of the following positions:

between the 1st nucleotide and the 2nd nucleotide at the 5' end of the sense strand;

between the 2nd nucleotide and the 3rd nucleotide at the 5' end of the sense strand;

between the 1st nucleotide and the 2nd nucleotide at the 3' end of the sense strand;

between the 2nd nucleotide and the 3rd nucleotide at the 3' end of the sense strand;

between the 1st nucleotide and the 2nd nucleotide at the 5' end of the antisense strand;

between the 2nd nucleotide and the 3rd nucleotide at the 5' end of the antisense strand;

between the 1st nucleotide and the 2nd nucleotide at the 3' end of the antisense strand; and

between the 2nd nucleotide and the 3rd nucleotide at the 3' end of the antisense strand.

In some embodiments, the 5' end nucleotide of the antisense strand is a 5'-phosphate nucleotide or a 5'-phosphate analog-modified nucleotide. Commonly used 5'-phosphate nucleotides or 5'-phosphate analog-modified nucleotides are well known to those skilled in the art, for example, the 5'-phosphate nucleotides may have the following structure:

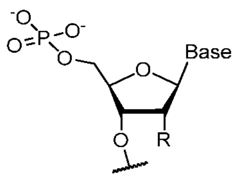
formula (2);

In another example, Anastasia Khvorova and Jonathan K. Watts, The chemical evolution of oligonucleotide therapies of clinical utility. Nature Biotechnology, 2017, 35(3):238-48 discloses the following four 5'-phosphate analog-modified nucleosides:

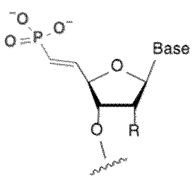
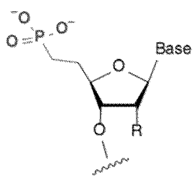
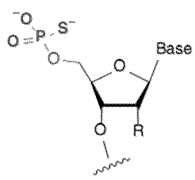
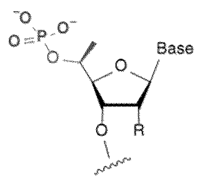

formula (3) formula (4) formula (5) formula (6)

wherein R is selected from H, OH, methoxy, fluorine; and Base represents a nucleic acid base, selected from A, U, C, G or T.

In some embodiments, the 5'-phosphate nucleotide is a nucleotide containing a 5'-phosphate modification represented by formula (2), and the 5'-phosphate analog-modified nucleotide is a vinylphosphate (5'-(E)-vinylphosphonate, E-VP) modified nucleotide as represented by formula (3), or phosphorothioate modified nucleotide as represented by formula (5).

The double-stranded oligonucleotide of the present disclosure may be a variety of double-stranded oligonucleotides that regulate gene expression. In some embodiments, it may be a double-stranded oligonucleotide that inhibits or down-regulates gene expression, such as siRNA. In some embodiments, it may be a double-stranded oligonucleotide that activates or up-regulates gene expression, such as saRNA.

The double-stranded oligonucleotide employing the modification scheme of the present disclosure unexpectedly has increased stability in blood, increased stability in lysosomes, and exhibits excellent target gene expression regulation activity while having low off-target effects.

The modified double-stranded oligonucleotide, pharmaceutical composition and oligonucleotide conjugate provided by the present disclosure may be used to regulate abnormal expression of various genes and treat various pathological conditions or diseases caused by abnormal expression of genes. These genes may be various endogenous

genes in the human body or animals, or genes of pathogens that reproduce in the human body or animals. A double-stranded oligonucleotide with a specific nucleotide sequence and the modification scheme may be designed and prepared according to the mRNA expressed by the target gene. In some embodiments, the mRNA expressed by the target gene is selected from one of the mRNAs transcribed from the following genes: ACE2, AGT, ANGPTL3, ApoA, ApoB, ApoC, AR, ASK1, C3, C5, Col1A1, CTGF, Ebola, FOXO1, FTO, FVII, FXI, FXII, GCGR, HBV, HCV, HSD, p53, PCSK9, PNP, PLG, PKK, KNG, RAGE, RPTOR, SARS-CoV-2, SCD1, SCNN1A, SOD1, STAT3, TIMP -1, TMPRSS6 and XO. In some embodiments, the double-stranded oligonucleotide is siRNA, and the mRNA expressed by the target gene is selected from an mRNA expressed by a hepatitis B virus gene (HBV), an mRNA expressed by an angiopoietin-like protein 3 (ANGPTL3) gene, or an mRNA expressed by an apolipoprotein C3 (ApoC3) gene.

Second double-stranded oligonucleotide

In another aspect, the present disclosure provides a second double-stranded oligonucleotide, comprising a sense strand and an antisense strand, and each nucleotide of the sense strand and the antisense strand is a modified nucleotide, wherein the sense strand comprises a nucleotide sequence I, and the antisense strand comprises a nucleotide sequence II; both the nucleotide sequence I and the nucleotide sequence II consist of 19 nucleotides; the nucleotide sequence I and the nucleotide sequence II are at least partially reversely complementary to form a double-stranded region, and the nucleotide sequence II is at least partially reversely complementary to a first nucleotide sequence segment; the first nucleotide sequence segment is a nucleotide sequence of 19 nucleotides in an mRNA expressed by a target gene; in the direction from the 5’ end to the 3’ end, the 7th-9th nucleotides in the nucleotide sequence I are fluoro-modified nucleotides, and each of nucleotides at other positions in the nucleotide sequence I is independently one of non-fluoro-modified nucleotides; in the direction from the 5’ end to the 3’ end, the 2nd, 6th, 14th, and 16th nucleotides in the nucleotide sequence II are fluoro-modified nucleotides, and each of nucleotides at other positions in the nucleotide sequence II is independently one of non-fluoro-modified nucleotides; in the direction from the 5’ end to the 3’ end, the 3th and/or 5th nucleotide(s) in the nucleotide sequence II are/is 2’-O-methoxyethyl-modified nucleotide(s). In some embodiments, in the direction from the 5’ end to the 3’ end, one of the 4th, 7th and 9th nucleotides in the nucleotide sequence II is a 2’-O-methoxyethyl-modified nucleotide, and the steric hindrance of each of the other non-fluoro-modified substituents in the nucleotide sequence II is no greater than that of 2’-O-methyl.

In some embodiments, in the second double-stranded oligonucleotide of the present disclosure, in the direction from the 5’ end to the 3’ end, the 3rd and/or 5th nucleotide(s) in the nucleotide sequence II have/has a ribose 2’-O-methoxyethyl modification; in the direction from the 5’ end to the 3’ end, the 3rd and 9th nucleotides in the nucleotide sequence II have a ribose 2’-O-methoxyethyl modification; in the direction from the 5’ end to the 3’ end, the 5th and 7th nucleotides in the nucleotide sequence II have a ribose 2’-O-methoxyethyl modification; or, in the direction from the 5’ end to the 3’ end, the 5th and 9th nucleotides in the nucleotide sequence II have a ribose 2’-O-methoxyethyl modification. In some embodiments, none of the other nucleotides in the nucleotide sequence II has a ribose 2’-O-methoxyethyl modification.

The double-stranded oligonucleotides with the modification may exhibit an excellent mRNA level regulation effect on target gene expression while having a low off-target effect.

In some embodiments, in the second double-stranded oligonucleotide of the present disclosure, the nucleotide sequence II is substantially reversely complementary, virtually reversely complementary or completely reversely complementary to the first nucleotide sequence segment. In some embodiments, in the direction from the 5’ end to the 3’ end, the nucleotides at the 2nd-19th positions of the nucleotide sequence II are completely reversely complementary to the nucleotides at the 1st-18th positions of the first nucleotide sequence segment.

In some embodiments, in the second double-stranded oligonucleotide of the present disclosure, the nucleotide sequence II is substantially reversely complementary, virtually reversely complementary or completely reversely complementary to the nucleotide sequence I. In some embodiments, the nucleotide sequence II is completely reversely complementary to the nucleotide sequence I, or there is a base mismatch between the 2nd nucleotide in the nucleotide sequence II in the direction from the 5’ end to the 3’ end and the 2nd nucleotide in the nucleotide sequence I in the direction from the 3’ end to the 5’ end. By including such base mismatch, the target gene expression regulation activity of the double-stranded oligonucleotide of the present disclosure may be further improved while maintaining a low off-target effect.

Further, in the first double-stranded oligonucleotide of the present disclosure, the descriptions about the nucleotide sequence III, 4 and/or 5 as well as the phosphate group with a modified group and/or the 5’ end nucleotide of the antisense strand or the like also apply to the second double-stranded oligonucleotide of the present disclosure.

In some embodiments, the double-stranded oligonucleotide of the present disclosure may be, for example, one of the siRNAs shown in Table 1:

Table 1. siRNA sequences of the present disclosure

| siRNA No. | SEQ ID NO: | Sequence direction 5'- 3' |
|---|---|---|
| siRNAa1 | 1 | CmsCmsUmUmGmAmGfGfCfAmUmAmCmUmUmCmAmAmAm |
| | 2 | UmsUmsTSGmAmAfGmUmAmUmGmCmCmUfCmAfAmGmGmsUmsUm |
| siRNAa2 | 3 | CmsCmsUmUmGmAmGfGfCfAmUmAmCmUmUmCmAmAmAm |
| | 4 | UmsUmsUmGSAmAfGmUmAmUmGmCmCmUfCmAfAmGmGmsUmsUm |
| siRNAa3 | 5 | CmsCmsUmUmGmAmGfGfCfAmUmAmCmUmUmCmAmAmAm |
| | 6 | UmsUmsUmGmASAfGmUmAmUmGmCmCmUfCmAfAmGmGmsUmsUm |
| siRNAa4 | 7 | CmsCmsUmUmGmAmGfGfCfAmUmAmCmUmUmCmAmAmAm |
| | 8 | UmsUmsUmGmAmASGmUmAmUmGmCmCmUfCmAfAmGmGmsUmsUm |
| siRNAa5 | 9 | CmsCmsUmUmGmAmGfGfCfAmUmAmCmUmUmCmAmAmAm |
| | 10 | P1UmsUmsTSGmAmAfGmUmAmUmGmCmCmUfCmAfAmGmGmsUmsUm |
| siRNAa6 | 11 | CmsCmsUmUmGmAmGfGfCfAmUmAmCmUmUmCmAmAmAm |
| | 12 | P1UmsUmsUmGmASAfGmUmAmUmGmCmCmUfCmAfAmGmGmsUmsUm |
| siRNAa7 | 13 | CmsCmsUmUmGmAmGfGfCfAmUmAmCmUmUmCmAmAmAm |
| | 14 | UmsUmsTSGmASAfGmUmAmUmGmCmCmUfCmAfAmGmGmsUmsUm |
| siRNAa8 | 15 | CmsCmsUmUmGmAmGfGfCfAmUmAmCmUmUmCmAmAmAm |
| | 16 | UmsUmsUmGmASAfGSUmAmUmGmCmCmUfCmAfAmGmGmsUmsUm |
| siRNAa9 | 17 | UmsGmsCmUmAmUmGfCfCfUmCmAmUmCmUmUmCmUmAm |
| | 18 | P1UmsAfsGSAmAmGfAmUmGmAmGmGmCmAfUmAfGmCmAmsUmsUm |
| siRNAa10 | 19 | UmsGmsCmUmAmUmGfCfCfUmCmAmUmCmUmUmCmUmAm |
| | 20 | P1UmsAfsGmAmASGfAmUmGmAmGmGmCmAfUmAfGmCmAmsUmsUm |
| siRNAb 11 | 21 | GmsGmsAmCmAmGmUfAfUfUmCmUmCmAmGmUmGmCmUm |
| | 22 | P1AmsGfsCSAmCmUfGmAmGmAmAmUmAmCfUmGfUmCmCmsCmsUm |
| siRNAb 12 | 23 | GmsGmsAmCmAmGmUfAfUfUmCmUmCmAmGmUmGmCmUm |
| | 24 | PlAmsGfsCmAmCSUfGmAmGmAmAmUmAmCfUmGfUmCmCmsCmsUm |
| siRNAb 13 | 25 | UmsUmsAmAmAmAmGfGfGfAmCmAmGmUmAmUmUmCmUm |
| | 26 | P1AmsGfsASAmUmAfCmUmGmUmCmCmCmUfUmUfUmAmAmsGmsCm |
| siRNAb 14 | 27 | UmsUmsAmAmAmAmGfGfGfAmCmAmGmUmAmUmUmCmUm |
| | 28 | P1AmsGfsAmAmTSAfCmUmGmUmCmCmCmUfUmUfUmAmAmsGmsCm |
| siRNAb 15 | 29 | CmsAmsAmUmAmAmAfGfCfUmGmGmAmCmAmAmGmAmAm |
| | 30 | PlUmsUfsCSUmUmGfUmCmCmAmGmCmUmUfUmAfUmUmGmsGmsGm |
| siRNAb16 | 31 | CmsAmsAmUmAmAmAfGfCfUmGmGmAmCmAmAmGmAmAm |
| | 32 | PlUmsUfsCmUmTSGfUmCmCmAmGmCmUmUfUmAfUmUmGmsGmsGm |
| siRNAc17 | 33 | UmsUmsAmAmAmAmGfGfGfAmCmAmGmUmAmUmUmCmUm |
| | 34 | AmsGfsASAmUmAfCmUmGmUmCmCmCmUfUmUfUmAmAmsUmsUm |
| siRNAc18 | 35 | CmsCmsAmAmGmAmGfCfAfCmCmAmAmGmAmAmCmUmAm |
| | 36 | P1UmsAfsGSUmUmCfUmUmGmGmUmGmCmUfCmUfUmGmGmsCmsUm |

(continued)

| siRNA No. | SEQ ID NO: | Sequence direction 5'- 3' |
|---|---|---|
| siRNAc19 | 37 | CmsCmsAmAmGmAmGfCfAfCmCmAmAmGmAmAmCmUmAm |
| | 38 | PlUmsAfsGmUmTSCfUmUmGmGmUmGmCmUfCmUfUmGmGmsCmsUm |
| siRNAc20 | 39 | CmsCmsAmAmGmAmGfCfAfCmCmAmAmGmAmAmCmUmAm |
| | 40 | P1UmsAfsGmTSTSCfUmUmGmGmUmGmCmUfCmUfUmGmGmsCmsUm |
| siRNAc21 | 41 | CmsCmsAmAmGmAmGfCfAfCmCmAmAmGmAmAmCmUmAm |
| | 42 | P1UmsAfsGmTSUmCfUmUmGmGmUmGmCmUfCmUfUmGmGmsCmsUm |
| siRNAa22 | 43 | CmsCmsUmUmGmAmGfGfCfAmUmAmCmUmUmCmAmAmAm |
| | 44 | P1UmsUmsTSGmAmAfGmUmASUmGmCmCmUfCmAfAmGmGmsUmsUm |
| siRNAa23 | 45 | CmsCmsUmUmGmAmGfGfCfAmUmAmCmUmUmCmAmAmAm |
| | 46 | P1UmsUmsUmGmASAfGmUmASUmGmCmCmUfCmAfAmGmGmsUmsUm |

The uppercase letters C, G, U, and A represent the base composition of nucleotides; the lowercase letter m indicates that the nucleotide adjacent to the letter m on the left side is a methoxy-modified nucleotide; the lowercase letter f indicates that the nucleotide adjacent to the letter f on the left side is a fluoro-modified nucleotide; the uppercase letter S underlined indicates that the nucleotide adjacent to the letter S on the left side is a stabilizing modified nucleotide; the lowercase letter s indicates that the two nucleotides on the left and right sides of the letter are linked by a phosphorothioate group; P1 indicates that the nucleotide adjacent to the P1 on the right side is a 5'-phosphate nucleotide or 5'-phosphate analog-modified nucleotide. In some embodiments, S represents a specific stabilizing modification such as moe, wherein the underlined letter combination moe indicates that a nucleotide adjacent to the left side of the letter combination moe has a 2'-O-methoxyethyl-modified nucleotide. In some embodiments, P1 is VP, Ps or P representing a specific modification, wherein the letter combination VP indicates that a nucleotide adjacent to the right side of the letter combination VP is vinylphosphate (5'-(E)- inylphosphonate, E-VP) modified nucleotide, the letter combination Ps indicates that a nucleotide adjacent to the right side of the letter combination Ps is a phosphorothioate modified nucleotide, and the uppercase letter P indicates that a nucleotide adjacent to the right side of the letter P is 5'-phosphate nucleotide. Moreover, each U in the sequence may be substituted with T, and this substitution will not significantly reduce the gene expression regulation activity and/or off-target effect inhibition ability of the double-stranded oligonucleotide.

In the double-stranded oligonucleotide of the present disclosure and the pharmaceutical composition or the oligonucleotide conjugate as described below, adjacent nucleotides are linked by a phosphodiester bond or a phosphorothioate diester bond; non-bridging oxygen atoms or sulfur atoms in the phosphodiester bond or phosphorothioate diester bond have negative charge which may exist in the form of hydroxyl or thiol group. Hydrogen ions in the hydroxyl or thiol group may also be partially or fully substituted by cation. The cation may be any cation, such as one of metal cation, ammonium ion $NH_4^+$, and organic ammonium cation. In consideration of improving solubility, in one embodiment, the cation is selected from one or more of alkali metal ions, ammonium cations formed from tertiary amines, and quaternary ammonium cations. The alkali metal ions may be $K^+$ and/or $Na^+$, and the cations formed from tertiary amines may be ammonium ions formed from triethylamine and/or ammonium ions formed from N,N-diisopropylethylamine. Accordingly, the double-stranded oligonucleotide or oligonucleotide conjugate of the present disclosure may exist at least in part in the form of a salt. In one mode, the non-bridging oxygen atom or sulfur atom in the phosphodiester bond or phosphorothioate diester bond is at least partially bound to a sodium ion, and the double-stranded oligonucleotide or oligonucleotide conjugate of the present disclosure exists in the form of sodium salts or partial sodium salts.

The double-stranded oligonucleotide provided by the present disclosure may be obtained by conventional double-stranded oligonucleotide preparation methods (such as solid-phase synthesis and liquid-phase synthesis methods) in the art. Among them, the solid-phase synthesis has commercialized customized services. The modified nucleotide groups may be introduced into the double-stranded oligonucleotides of the present disclosure by using nucleoside monomers with corresponding modifications. The methods for preparing the nucleoside monomers with corresponding modifications and the method for introducing the modified nucleotide groups into the double-stranded oligonucleotide are also well known to those skilled in the art.

It is clear to those skilled in the art that the modified nucleotide groups may be introduced into the double-stranded oligonucleotide of the present disclosure by using the nucleoside monomers with corresponding modifications. The methods for preparing the nucleoside monomers with corresponding modifications and the method for introducing the modified nucleotide groups into the double-stranded oligonucleotide are also well known to those skilled in the art.

All modified nucleoside monomers are either commercially available or prepared using known methods.

The modified double-stranded oligonucleotide provided by the present disclosure may be used alone, or form a pharmaceutical composition with a pharmaceutically acceptable carrier, or combine with a conjugated molecule to form an oligonucleotide conjugate, or used in other forms. An effective amount of the double-stranded oligonucleotides, the pharmaceutical compositions or the oligonucleotide conjugates are contacted with a cell to regulate the expression of the target gene, or the double-stranded oligonucleotides, the pharmaceutical compositions or conjugates are administered to a subject to regulate the expression of the target gene, thereby achieving the purpose of treating pathological conditions or diseases associated with the expression level of the target gene.

The blood stability of the double-stranded oligonucleotide of the present disclosure may be further improved, the targeting ability thereof may be improved, and the *in vivo* delivery of the double-stranded oligonucleotide of the present disclosure may be solved by forming a pharmaceutical composition with a suitable carrier or by forming an oligonucleotide conjugate with a suitable conjugated molecule. For double-stranded oligonucleotides, carriers or conjugated molecules that may impart or improve targeting will be very beneficial, which will greatly improve the efficiency of the double-stranded oligonucleotides in regulating the expression of the target gene and reduce potential side effects. Furthermore, after the introduction of targeted carriers or conjugated molecules, the double-stranded oligonucleotides also need to be able to function at a target site, that is, the encapsulation/conjugation of the carrier or conjugated molecules cannot affect the activity of the double-stranded oligonucleotide itself (for example, in the case where the double-stranded oligonucleotide is siRNA, the RNAi machinery, i.e. an RISC complex, for loading the siRNA into the cell cannot be affected). In addition, these targeting carriers or conjugated molecules are also required to have good biocompatibility and as low toxicity as possible.

The pharmaceutical composition may be distributed systematically to various parts of a body or be enriched in a specific part of the body with a targeted effect. The conjugates are generally targeted, and the type of the conjugated molecules may be adaptively changed according to the expression distribution of the target gene in the human body or animal, so as to achieve the purpose of delivering the double-stranded oligonucleotide to a relevant site. For example, the conjugated molecule may be a conjugated molecule targeted to liver, lung, kidney or cancer cells.

## Method for reducing off-target effects

In another aspect, the present disclosure also provides a method for reducing the off-target effect of a double-stranded oligonucleotide comprising a sense strand and an antisense strand, wherein the off-target effect refers to an effect of the double-stranded oligonucleotide on regulating the expression level of an mRNA other than the mRNA expressed by a target gene. The method comprises substituting at least one of the 3rd-6th nucleotides in the 5'-3' direction of the antisense strand with the stabilizing modified nucleotide of the present disclosure.

By making such a substitution, the method of the present disclosure may significantly reduce the off-target effects of the double-stranded oligonucleotide. In some embodiments, the method of the present disclosure significantly reduces the off-target effect of the double-stranded oligonucleotide while maintaining the target gene expression regulation ability of the double-stranded oligonucleotide. In some embodiments, the substitution is made to the 3rd or 5th nucleotide in the antisense strand in the direction from the 5' end to the 3' end. In some embodiments, the substitution is made to no more than 2 nucleotides among the 3rd-9th nucleotides in the antisense strand in the direction from the 5' end to the 3' end. By limiting the number of stabilizing modified nucleotides at a specific position, the double-stranded oligonucleotide obtained by the method of the present disclosure may achieve an optimal balance between pharmaceutical activity and low off-target effects. In some embodiments, the substitution is made to the 3rd and/or 5th nucleotide(s) in the antisense strand in the direction from the 5' end to the 3' end. In some embodiments, the substitution is also made on one of the 4th, 7th or 9th nucleotides of the antisense strand.

In the method of the present disclosure, the definitions and selection ranges of the stabilizing modified nucleotides and stabilizing modification groups are as described above.

In some embodiments, the methods of the present disclosure comprise making one of the following substitutions on the antisense strand in the direction from the 5' end to the 3' end:

substituting the 3rd and/or 5th nucleotide(s) with stabilizing modified nucleotide(s);

substituting the 3rd and 9th nucleotides with stabilizing modified nucleotides;

substituting the 5th and 7th nucleotides with stabilizing modified nucleotides; or

substituting the 5th and 9th nucleotides with stabilizing modified nucleotides.

In some embodiments, the substitution is not made to the nucleotides other than the 3rd-9th nucleotides in the

antisense strand in the direction from the 5' end to the 3' end.

In some embodiments, the method of the present disclosure further comprises substituting the nucleotide in the sense strand of the double-stranded oligonucleotide corresponding to the position of the 2nd nucleotide in the direction from the 5' end to the 3' end in the antisense strand with a nucleotide mismatched with the 2nd nucleotide in the antisense strand. At this time, the double-stranded oligonucleotide obtained according to the method of the present disclosure shows further improved target gene expression regulation activity while having a reduced off-target effect.

Pharmaceutical composition

In another aspect, the present disclosure provides a pharmaceutical composition, comprising the double-stranded oligonucleotide provided by the present disclosure or the double-stranded oligonucleotide obtained by the method of the present disclosure, and a pharmaceutically acceptable carrier.

The pharmaceutically acceptable carrier may be a carrier routinely used in the field of double-stranded oligonucleotide administration, such as but not limited to magnetic nanoparticles (such as nanoparticles based on $Fe_3O_4$ or $Fe_2O_3$), carbon nanotubes, mesoporous silicon, calcium phosphate nanoparticles, polyethyleneimine (PEI), polyamidoamine (PAMAM) dendrimer, poly(L-lysine) (PLL), chitosan, 1,2-dioleoyl-3-trimethylammonium-propane (DOTAP), poly(D&L-lactic/glycolic acid)copolymer (PLGA), poly(2-aminoethylethylene phosphate) (PPEEA) and poly(2-dimethylaminoethyl methacrylate) (PDMAEMA) as well as derivatives thereof.

In some embodiments, in the pharmaceutical composition, there is no special requirement on the content of the double-stranded oligonucleotides and the pharmaceutically acceptable carriers. In some embodiments, the weight ratio of the double-stranded oligonucleotide to the pharmaceutically acceptable carrier may be 1:(1-500), and in some embodiments, the weight ratio is 1:(1-50).

In some embodiments, the pharmaceutical composition may further comprise other pharmaceutically acceptable excipients, which may be one or more of various preparations or compounds routinely used in the art. For example, the other pharmaceutically acceptable excipients may include at least one of a pH buffer, a protective agent and an osmotic pressure regulator.

The pH buffer may be a Tris hydrochloride buffer with a pH value of 7.5-8.5 and/or a phosphate buffer with a pH value of 5.5-8.5, for example, a phosphate buffer with a pH value of 5.5-8.5.

The protective agent may be at least one of inositol, sorbitol, sucrose, trehalose, mannose, maltose, lactose and glucose. Based on the total weight of the pharmaceutical composition, the content of the protective agent may be 0.01-30 wt%.

The osmotic pressure regulator may be sodium chloride and/or potassium chloride. The content of the osmotic pressure regulator makes the osmotic pressure of the pharmaceutical composition 200-700 milliosmol/kg (mOsm/kg). According to the desired osmotic pressure, those skilled in the art may easily determine the content of the osmotic pressure regulator. In some embodiments, the dose of the preparation made from the pharmaceutical composition during administration will be adjusted due to different administration methods.

In some embodiments, the pharmaceutical composition may be a liquid preparation, such as an injection; it may also be a freeze-dried powder injection, which is mixed with a liquid excipient during administration to prepare a liquid preparation. The liquid preparation may be used for subcutaneous, intramuscular or intravenous injection administration, but may also be administered to the lungs by spraying, or administered to other organs (such as the liver) through the lungs by spraying, or deliver the pharmaceutical composition through oropharyngeal inhalation or nasal administration. In some embodiments, the pharmaceutical composition is for spray administration.

In some embodiments, the pharmaceutical composition may be in the form of a liposomal formulation. In some embodiments, the pharmaceutically acceptable carrier used in the liposome formulation comprises an amine-containing transfection compound (hereinafter also referred to as an organic amine), a helper lipid and/or a PEGylated lipid. The organic amine, helper lipid and pegylated lipid may be selected from one or more of the amine-containing transfection compounds described in Chinese patent application CN103380113A (which is incorporated herein by reference in its entirety) or pharmaceutically acceptable salts or derivatives, helper lipids and PEGylated lipids thereof.

In some embodiments, the organic amine may be a compound represented by formula (201) as described in Chinese patent application CN103380113A or a pharmaceutically acceptable salt thereof:

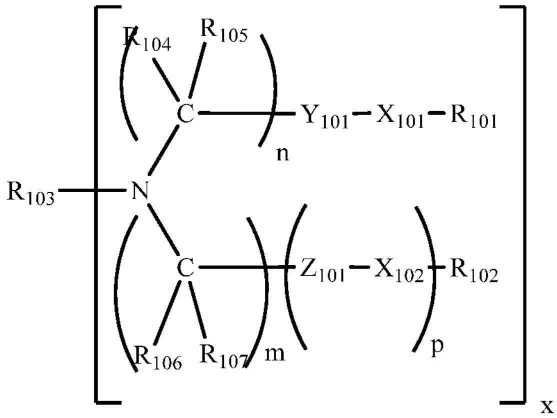

formula (201),

wherein:

$X_{101}$ and $X_{102}$ are each independently O, S, N-A or C-A, wherein A is hydrogen or a $C_1$-$C_{20}$ hydrocarbon chain;

$Y_{101}$ and $Z_{101}$ are each independently C=O, C=S, S=O, CH-OH or $SO_2$;

$R_{101}$, $R_{102}$, $R_{103}$, $R_{104}$, $R_{105}$, $R_{106}$ and $R_{107}$ are each independently hydrogen, cyclic or acyclic, substituted or unsubstituted, branched or straight-chain aliphatic group, cyclic or acyclic substituted or unsubstituted, branched or straight-chain heteroaliphatic, substituted or unsubstituted, branched or straight-chain acyl, substituted or unsubstituted, branched or straight-chain aryl, substituted or unsubstituted, branched or straight-chain heteroaryl;

x is an integer of 1 to 10;

n is an integer of 1-3, m is an integer of 0-20, p is 0 or 1; wherein if m = p = 0, $R_{102}$ is hydrogen;

and, if at least one of n or m is 2, $R_{103}$ and nitrogen in formula (201) form a structure as represented by formula (202) or formula (203):

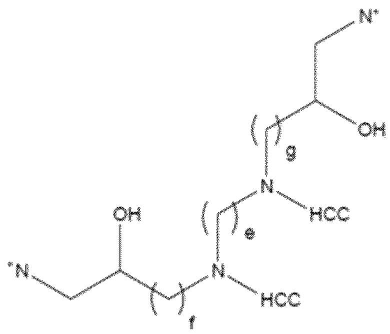

formula (202),

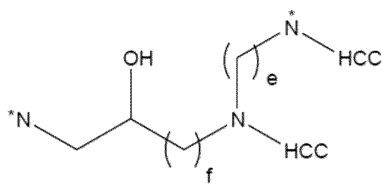

formula (203);

wherein g, e, and f are each independently an integer of 1-6, "HCC" represents a hydrocarbon chain, and each *N represents a nitrogen atom in formula (201).

In some embodiments, $R_{103}$ is a polyamine. In other embodiments, $R_{103}$ is a ketal. In some embodiments, each of $R_{101}$ and $R_{102}$ in formula (201) is independently any substituted or unsubstituted, branched or straight-chain alkyl or alkenyl, the alkyl or alkenyl group has 3 to about 20 carbon atoms, for example, 8 to about 18 carbon atoms, and 0 to 4 double bonds, for example, 0 to 2 double bonds.

In some embodiments, if each of n and m independently has a value of 1 or 3, $R_{103}$ may be any of formulas (204)-(213):

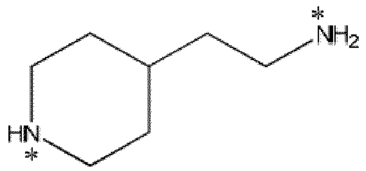 formula (204), 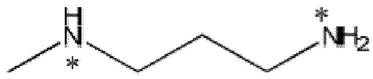 formula (205),

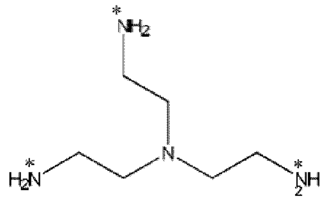 formula (206), 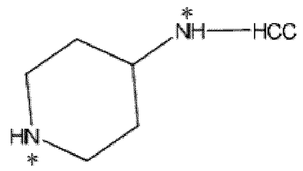 formula (207),

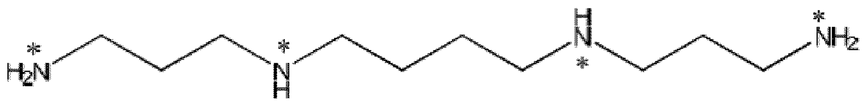 formula (208),

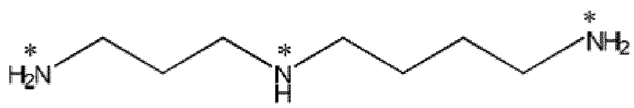 formula (209),

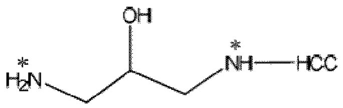 formula (210), 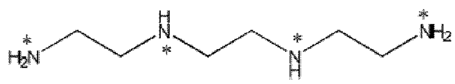 formula (211),

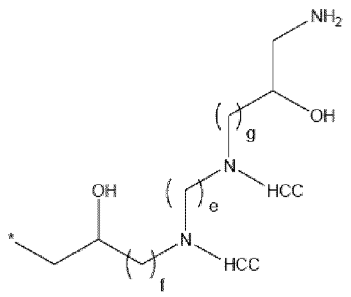 formula (212) and 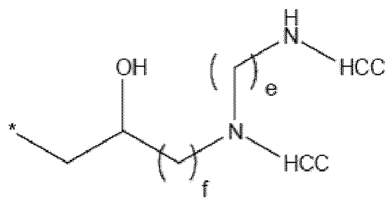 formula (213);

wherein in formula (204)-formula (213), g, e and f are each independently an integer of 1-6, each "HCC" represents a hydrocarbon chain, and each * shows possible points of attachment of $R_{103}$ to the nitrogen atom in formula (201), wherein each H at any * position may be substituted to achieve linkage to the nitrogen atom in formula (201).

Those skilled in the art may obtain the compound represented by formula (201) by any reasonable method. In some embodiments, the compound represented by formula (201) may be prepared according to the description in Chinese patent application CN103380113A.

In some embodiments, the organic amine is an organic amine represented by formula (214) and/or an organic amine represented by formula (215):

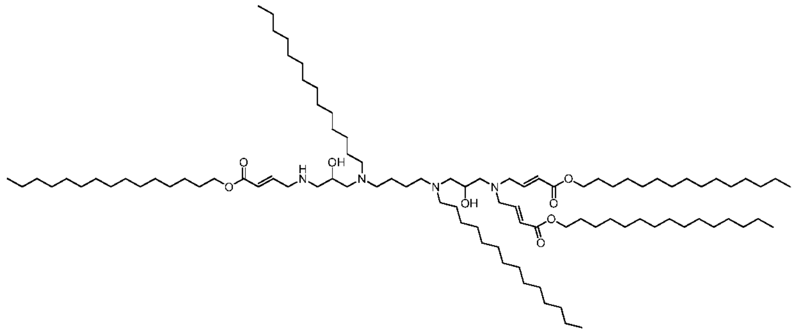

formula (214),

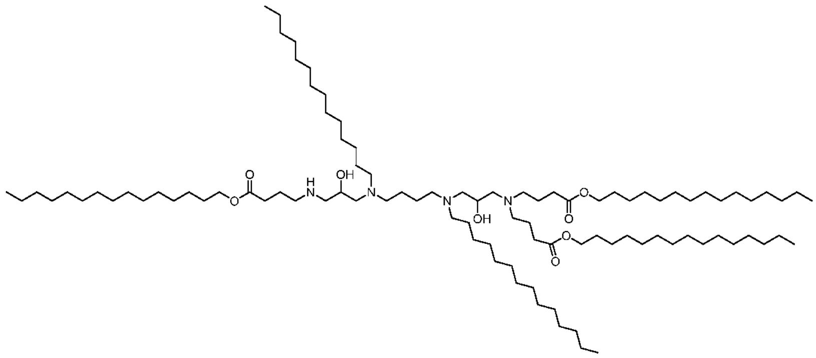

formula (215);

The helper lipid is cholesterol, a cholesterol analog and/or a cholesterol derivative;
the PEGylated lipid is 1,2-dipalmitoyl-sn-glycerol-3-phosphatidylethanolamine-N-[methoxyl (polyethylene glycol)]-2000.

In some embodiments, in the pharmaceutical composition, the molar ratio among the organic amine, the helper lipid and the PEGylated lipid is (19.7-80):(19.7-80):(0.3-50), for example, (50-70):(20-40): (3-20).

In some embodiments, the particles of the pharmaceutical composition formed from the double-stranded oligonucleotide of the present disclosure and the amine-containing transfection reagents described above have an average diameter of about 30 nm to about 200 nm, typically about 40 nm to about 135 nm, more typically, the average diameter of the liposome particles is about 50nm to about 120nm, about 50nm to about 100nm, about 60nm to about 90nm or about 70nm to about 90nm, for example, the average diameter of the liposome particles is about 30, 40, 50, 60, 70, 75, 80, 85, 90, 100, 110, 120, 130, 140, 150 or 160nm.

In some embodiments, in the pharmaceutical composition formed by the double-stranded oligonucleotide of the present disclosure and the amine-containing transfection reagent, the weight ratio of the double-stranded oligonucleotide to all lipids (such as the organic amines, helper lipids and/or PEGylated lipids) have a weight ratio (weight/weight ratio) from about 1:1 to about 1:50, from about 1:1 to about 1:30, from about 1:3 to about 1:20, from about 1:4 to about 1:18, from about 1:5 to about 1:17, from about 1:5 to about 1:15, from about 1:5 to about 1:12, from about 1:6 to about 1:12 or from about 1:6 to about 1:10, for example, the weight ratio of the double-stranded oligonucleotide of the present disclosure to the total lipid is about 1:5, 1:6, 1:7. 1:8, 1:9, 1:10, 1:11, 1:12, 1:13, 1:14, 1:15, 1:16, 1:17, or 1:18.

In some embodiments, each component of the pharmaceutical composition may exist independently when sold, and may exist in the form of a liquid formulation when used. In some embodiments, the pharmaceutical composition formed by the double-stranded oligonucleotide provided by the present disclosure and the pharmaceutically acceptable carrier may be prepared according to various known methods, and may be formed by substituting the existing double-stranded oligonucleotide with the double-stranded oligonucleotide provided by the present disclosure. In some embodiments, the composition may be prepared as follows:
the organic amine, helper lipid and PEGylated lipid are suspended in the alcohol according to the molar ratio and mixed to obtain a lipid solution, wherein the amount of alcohol is such that the total mass concentration of the obtained lipid solution is 2-25 mg/mL, for example, it may be 8-18 mg/mL. The alcohol is selected from pharmaceutically acceptable alcohols, such as alcohols that are liquid at about room temperature, for example, one or more of ethanol, propylene glycol, benzyl alcohol, glycerin, polyethylene glycol 200, polyethylene glycol 300, polyethylene glycol 400, for example, it may be ethanol.

The double-stranded oligonucleotide provided by the present disclosure is dissolved in a buffered saline solution to obtain a double-stranded oligonucleotide aqueous solution. The concentration of the buffered saline solution is 0.05-0.5 M, for example, 0.1-0.2 M; the pH of the buffered saline solution is adjusted to 4.0-5.5, for example, 5.0-5.2; and the amount of the buffered saline solution makes the concentration of the double-stranded oligonucleotide not exceed 0.6

mg/mL, for example, 0.2-0.4 mg/mL. The buffer salt is selected from one or more of soluble acetate and soluble citrate, for example, sodium acetate and/or potassium acetate.

The lipid solution and the double-stranded oligonucleotide aqueous solution are mixed, and the mixed product is incubated at 40-60 °C for at least 2 min, for example, 5-30 min, to obtain an incubated liposome preparation. The volume ratio of the lipid solution and the double-stranded oligonucleotide aqueous solution is 1:(2-5), for example, 1:4.

The incubated liposome preparation is concentrated or diluted, impurities are removed, and the preparation is sterilized to obtain the pharmaceutical composition provided by the present disclosure, whose physical and chemical parameters are pH of 6.5-8, encapsulation efficiency of not less than 80%, particle size of 40-200 nm, polydispersity index of not higher than 0.30, and osmotic pressure of 250-400 mOsm/kg. For example, the physical and chemical parameters may be pH of 7.2-7.6, encapsulation efficiency of not less than 90%, particle size of 60-100nm, polydispersity index of not higher than 0.20, and osmotic pressure of 300-400 mOsm/kg.

The concentration or dilution may be performed before, after or simultaneously with the removal of impurities. Various existing methods may be used to remove impurities, for example, a tangential flow system and a hollow fiber column may be used for ultrafiltration at 100K Da, wherein the ultrafiltration exchange solution is phosphate buffered saline (PBS) with pH of 7.4. Various existing methods may be used for the sterilization, for example, filtration sterilization on a 0.22 μm filter may be used.

Oligonucleotide conjugate

In another aspect, the present disclosure provides an oligonucleotide conjugate, comprising the double-stranded oligonucleotide provided by the present disclosure or the double-stranded oligonucleotide obtained by the method of the present disclosure, and a conjugated group that is conjugated to the double-stranded oligonucleotide. In some embodiments, the conjugated group contains a linker and a pharmaceutically acceptable targeting group and/or a delivery auxiliary group, and the double-stranded oligonucleotide, the linker and the targeting groups or the delivery auxiliary group are sequentially linked covalently or non-covalently; each of the targeting groups is selected from ligands capable of binding to a cell surface receptor, and each delivery auxiliary group is selected from groups capable of increasing the biocompatibility of the oligonucleotide conjugate in a delivery target organ or tissue.

In the context of the present disclosure, unless otherwise stated, "conjugation" means that two or more chemical moieties each having a specific function are covalently linked to each other; accordingly, "conjugate" refers to a compound formed by covalent linkage between the various chemical moieties. Further, "oligonucleotide conjugate" refers to a compound formed by covalently linking one or more chemical moieties with specific functions to an oligonucleotide. The oligonucleotide conjugate should be understood as a general term of multiple oligonucleotide conjugates or an oligonucleotide conjugate represented by a certain chemical formula according to the context. In the context of the present disclosure, "conjugate molecule" should be understood as a specific compound that may be conjugated to an oligonucleotide through a reaction, ultimately forming an oligonucleotide conjugate of the present disclosure.

Generally, the conjugated group comprises at least one targeting group that is pharmaceutically acceptable and optionally a linker, and the double-stranded oligonucleotide, the linker and the targeting group are linked sequentially. In one embodiment, there are 1-6 targeting groups. In one embodiment, there are 2-4 targeting groups. The double-stranded oligonucleotide molecule A1 may be non-covalently or covalently conjugated to the conjugated group, for example may be covalently conjugated to the conjugated group. The conjugation site of the double-stranded oligonucleotide and the conjugated group may be at the 3' or 5' end of the sense strand of the double-stranded oligonucleotide, or at the 5' end of the antisense strand, or in the internal sequence of the double-stranded oligonucleotide. In some specific embodiments, the conjugation site between the double-stranded oligonucleotide and the conjugated group is at the 3' end of the sense strand of the double-stranded oligonucleotide.

In some embodiments, the conjugated group may be linked to the phosphate group, the 2'-position hydroxyl or the base of the nucleotide. In some embodiments, the conjugated group may be linked to the 3'-position hydroxyl, and at this time, the nucleotides are linked by a 2'-5' phosphodiester bond. When the conjugated group is linked to an end of the double-stranded oligonucleotide chain, the conjugation group is usually linked to the phosphate group of the nucleotide. When the conjugated group is linked to the internal sequence of the double-stranded oligonucleotide, the conjugated group is usually linked to the ribose sugar ring or the base. For various linkage methods, please refer to Muthiah Manoharan *et. al.* siRNA conjugates carrying sequentially assembled trivalent N-acetylgalactosamine linked through nucleotides elicit robust gene silencing *in vivo* in hepatocytes. ACS Chemical biology, 2015, 10(5):1181-7.

In some embodiments, the double-stranded oligonucleotide and the conjugated group may be linked by acid-labile or reducible chemical bonds, and these chemical bonds may be degraded in the acidic environment of the endosome of the cell, so that the double-stranded oligonucleotide becomes free. For non-degradable conjugation, the conjugated group may be linked to the sense strand of the dsoligonucleotide, thereby minimizing the effect of conjugation on the activity of the double-stranded oligonucleotide.

The targeting group may be linked to the double-stranded oligonucleotide molecule through a suitable linker,

and those skilled in the art may select a suitable linker according to the specific type of the targeting group. The types of these linkers, targeting groups and the linkage methods with the double-stranded oligonucleotides may be found in the disclosure of WO2015006740A2, the entire contents of which are incorporated herein by reference.

In some embodiments, the targeting group may be a ligand routinely used in the field of double-stranded oligonucleotide administration, such as various ligands described in WO2009082607A2, the entire disclosure of which is incorporated herein by reference.

In some embodiments, at least one or each of the targeting groups is selected from ligands capable of binding to receptors on the surfaces of cells expressing the target gene.

In some embodiments, at least one or each of the targeting groups is selected from ligands capable of binding to receptors on the surfaces of mammalian hepatocytes. In some embodiments, each of the targeting groups is independently a ligand that has an affinity for an asialoglycoprotein receptor on the surface of mammalian hepatocytes. In some embodiments, each of the targeting groups is independently an asialoglycoprotein or a sugar. In some embodiments, each of the targeting groups is independently selected from one of D-mannopyranose, L-mannopyranose, D-arabinose, D-xylofuranose, L-xylofuranose, D-glucose, L-glucose, D-galactose, L-galactose, α-D-mannofuranose, β-D-mannofuranose, α-D-mannopyranose, β-D-mannopyranose, α-D-glucopyranose, β-D-glucopyranose, α-D-glucopyranose, β-D-glucofuranose, α-D-fructofuranose, α-D-fructopyranose, α-D-galactopyranose, β-D-galactopyranose, α-D-galactofuranose, β-D-galactofuranose, glucosamine, sialic acid, galactosamine, N-acetylgalactosamine, N-trifluoroacetylgalactosamine, N-propionylgalactosamine, N-n-butyrylgalactosamine, N-isobutyrylgalactosamine, 2-amino-3-O-[(R)-1-carboxyethyl]-2-deoxy-β-D-glucopyranose, 2-deoxy-2-methylamino-L-glucopyranose, 4,6-dideoxy-4-carboxamido-2,3-di-O-methyl-D-mannopyranose, 2-deoxy-2-sulfoamino-D-glucopyranose, N-glycoloyl-α-neuraminic acid, 5-thio-β-D-glucopyranose, 2,3,4-tri-O-acetyl-1-thio-6-O-trityl-α-D-glucopyranoside methyl ester, 4-thio-β-D-galactopyranose, 3,4,6,7-tetra-O-acetyl-2-deoxy-1,5-dithio-α-D-glucopyranoside ethyl ester, 2,5-anhydro-D-allose nitrile, ribose, D-ribose, D-4-thioribose, L-ribose, and L-4-thioribose. In some embodiments, at least one or each of the targeting groups is galactose or N-acetylgalactosamine.

In some embodiments, at least one or each of the targeting groups is selected from ligands capable of binding to receptors on the surfaces of lung epithelial cells. In some embodiments, each of the targeting groups is selected from a group targeting integrin αvβ6 or a group targeting integrin αvβ3. In some embodiments, each of the targeting groups is independently a polypeptide or a small molecule ligand.

In some embodiments, at least one or each of the delivery auxiliary groups is selected from groups capable of increasing the biocompatibility of the oligonucleotide conjugate in the central nervous system. In some embodiments, at least one or each of the delivery auxiliary groups is selected from lipophilic molecules. In some embodiments, each of the delivery auxiliary groups is a $C_5$-$C_{18}$ straight-chain hydrocarbon group or a steroid.

In some embodiments, the linker in the oligonucleotide conjugates of the present disclosure has a structure as represented by formula (301):

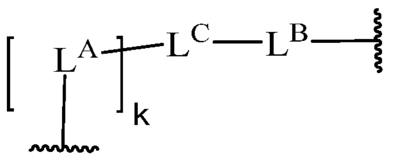

formula (301),

wherein k is an integer of 1 to 3;

$L^A$ has a structure containing an amide bond as represented by formula (302), $L^B$ has a structure containing N-acylpyrrolidine as represented by formula (303), containing carbonyl and oxygen atoms, and $L^C$ is a linking group based on hydroxymethylaminomethane, dimethylolaminomethane or trishydroxymethylaminomethane;

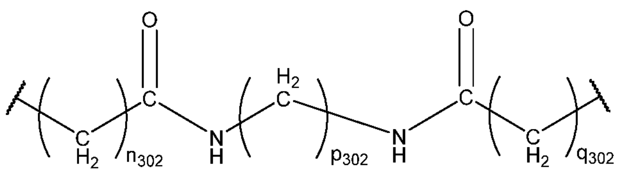

formula (302);

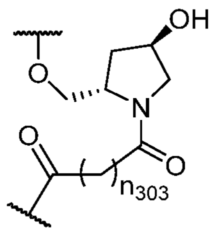

formula (303);

wherein $n_{302}$, $q_{302}$ and $p_{302}$ are each independently an integer of 2-6, and optionally, $n_{302}$, $q_{302}$ and $p_{302}$ are each independently 2 or 3; and $n_{303}$ is an integer of 4-16, and optionally, $n_{303}$ is an integer of 8-12. indicates the point at which the group is covalently linked.

In the linker, each $L^A$ is linked to one of the targeting groups through an ether bond, and is linked to the $L^C$ part through the oxygen atom of the carbonyl group in the $L^C$ part by forming an ether bond. $L^B$ is linked to the nitrogen atom in the $L^C$ part through the carbonyl group in formula (303) by forming an amido bond, and is linked to the double-stranded oligonucleotide through the oxygen atom in formula (303) by forming a phosphoester bond or a phosphorothioate bond.

In some embodiments, the oligonucleotide conjugate provided by the present disclosure has a structure as represented by formula (305):

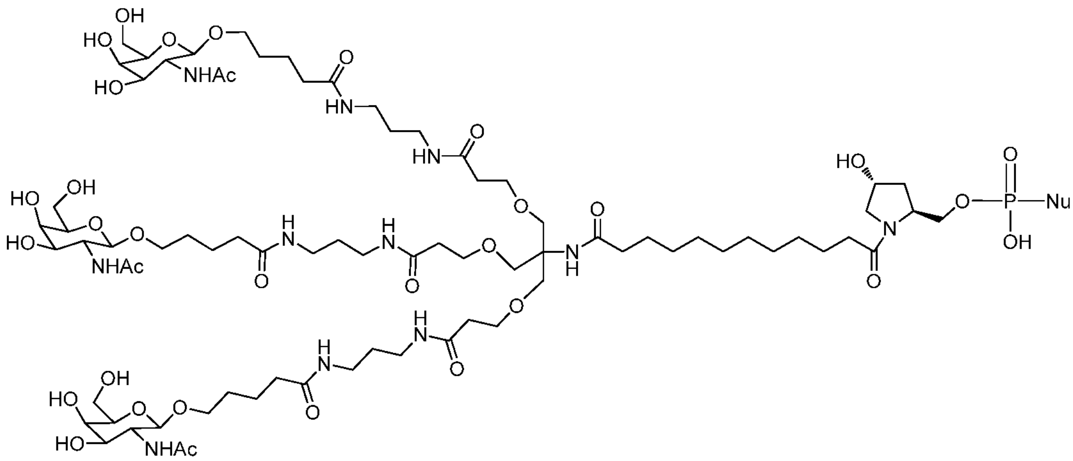

,

formula (305)

wherein, Nu represents a double-stranded oligonucleotide provided by the present disclosure, or a double-stranded oligonucleotide obtained by the method of the present disclosure.

In some embodiments, the linker in the oligonucleotide conjugates of the present disclosure has a structure as represented by formula (306):

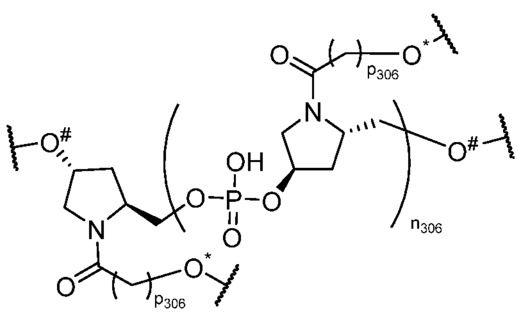

formula (306),

wherein, $n_{306}$ is an integer of 0-3, each $p_{306}$ is independently an integer of 1-6, and represents the site where

the group is covalently linked; the linking group forms ether bond linkage to the targeting group through the oxygen atom marked by *. The linking group forms at least one of the oxygen atoms marked by # to form a phosphoester bond or a phosphorothioate bond with the double-stranded oligonucleotide, and the rest are linked to a hydrogen atom through an oxygen atom marked by # to form a hydroxyl, or linked to a $C_1$-$C_3$ alkyl group to form a $C_1$-$C_3$ alkoxyl group;

In some embodiments, the oligonucleotide conjugate of the present disclosure has a structure as represented by formula (307):

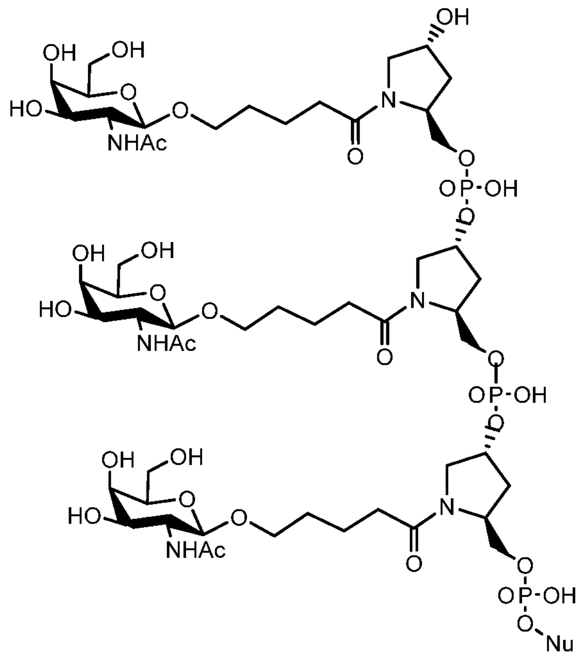

formula (307)

wherein, Nu represents a double-stranded oligonucleotide provided by the present disclosure, or a double-stranded oligonucleotide obtained by the method of the present disclosure.

In some embodiments, the oligonucleotide conjugate of the present disclosure has a structure as represented by formula (308):

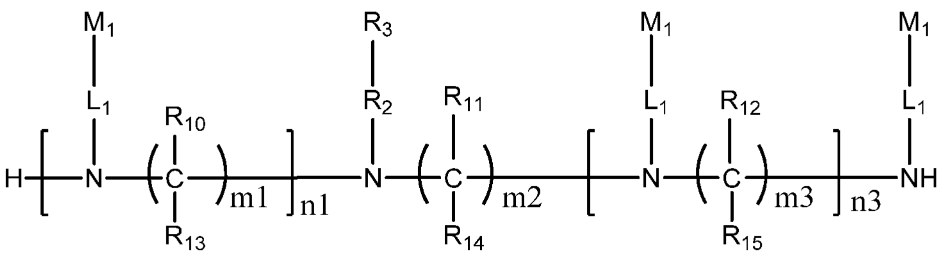

formula (308),

wherein,

n1 is an integer selected from 1 to 3, and n3 is an integer selected from 0 to 4;

each m1, m2, or m3 is independently an integer selected from 2 to 10;

$R_{10}$, $R_{11}$, $R_{12}$, $R_{13}$, $R_{14}$ or $R_{15}$ are each independently H, or are selected from the group consisting of the following groups: $C_1$-$C_{10}$ alkyl, $C_1$-$C_{10}$ haloalkyl and $C_1$-$C_{10}$ alkoxy;

$R_3$ has a structure represented by formula A59:

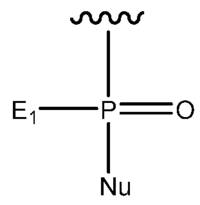

(A59)

wherein, $E_1$ is OH, SH or $BH_2$, Nu represents a double-stranded oligonucleotide provided by the present disclosure, or a double-stranded oligonucleotide obtained by the method of the present disclosure; $R_2$ is a straight-chain alkylene group that is 1-20 carbon atoms in length, wherein one or more carbon atoms are optionally substituted with any one or more selected from the group consisting of the following groups: C(O), NH, O, S, CH=N, $S(O)_2$, $C_2$-$C_{10}$ alkenylene, $C_2$-$C_{10}$ alkynylene, $C_6$-$C_{10}$ arylene, $C_3$-$C_{18}$ heterocyclylene and $C_5$-$C_{10}$ heteroarylene; and wherein $R_2$ may optionally have any one or more substituents from the group consisting of the following groups: $C_1$-$C_{10}$ alkyl, $C_6$-$C_{10}$ aryl, $C_5$-$C_{10}$ heteroaryl, $C_1$-$C_{10}$ haloalkyl, -$OC_1$-$C_{10}$ alkyl, -$OC_1$-$C_{10}$ alkylphenyl, -$C_1$-$C_{10}$ alkyl-OH, -OCi-$C_{10}$ haloalkyl, -$SC_1$-$C_{10}$ alkyl, -$SC_1$-$C_{10}$ alkylphenyl, -$C_1$-$C_{10}$ alkyl-SH, -$SC_1$-$C_{10}$ haloalkyl, halogen substituent, -OH, -SH, -$NH_2$, -$C_1$-$C_{10}$ alkyl-$NH_2$, -N($C_1$-$C_{10}$ alkyl)($C_1$-$C_{10}$ alkyl), -NH($C_1$-$C_{10}$ alkyl), -N($C_1$-$C_{10}$ alkyl)($C_1$-$C_{10}$ alkylphenyl), -NH($C_1$-$C_{10}$ alkylphenyl), cyano, nitro, -$CO_2$H, -C(O)O(Ci-$C_{10}$ alkyl), -CON($C_1$-$C_{10}$ alkyl)($C_1$-$C_{10}$ alkyl), -CONH($C_1$-$C_{10}$ alkyl), -$CONH_2$, -NHC(O)($C_1$-$C_{10}$ alkyl), -NHC(O)(phenyl), -N($C_1$-$C_{10}$ alkyl)C(O)($C_1$-$C_{10}$ alkyl), -N($C_1$-$C_{10}$ alkyl)C(O)(phenyl), - C(O)$C_1$-$C_{10}$ alkyl, -C(O)$C_1$-$C_{10}$ alkylphenyl, -C(O)$C_1$-$C_{10}$ haloalkyl, -OC(O)$C_1$-$C_{10}$ alkyl, -$SO_2$($C_1$-$C_{10}$ alkyl), -$SO_2$(phenyl), -$SO_2$($C_1$-$C_{10}$ haloalkyl), -$SO_2NH_2$, -$SO_2$NH($C_1$-$C_{10}$ alkyl), -$SO_2$NH (phenyl), -$NHSO_2$($C_1$-$C_{10}$ alkyl), -$NHSO_2$(phenyl) and -$NHSO_2$($C_1$-$C_{10}$ haloalkyl);

each Li is independently a straight-chain alkylene group that is 1-70 carbon atoms in length, wherein one or more carbon atoms are optionally substituted with any one or more selected from the group consisting of the following groups: C(O), NH, O, S, CH=N, $S(O)_2$, $C_2$-$C_{10}$ alkenylene, $C_2$-$C_{10}$ alkynylene, $C_6$-$C_{10}$ arylene, $C_3$-$C_{18}$ heterocyclylene and $C_5$-$C_{10}$ heteroarylene; and wherein Li may optionally have any one or more substituents from the group consisting of the following groups: Ci-$C_{10}$ alkyl, $C_6$-$C_{10}$ aryl, $C_5$-$C_{10}$ heteroaryl, $C_1$-$C_{10}$ haloalkyl, -$OC_1$-$C_{10}$ alkyl, -$OC_1$-$C_{10}$ alkylphenyl, - $C_1$-$C_{10}$ alkyl-OH, -$OC_1$-$C_{10}$ haloalkyl, -$SC_1$-$C_{10}$ alkyl, -SCi- $C_{10}$ alkylphenyl, -$C_1$-$C_{10}$ alkyl-SH, -SCi-$C_{10}$ haloalkyl, halogen substituent, -OH, -SH, -$NH_2$, -$C_1$-$C_{10}$ alkyl-$NH_2$, -N($C_1$-$C_{10}$ alkyl)($C_1$-$C_{10}$ alkyl), -NH($C_1$-$C_{10}$ alkyl), -N($C_1$-$C_{10}$ alkyl)($C_1$-$C_{10}$ alkylphenyl), -NH($C_1$-$C_{10}$ alkylphenyl), cyano, nitro, -$CO_2$H, -C(O)O($C_1$-$C_{10}$ alkyl), -CON($C_1$-$C_{10}$ alkyl)($C_1$-$C_{10}$ alkyl), -CONH($C_1$-$C_{10}$ alkyl), - $CONH_2$, -NHC(O)($C_1$-$C_{10}$ alkyl), -NHC(O)(phenyl), -N($C_1$-$C_{10}$ alkyl)C(O)($C_1$-$C_{10}$ alkyl), -N($C_1$-$C_{10}$ alkyl)C(O)(phenyl), -C(O)$C_1$-$C_{10}$ alkyl, -C(O)$C_1$-$C_{10}$ alkylphenyl, -C(O)$C_1$-$C_{10}$ haloalkyl, -OC(O)Ci-$C_{10}$ alkyl, -$SO_2$($C_1$-$C_{10}$ alkyl), -$SO_2$(phenyl), -$SO_2$($C_1$-$C_{10}$ haloalkyl), -$SO_2NH_2$, -$SO_2$NH($C_1$-$C_{10}$ alkyl), -$SO_2$NH(phenyl), -$NHSO_2$($C_1$-$C_{10}$ alkyl), -$NHSO_2$(phenyl) and -$NHSO_2$($C_1$-$C_{10}$ haloalkyl);

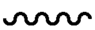 indicates the site where the group is covalently linked;

Mi represents a targeting group, and its definition and optional range are the same as above. In some embodiments, each Mi is independently selected from one of the ligands that have an affinity for the asialoglycoprotein receptor on the surface of mammalian hepatocytes.

Those skilled in the art will appreciate that although Li is defined as a straight-chain alkyl group for convenience, it may not be a linear group or have a different name, such as amine or alkenyl as a result of the substitutions and/or substitutions described above. For the purposes of the present disclosure, the length of Li is the number of atoms in the chain connecting the two points of attachment. For such purpose, a ring obtained by substituting a carbon atom of the straight-chain alkylene group, such as a heterocyclylene or heteroarylene, is counted as one atom.

When Mi is a ligand having affinity for asialoglycoprotein receptors on the surface of mammalian hepatocytes, in some embodiments, n1 may be an integer from 1 to 3, and n3 may be an integer from 0 to 4, such that the number of Mi ligands in the conjugate is at least 2. In some embodiments, $n1 + n3 \geq 2$, so that the number of Mi ligands is at least 3, and the Mi ligands more readily bind to the hepatic surface asialoglycoprotein receptor, which in turn facilitates the entry of the conjugate into the cell by endocytosis. Experiments have shown that when the number of Mi ligands is greater than 3, the ease of binding of Mi ligands to asialoglycoprotein receptors on the liver surface does not increase significantly. Therefore, from the perspective of ease of synthesis, structure/process cost and delivery efficiency and

other aspects into consideration, in some embodiments, n1 is an integer of 1-2, n3 is an integer of 0-1, and n1 + n3 = 2-3.

In some embodiments, when m1, m2, and m3 are independently selected from an integer of 2-10, the spatial positions between multiple Mi ligands may be suitable for the binding of Mi ligands to asialoglycoprotein receptors on the liver surface. In order to make the conjugates provided by the present disclosure simpler, easier to synthesize and/or reduce costs, in some embodiments, m1, m2 and m3 are each independently an integer of 2-5, and in some embodiments, m1 = m2 = m3.

Those skilled in the art will understand that when $R_{10}$, Rn, $R_{12}$, $R_{13}$, $R_{14}$ and $R_{15}$ are each independently selected from one of H, $C_1$-$C_{10}$ alkyl, $C_1$-$C_{10}$ haloalkyl, and $C_1$-$C_{10}$ alkoxy, the purpose of the present disclosure may be achieved without changing the properties of the conjugates disclosed herein. In some embodiments, $R_{10}$, Rn, $R_{12}$, $R_{13}$, $R_{14}$, and $R_{15}$ are each independently selected from H, methyl, and ethyl. In some embodiments, $R_{10}$, Rn, $R_{12}$, $R_{13}$, $R_{14}$, and $R_{15}$ are all H.

According to the oligonucleotide conjugate provided by the present disclosure, $R_3$ is a group with the structure represented by formula A59, wherein $E_1$ is OH, SH or $BH_2$. On the basis of the consideration of the availability of raw materials for preparation, $E_1$ is OH or SH in some embodiments.

In some embodiments, $R_2$ is selected to achieve linkage between N on the nitrogen-containing backbone and A59. In the context of the present disclosure, "nitrogen-containing backbone" refers to a chain structure in which carbon atoms linked to $R_{10}$, Rn, $R_{12}$, $R_{13}$, $R_{14}$ and $R_{15}$ are linked to N. Thus, $R_2$ may be any linking group capable of linking the A59 group to the N of the nitrogen-containing backbone in an appropriate manner. In some embodiments, in the case of preparing the oligonucleotide conjugates of the present disclosure by a solid-phase synthesis process, the $R_2$ group needs to contain both the linking site linked to the N on the nitrogen-containing backbone and the linking site linked to the P on in the $R_3$ group. In some embodiments, the site linked to N on the nitrogen-containing skeleton in the $R_2$ group forms an amide bond with N, and the site linked to P on the $R_3$ group forms a phosphoester bond with P. In some embodiments, $R_2$ is B5, B6, B5' or B6':

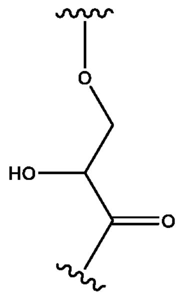
,
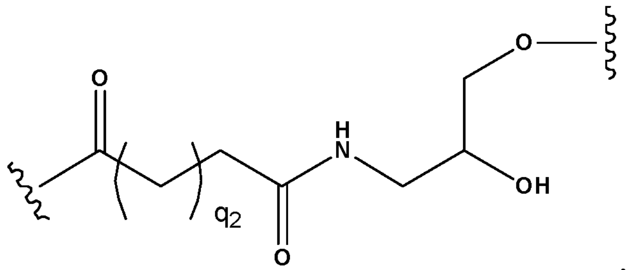
,

(B5) (B6)

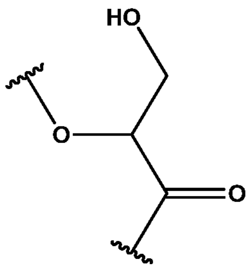
,
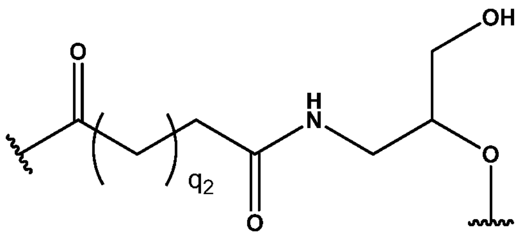
;

(B5') (B6')

wherein 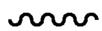 represents the site where the group is covalently linked.

The value range of $q_2$ may be an integer of 1-10, and in some embodiments, $q_2$ is an integer of 1-5.

The function of Li is to link the Mi ligand to N on the nitrogen-containing backbone, providing a targeting function for the oligonucleotide conjugate of the present disclosure. In some embodiments, Li is selected from a combination of one or more of the groups in formulas A1-A26. In some embodiments, Li is selected from a combination of one or more of the linkages A1, A4, A5, A6, A8, A10, A11 and A13. In some embodiments, Li is selected from a combination of at

least 2 of the linkages A1, A4, A8, A10 and A11. In some embodiments, Li is selected from a combination of at least 2 of the linkages A1, A8, and A10.

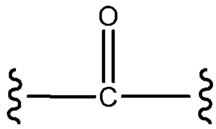, 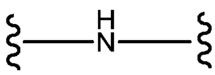, 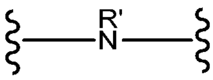, 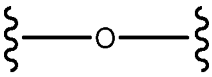,

(A1) (A2) (A3) (A4)

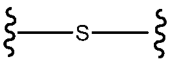, 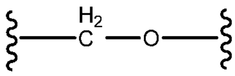, 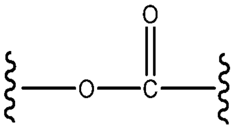, 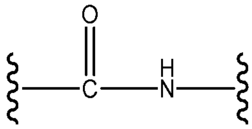,

(A5) (A6) (A7) (A8)

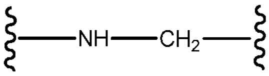, 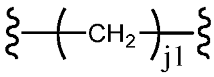, 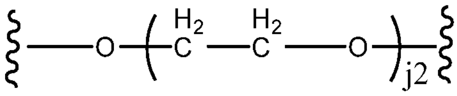,

(A9) (A10) (A11)

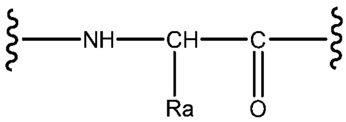, 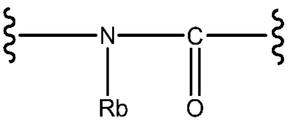, 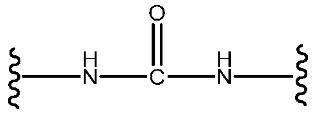,

(A12) (A13) (A14)

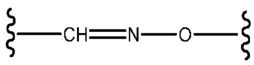, 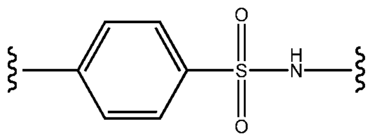, 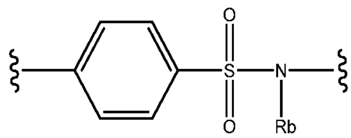,

(A15) (A16) (A17)

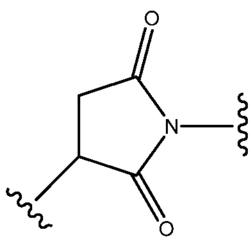, 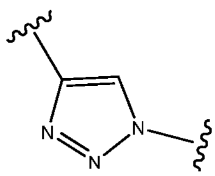, 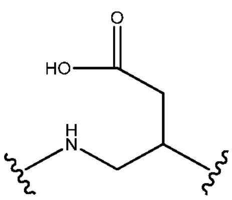, 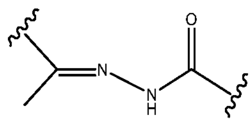,

(A18) (A19) (A20) (A21)

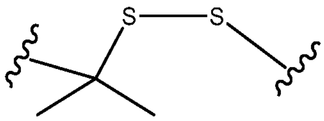, 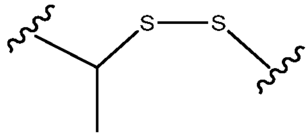, 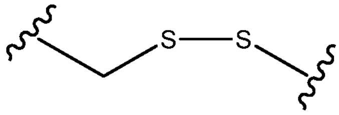,

(A22) (A23) (A24)

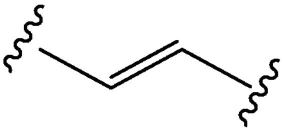 and 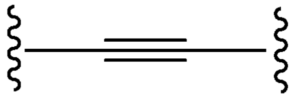;

(A25) (A26)

In some embodiments, the length of $L_1$ may be 3-25 atoms, 3-20 atoms, 4-15 atoms, or 5-12 atoms. In some embodiments, the length of $L_1$ is 3, 4, 5, 6, 7, 8, 9, 10, 11, 12, 13, 14, 15, 16, 17, 18, 19, 20, 21, 22, 23, 24, 25, 30, 35, 40, 45, 50, 55 or 60 atoms.

In some embodiments, j 1 is an integer of 2-10, and in some embodiments, j 1 is an integer of 3-5. In some embodiments, j2 is an integer of 2-10, and in some embodiments, j2 is an integer of 3-5. R' is C1-C4 alkyl, and in some embodiments, R' is one of methyl, ethyl and isopropyl. Ra is one of A27, A28, A29, A30 and A31, and in some embodiments, Ra is A27 or A28. Rb is C1-C5 alkyl, and in some embodiments, Rb is one of methyl, ethyl, isopropyl and butyl. In some embodiments, each of j1, j2, R', Ra, and Rb in formulas A1-A26 is selected to achieve the linkage of the Mi ligand to the N on the nitrogen-containing backbone, and to make the spatial position between the Mi ligands more suitable for the Mi ligand to bind to the liver surface asialoglycoprotein receptor.

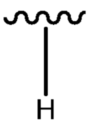, 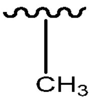, 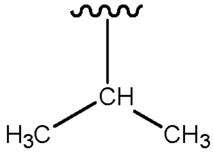, 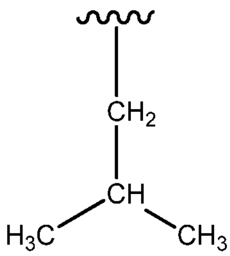, 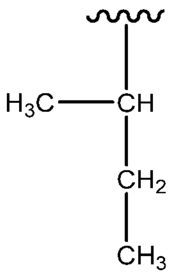

(A27) (A28) (A29) (A30) (A31)

In some embodiments, an oligonucleotide conjugate of the present disclosure has the structure represented by formula (403), (404), (405), (406), (407), (408), (409), (410), (411), (412), (413), (414), (415), (416), (417), (418), (419), (420), (421) or (422):

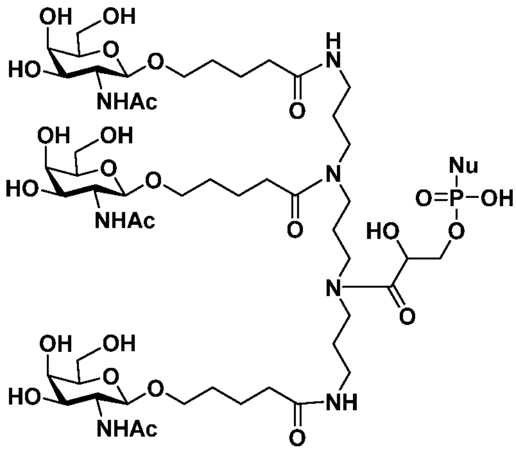

formula (403)

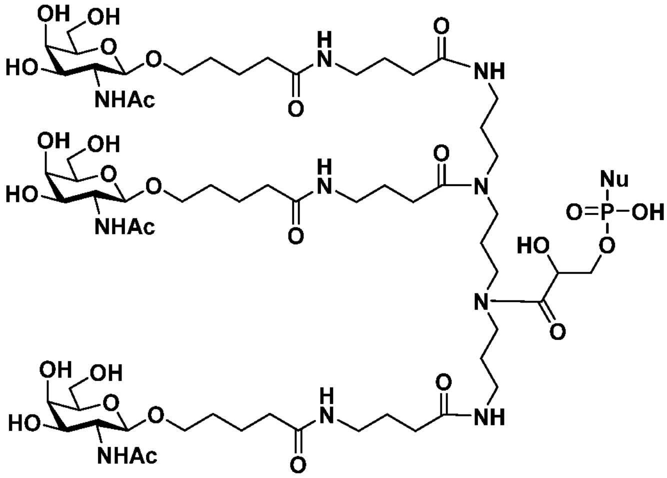

formula (404)

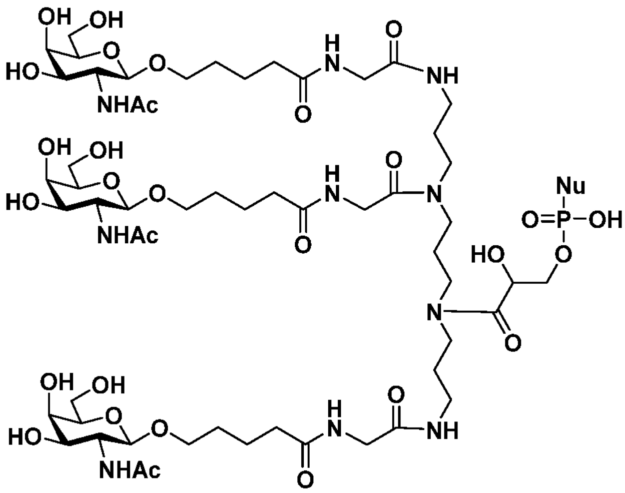

formula (405)

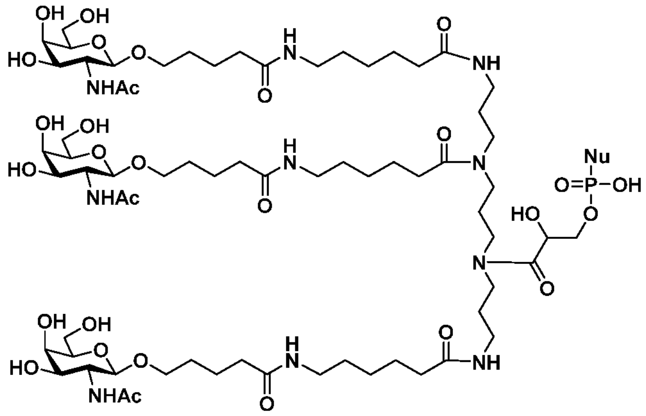

formula (406)

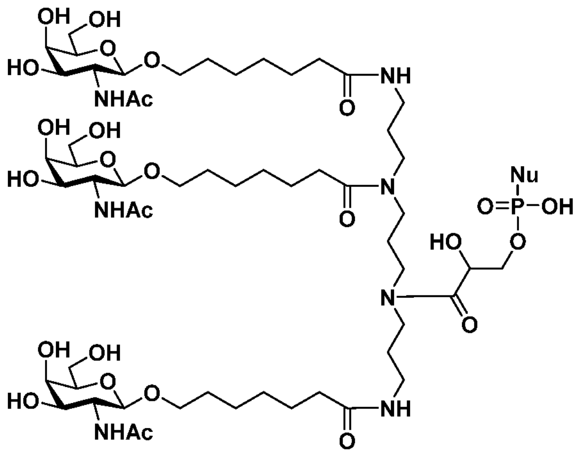

formula (407)

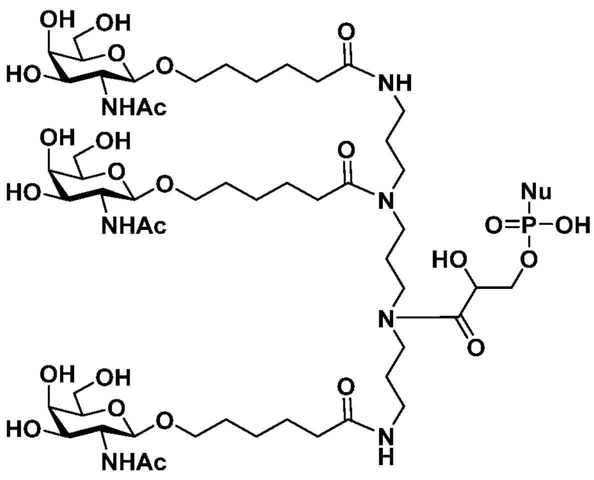

formula (408)

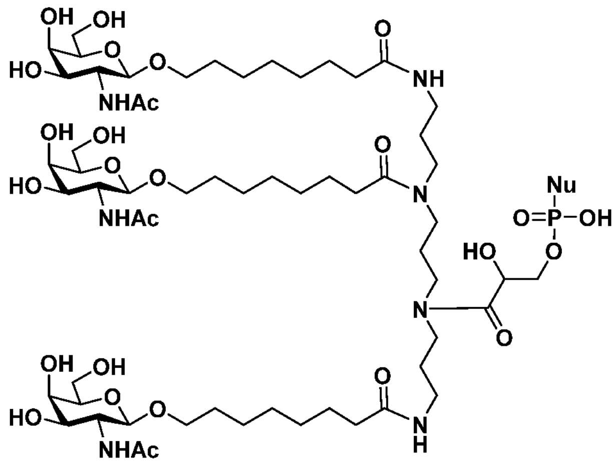

formula (409)

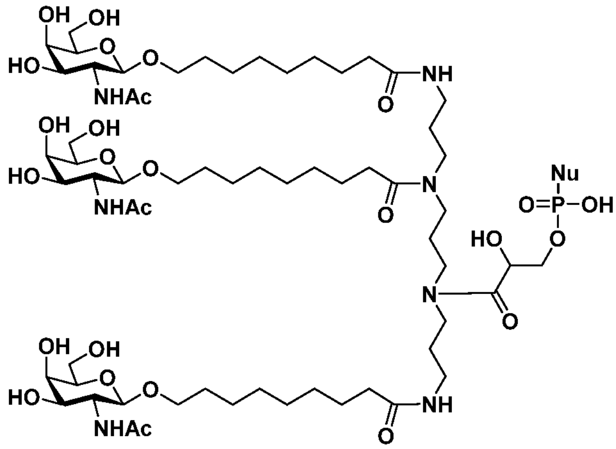

formula (410)

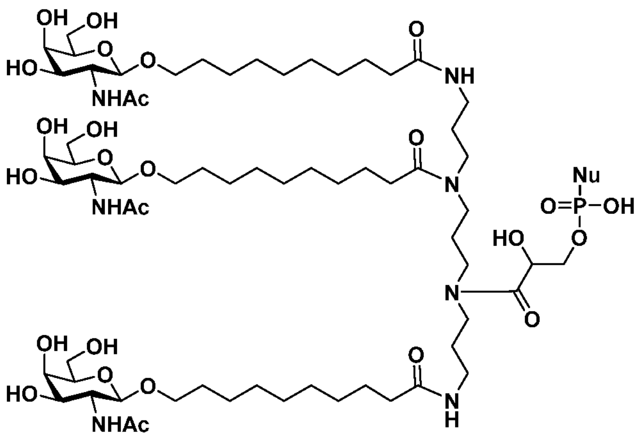

formula (411)

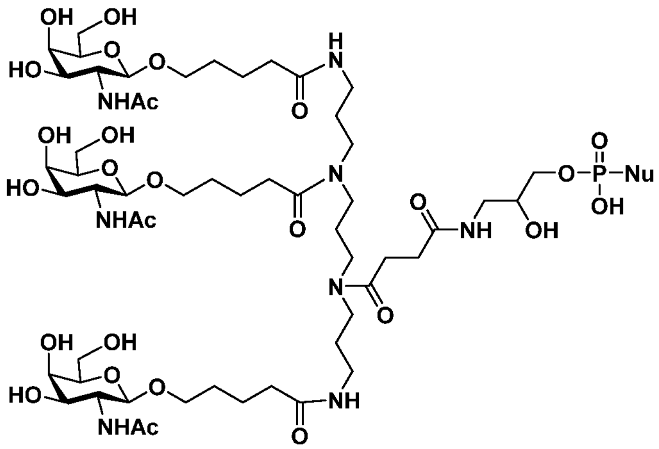

formula (412)

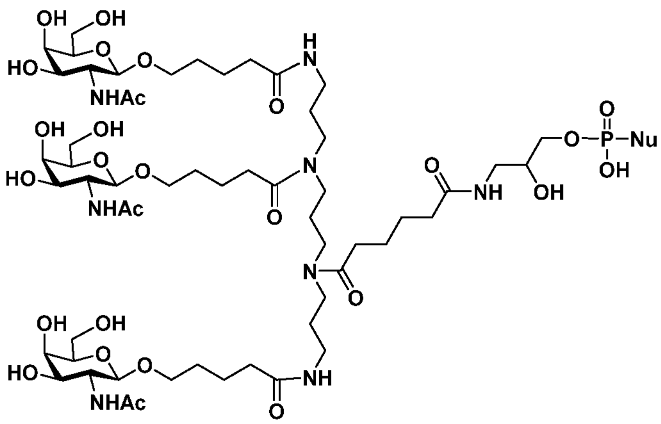

formula (413)

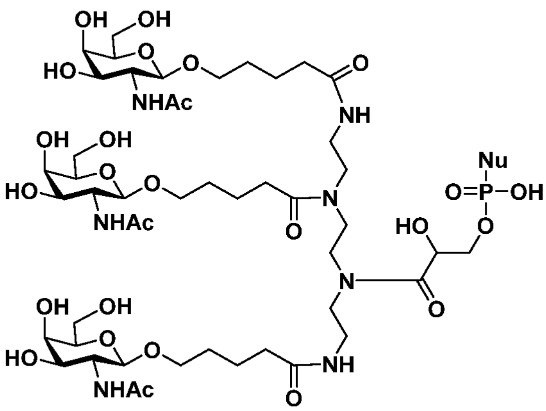

formula (414)

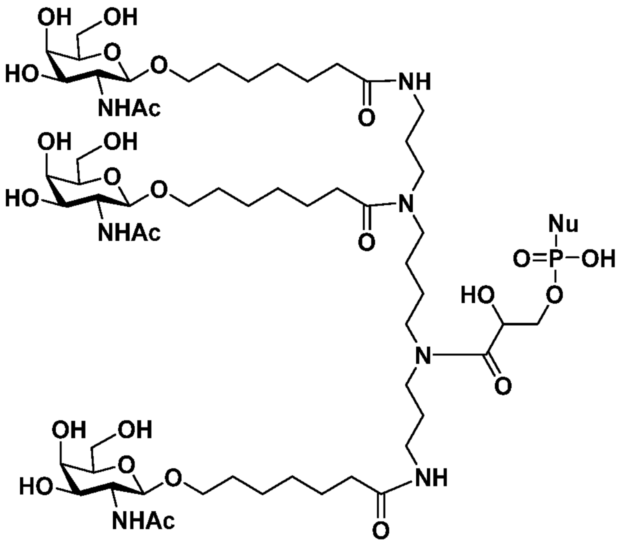

formula (415)

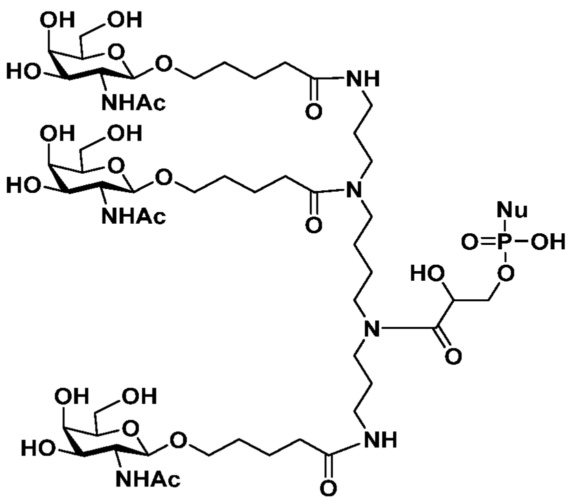

formula (416)

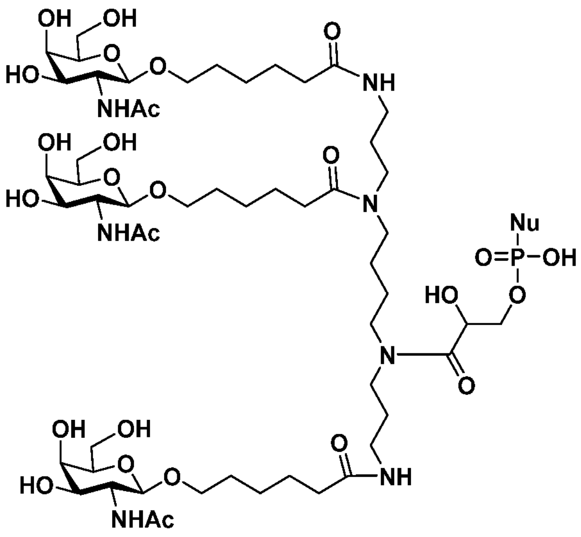

formula (417)

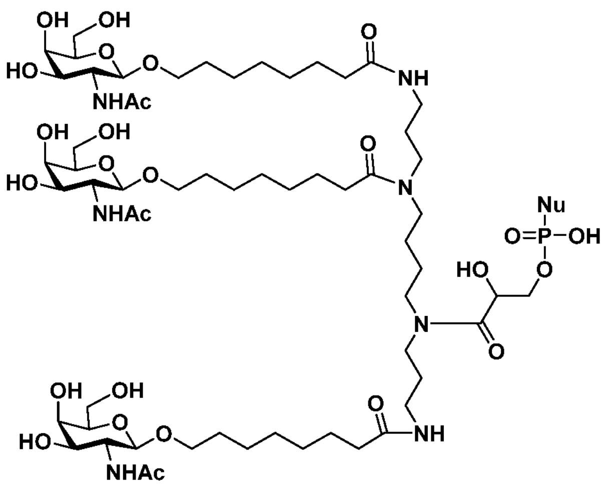

formula (418)

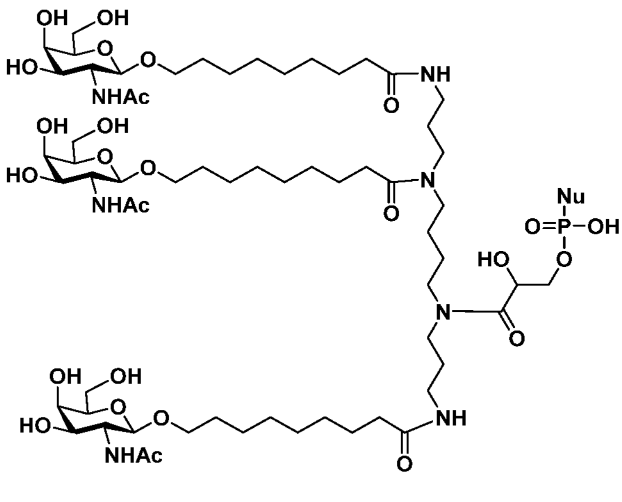

formula (419)

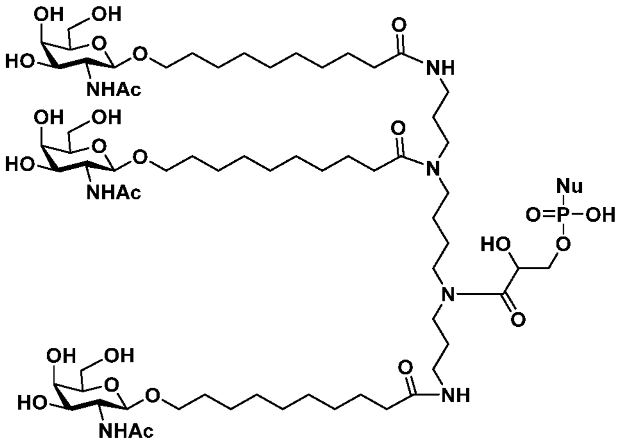

formula (420)

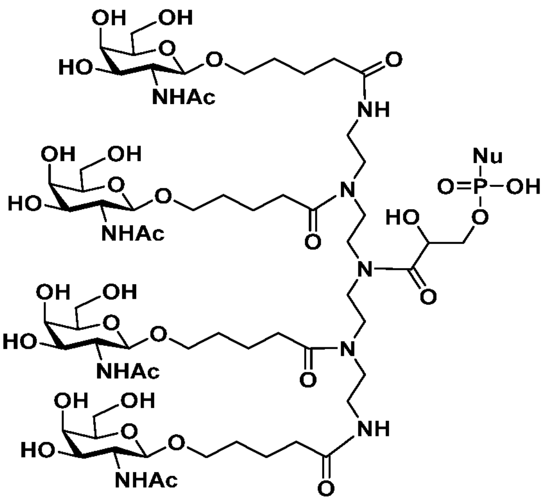

formula (421)

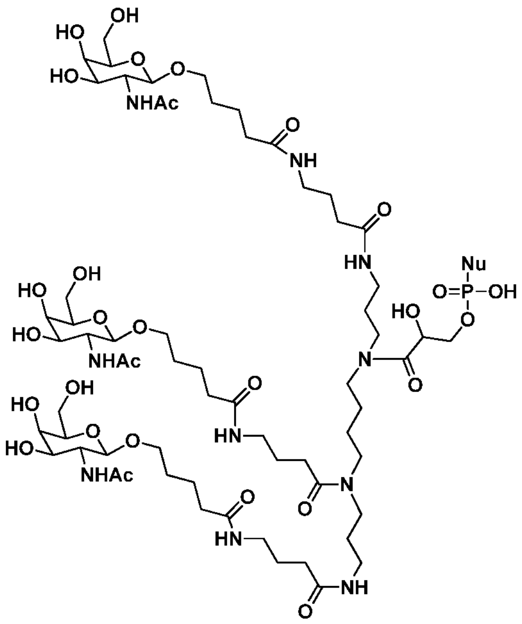

formula (422)

In some embodiments, P in formula A59 may be linked to any possible position in the double-stranded oligonucleotide sequence, for example, P in formula A59 may be linked to any nucleotide in the sense strand or antisense strand of the double-stranded oligonucleotide. In some embodiments, P in formula A59 is linked to any nucleotide in the sense strand of the double-stranded oligonucleotide. In some embodiments, P in formula A59 is linked to an end of the sense or antisense strand of the double-stranded oligonucleotide. In some embodiments, P in formula A59 is linked to an end of the sense strand of the double-stranded oligonucleotide. The end refers to the first 4 nucleotides in the sense strand or the antisense strand from one end thereof. In some embodiments, P in formula A59 is linked to an end of the sense or antisense strand of the double-stranded oligonucleotide. In some embodiments, P in formula A59 is linked to the 3’ end of the sense strand of the double-stranded oligonucleotide. In the case of being linked to the position of the sense strand of the double-stranded oligonucleotide, after the conjugate provided by the present disclosure enters the cell, upon unwinding, a separate antisense strand of the double-stranded oligonucleotide may be released to regulate target gene expression.

P in formula A59 may be linked to any possible position on the nucleotide in the double-stranded oligonucleotide, for example, the 5’ position of the nucleotide, the 2’ position of the nucleotide, the 3’ position of the nucleotide or the base of the nucleotide. In some embodiments, P in formula A59 may be linked to the 2’ position, the 3’ position or the 5’ position of the nucleotides in the double-stranded oligonucleotide by forming a phosphodiester bond. In some embodiments, the P in formula A59 is linked to the oxygen atom formed after the dehydrogenation of the 3’ hydroxyl of the 3’ end nucleotide in the sense strand of the double-stranded oligonucleotide, or the P in formula A59 is linked to the nucleotide by substituting the hydrogen in 2’-hydroxyl of one nucleotide in the sense strand of the oligonucleotide, or the P in formula A59 is linked to the nucleotide by substituting the hydrogen in 5’-hydroxyl of the nucleotide at the 5’ end in the sense strand of the oligonucleotide.

In some embodiments, the double-stranded oligonucleotide contained in the oligonucleotide conjugate of the present disclosure may be siRNA, and in this case, the oligonucleotide conjugate of the present disclosure is also referred to as siRNA conjugate. In some embodiments, the double-stranded oligonucleotide contained in the oligonucleotide conjugate of the present disclosure may be, for example, siRNAs listed in Table 1. Oligonucleotide conjugates containing these siRNAs exhibit low off-target effects and high mRNA inhibition activity on target gene expression.

Preparation of oligonucleotide conjugate of the present disclosure

The above oligonucleotide conjugate may be synthesized by methods that have been described in detail in the prior art. For example, WO2015006740A2 describes preparation methods for various siRNA conjugates in detail. In cases where the double-stranded oligonucleotide is siRNA, the oligonucleotide conjugate of the present disclosure may also be obtained by means well known to those skilled in the art. For example, the preparation method for the structure represented by formula (305) is described in WO2014025805A1, and the preparation method for the structure represented

by formula (307) is described by Rajeev et al. in ChemBioChem 2015, 16, 903-908. Chinese patent application CN110959011A also discloses in detail the method for preparing the oligonucleotide conjugate represented by formula (308). The contents of the documents are incorporated herein in their entirety by reference.

The oligonucleotide conjugate of the present disclosure may also be used in combination with other pharmaceutically acceptable adjuvants. The adjuvants may be one or more of various preparations or compounds routinely used in the art. For details, please refer to the description about the pharmaceutical composition of the present disclosure.

Use of double-stranded oligonucleotide, pharmaceutical composition and oligonucleotide conjugate of the present disclosure

In some embodiments, the present disclosure provides use of the double-stranded oligonucleotide provided by the present disclosure, the double-stranded oligonucleotide obtained by the method of the present disclosure, the pharmaceutical composition and/or the oligonucleotide conjugate in the preparation of a medicament for treating and/or preventing diseases or symptoms associated with levels of an mRNA expressed by a target gene. In some embodiments, the specific gene is a gene abnormally expressed in hepatocytes. In some embodiments, the specific gene is an endogenous gene expressed in the liver. In some embodiments, the specific gene is a pathogen gene that proliferates in the liver. In some embodiments, the specific gene is a gene expressed in lung epithelial cells. In some embodiments, the specific gene is a gene expressed in the central nervous system. In some embodiments, the specific gene is a gene expressed in tumor cells. In some embodiments, the mRNA expressed by the target gene is selected from one of the mRNAs transcribed from the following genes: ACE2, AGT, ANGPTL3, ApoA, ApoB, ApoC, AR, ASK1, C3, C5, Col1A1, CTGF, Ebola, FOXO1, FTO, FVII, FXI, FXII, GCGR, HBV, HCV, HSD, p53, PCSK9, PNP, PLG, PKK, KNG, RAGE, RPTOR, SARS-CoV-2, SCD1, SCNN1A, SOD1, STAT3, TIMP-1, TMPRSS6 and XO. In some embodiments, the mRNA expressed by the target gene is selected from an mRNA expressed by a hepatitis B virus gene (HBV), an mRNA expressed by an angiopoietin-like protein 3 (ANGPTL3) gene, or an mRNA expressed by an apolipoprotein C3 (ApoC3) gene. In some embodiments, the disease or symptom associated with the level of the mRNA expressed by the target gene is chronic liver disease, hepatitis, liver fibrotic disease, liver proliferative disease and/or dyslipidemia. In some embodiments, the disease or symptom associated with the level of the mRNA expressed by the target gene is hepatitis B or dyslipidemia. In some embodiments, the dyslipidemia is hypercholesterolemia, hypertriglyceridemia, or atherosclerosis.

In some embodiments, the present disclosure provides a method for treating and/or preventing diseases or symptoms associated with the level of the mRNA expressed by the target gene, the method comprising: administering to a subject in need with an effective amount of the double-stranded oligonucleotides provided by the present disclosure, the double-stranded oligonucleotides obtained by the method of the present disclosure, the pharmaceutical composition and/or the oligonucleotide conjugate. In some embodiments, the mRNA expressed by the target gene is selected from one of the mRNAs transcribed from the following genes: ACE2, AGT, ANGPTL3, ApoA, ApoB, ApoC, AR, ASK1, C3, C5, Col1A1, CTGF, Ebola, FOXO1, FTO, FVII, FXI, FXII, GCGR, HBV, HCV, HSD, p53, PCSK9, PNP, PLG, PKK, KNG, RAGE, RPTOR, SARS-CoV-2, SCD1, SCNN1A, SOD1, STAT3, TIMP-1, TMPRSS6 and XO. In some embodiments, the mRNA expressed by the target gene is selected from an mRNA expressed by a hepatitis B virus gene (HBV), an mRNA expressed by an angiopoietin-like protein 3 (ANGPTL3) gene, or an mRNA expressed by an apolipoprotein C3 (ApoC3) gene. In some embodiments, the disease or symptom associated with the level of the mRNA expressed by the target gene is chronic liver disease, hepatitis, liver fibrotic disease, liver proliferative disease and/or dyslipidemia. In some embodiments, the disease or symptom associated with the level of the mRNA expressed by the target gene is hepatitis B or dyslipidemia. In some embodiments, the dyslipidemia is hypercholesterolemia, hypertriglyceridemia, or atherosclerosis.

In some embodiments, the conjugates provided by the present disclosure may also be used to treat other liver diseases, including diseases characterized by unwanted cell proliferation, blood diseases, metabolic diseases and diseases characterized by inflammation. A proliferative disease of the liver may be a benign or malignant disease, such as cancer, hepatocellular carcinoma (HCC), liver metastases, or hepatoblastoma. A hematological or inflammatory disease of the liver may be a disease involving coagulation factors, complement-mediated inflammation or fibrosis. Metabolic disorders of the liver include dyslipidemia and irregularities in glucose regulation. In one embodiment, the disease is treated by administering one or more double-stranded oligonucleotides with high homology to gene sequences involved in the disease.

In some embodiments, the present disclosure provides a method for regulating the expression level of a target gene in a cell, the method comprising: contacting an effective amount of the double-stranded oligonucleotides provided by the present disclosure, the double-stranded oligonucleotides obtained by the method of the present disclosure, the pharmaceutical composition and/or the oligonucleotide conjugate with the cell. In some embodiments, the mRNA expressed by the target gene is selected from one of the mRNAs transcribed from the following genes: ACE2, AGT, ANGPTL3, ApoA, ApoB, ApoC, AR, ASK1, C3, C5, Col1A1, CTGF, Ebola, FOXO1, FTO, FVII, FXI, FXII, GCGR, HBV, HCV, HSD, p53, PCSK9, PNP, PLG, PKK, KNG, RAGE, RPTOR, SARS-CoV-2, SCD1, SCNN1A, SOD1, STAT3, TIMP-1, TMPRSS6 and XO. In some embodiments, the regulation refers to inhibiting the expression of the target gene in the

cell. The mRNA expressed by the target gene is selected from an mRNA expressed by a hepatitis B virus gene (HBV), an mRNA expressed by an angiopoietin-like protein 3 (ANGPTL3) gene, or an mRNA expressed by an apolipoprotein C3 (ApoC3) gene.

By administering the double-stranded oligonucleotide provided by the present disclosure, the double-stranded oligonucleotide obtained by the method of the present disclosure, the pharmaceutical composition and/or the oligonucleotide conjugate to a subject in need, the purpose of preventing and/or treating pathological conditions or diseases caused by the expression of specific genes in cells may be achieved by a mechanism that regulates the gene expression. Therefore, the double-stranded oligonucleotide provided by the present disclosure, the double-stranded oligonucleotide obtained by the method of the present disclosure, the pharmaceutical composition and/or the oligonucleotide conjugate may be used to prevent and/or treat the pathological conditions or disease, or to prepare a medicament for the prevention and/or treatment of the pathological conditions or diseases as described herein.

As used herein, the term "administration/administer" refers to a method or approach of positioning at least a part of the double-stranded oligonucleotide, pharmaceutical composition and/or oligonucleotide conjugate at a desired site to produce a desired effect so as to place the double-stranded oligonucleotide, pharmaceutical composition and/or oligonucleotide conjugate into a subject. Routes of administration suitable for the methods of the present disclosure include topical and systemic administration. In general, the topical administration results in delivery of more double-stranded oligonucleotides, pharmaceutical compositions and/or oligonucleotide conjugates to a specific site compared to the entire body of a subject, whereas the systemic administration results in delivery of the double-stranded oligonucleotides, pharmaceutical compositions and/or oligonucleotide conjugates to substantially the entire body of the subject.

The administration to a subject may be by any suitable route known in the art, including, but not limited to: oral or parenteral routes, such as intravenous, intramuscular, subcutaneous, transdermal, airway (aerosol), pulmonary, nasal, rectal, and topical (including buccal and sublingual) administration. The frequency of administration may be one or more times every day, every week, every two weeks, every three weeks, every month or every year.

The doses of the double-stranded oligonucleotides, pharmaceutical compositions and/or oligonucleotide conjugates of the present disclosure may be conventional doses in the art, and the doses may be based on various parameters, especially the age, weight and sex of the subjects. Toxicity and efficacy may be determined by standard pharmaceutical procedures in cell culture or experimental animals, such as determining the LD50 (the dose that causes 50% of the population to die) and the ED50 (the dose that may cause 50% of the maximum response intensity in terms of quantitative response, and the dose that may cause 50% of the test subjects to have a positive reaction in terms of qualitative response). The range of doses for use in humans may be derived based on the data obtained from cell culture assays and animal studies.

When administering the double-stranded oligonucleotide, pharmaceutical composition and/or oligonucleotide conjugate of the present disclosure, for example, to C57BL/6J, or C3H/HeNCrlVr mice (male or female, 6-12 weeks old, weighing 18-25 g), the administration is based on the amount of the double-stranded oligonucleotides, the pharmaceutical compositions and/or the double-stranded oligonucleotides in the oligonucleotide conjugates: for the oligonucleotide conjugate formed by the double-stranded oligonucleotides and the pharmaceutically acceptable conjugated molecules, the amount of the double-stranded oligonucleotides may be 0.001-100 mg/kg body weight. In some embodiments, the amount is 0.01-50 mg/kg body weight. In a further embodiment, the amount is 0.05-20 mg/kg body weight. In a yet further embodiment, the amount is 0.1-15 mg/kg body weight. In a still further embodiment, the amount is 0.1-10 mg/kg body weight. The doses may be preferred when administering the double-stranded oligonucleotides, pharmaceutical compositions and/or oligonucleotide conjugates of the present disclosure.

In addition, by introducing the double-stranded oligonucleotide, pharmaceutical composition and/or oligonucleotide conjugate of the present disclosure into cells with abnormal expression of specific genes, it is also possible to achieve the purpose of inhibiting the expression of the specific genes in the cells through the mechanism of gene expression regulation. In some embodiments, the cells are hepatocytes. In some embodiments, the hepatocytes may be cells selected from hepatoma cell lines such as Hep3B, HepG2 and Huh7, or isolated primary hepatocytes, and in some embodiments, the cells are primary hepatocytes.

For using the method provided by the present disclosure to inhibit the expression of specific genes in cells, the amount of the double-stranded oligonucleotides in the provided double-stranded oligonucleotides, pharmaceutical compositions and/or oligonucleotide conjugates are easily determined by those skilled in the art according to the desired effect. For example, in some embodiments, the double-stranded oligonucleotide, pharmaceutical composition and/or oligonucleotide conjugate is a siRNA conjugate, and the amount of the siRNAs in the provided siRNA conjugate is such that: it is sufficient to reduce the expression of the target gene and result in an extracellular concentration of 1 pM to 1 μM, or 0.01 nM to 100 nM, or 0.05 nM to 50 nM, or to about 5 nM at the target cell surface. The amount necessary to achieve this local concentration will vary depending on various factors including the method of delivery, the site of delivery, the number of cell layers between the site of delivery and the target cell or tissue, delivery range (local or systemic delivery), and the like. The concentration at the site of delivery may be significantly higher than the concentration at the surface of the target cell or tissue.

Kit

The present disclosure provides a kit, comprising a double-stranded oligonucleotide provided by the present disclosure, a double-stranded oligonucleotide obtained by a method of the present disclosure, a pharmaceutical composition and/or an oligonucleotide conjugate.

In some embodiments, the kit may provide the double-stranded oligonucleotide, pharmaceutical composition and/or conjugate in one container. In some embodiments, the kit may contain a container providing a pharmaceutically acceptable excipient. In some embodiments, the kit may further comprise other components, such as stabilizers or preservatives. In some embodiments, the kit may contain at least one additional therapeutic agent in a container other than the container in which the double-stranded oligonucleotides, pharmaceutical compositions and/or conjugates are provided. In some embodiments, the kit may contain an instruction for mixing the double-stranded oligonucleotide, the pharmaceutical composition and/or the conjugate with a pharmaceutically acceptable carrier and/or an excipient or other components (if any).

In the kit of the present disclosure, the double-stranded oligonucleotide and the pharmaceutically acceptable carrier and/or adjuvant and the pharmaceutical composition and/or conjugate, and/or the pharmaceutically acceptable adjuvant may be provided in any form, for example, liquid form, dry form or lyophilized form. In some embodiments, the double-stranded oligonucleotide and the pharmaceutically acceptable carrier and/or adjuvant and the pharmaceutical composition and/or conjugate and optional pharmaceutically acceptable adjuvant are substantially pure and/or sterile. In some embodiments, sterile water may be provided in the kit of the present disclosure.

The following examples will further illustrate the present disclosure, but the present disclosure is not limited thereby.

Without wishing to be limiting, the present invention is described in further detail in the embodiments below and the examples regarding exemplary embodiments in which the double-stranded oligonucleotide in the pharmaceutical compositions and/or oligonucleotide conjugates of the present disclosure is small interfering RNA (siRNA). In this case, the double-stranded oligonucleotide, pharmaceutical composition, and oligonucleotide conjugate of the present disclosure are siRNA, pharmaceutical composition comprising the siRNA, and siRNA conjugate, respectively. In the context of the present disclosure, for ease of description, the siRNA, the pharmaceutical composition comprising the siRNA and the siRNA conjugate in these embodiments are also referred to as the siRNA of the present disclosure, the pharmaceutical composition of the present disclosure and the siRNA conjugate of the present disclosure. This does not mean that the double-stranded oligonucleotide of the present disclosure can only be siRNA, on the contrary, the double-stranded oligonucleotide may be other variants disclosed herein or known to those skilled in the art, for example, small activating RNA (saRNA). It is contemplated that, on the basis of the specification of the siRNA, the pharmaceutical composition comprising the siRNA and the siRNA conjugate, other double-stranded oligonucleotides will function similarly when being used alone or forming the pharmaceutical composition and/or oligonucleotide conjugate of the present disclosure.

## Examples

Unless otherwise specified, the reagents and media used in the following examples are all commercially available, and the procedures such as nucleic acid electrophoresis and real-time PCR used are all performed according to the methods described in Molecular Cloning (Cold Spring Harbor LBboratory Press (1989)).

Preparation Examples 1-4. Synthesis of siRNAs provided by the present disclosure

According to the method described in Preparation Example 1 in WO2019105418(A1), the siRNA sequences listed in Table 2 were synthesized by solid-phase synthesis, the only difference being that DEPC water was used to dissolve the equimolar complementary sense strand and antisense strand in Table 2. After annealing, siRNA1-siRNA4 provided by the present disclosure were obtained, and their sequences are shown in Table 2.

Table 2. siRNA sequences

| Preparation Example No. | siRNA No. | Sequence (5'-3') | | SEQ ID NO |
|---|---|---|---|---|
| Preparation Example 1 | siRNA 1 | Sense strand | CmsCmsUmUmGmAmGfGfCfAmUmAmCmUmUmCmAmAmAm | 47 |
| | | Antisense strand | UmsUmsTmoeGmAmAfGmUmAmUmGmCmCmUfCmAfAmGmGmsUmsUm | 48 |

(continued)

| Preparation Example No. | siRNA No. | Sequence (5'-3') | | SEQ ID NO |
|---|---|---|---|---|
| Preparation Example 2 | siRNA2 | Sense strand | CmsCmsUmUmGmAmGfGfCfAmUmAmCmUmUmCmAmAmAm | 49 |
| | | Antisense strand | UmsUmsUmGmoeAmAfGmUmAmUmGmCmCmUfCmAfAmGmGmsUmsUm | 50 |
| Preparation Example 3 | siRNA3 | Sense strand | CmsCmsUmUmGmAmGfGfCfAmUmAmCmUmUmCmAmAmAm | 51 |
| | | Antisense strand | UmsUmsUmGmAmoeAfGmUmAmUmGmCmCmUfCmAfAmGmGmsUmsUm | 52 |
| Preparation Example 4 | siRNA4 | Sense strand | CmsCmsUmUmGmAmGfGfCfAmUmAmCmUmUmCmAmAmAm | 53 |
| | | Antisense strand | UmsUmsUmGmAmAmoeGmUmAmUmGmCmCmUfCmAfAmGmGmsUmsUm | 54 |
| Comparative Preparation Example 1 | Reference siRNA1 | Sense strand | CmsCmsUmUmGmAmGfGfCfAmUmAmCmUmUmCmAmAmAm | 55 |
| | | Antisense strand | UmsUmsUmGmAmAfGmUmAmUmGmCmCmUfCmAfAmGmGmsUmsUm | 56 |
| Comparative Preparation Example 2 | Reference siRNA2 | Sense strand | CmsCmsUmUmGmAmGfGfCfAmUmAmCmUmUmCmAmAmAm | 57 |
| | | Antisense strand | UmsTmoesUmGmAmAfGmUmAmUmGmCmCmUfCmAfAmGmGmsUmsUm | 58 |
| Comparative Preparation Example 3 | siRNA NC | Sense strand | UmsUmsCmUmCmCmGfAfAfCmGmUmGmUmCmAmCmGmUm | 59 |
| | | Antisense strand | AmsCfsGmUmGmAfCmAmCmGmUmUmCmGfGmAfGmAmAmsCmsUm | 60 |

The uppercase letters C, G, U, A, and T represent the base composition of nucleotides; the lowercase letter m indicates that the nucleotide adjacent to the letter m on the left side is a methoxy-modified nucleotide; the lowercase letter f indicates that the nucleotide adjacent to the letter f on the left side is a fluoro-modified nucleotide; the lowercase letter combination moe underlined indicates that the nucleotide adjacent to the letter combination on the left side is a ribose 2'-O-methoxyethyl-modified nucleotide; the lowercase letter s indicates that the two nucleotides on the left and right sides of the letter s are linked by a phosphorothioate group.

Comparative Preparation Examples 1-3. Synthesis of reference siRNAs

According to the method described in Preparation Example 1 in WO2019105418(A1), the respective sense and antisense strands of the siRNAs designated reference siRNA1, reference siRNA2, and NC in Table 2 were synthesized by solid-phase synthesis. DEPC water was used to dissolve the obtained equimolar sense strand and antisense strand. After annealing, the reference siRNA designated NC was obtained.

Preparation Examples 5-23. Synthesis of siRNA conjugates provided by the present disclosure

According to the preparation method described in Preparation Example 1 of CN110959011A, the following conjugates 6-24 in Table 3 were prepared, the only difference being that the sense strand and antisense strand of the siRNA contained in each siRNA conjugate were as shown in Table 3. For the nucleic acid sequences, according to the nucleic acid sequences of the siRNAs designated conjugate 5-conjugate 23 in Table 3, the sense strands and the antisense strands of the siRNAs were synthesized. Ultrapure water (Milli-Q ultrapure water instrument, resistivity 18.2

M92*cm (25 °C)) was used to dilute each siRNA conjugate to a concentration of 0.2 mg/mL (based on siRNA), and then a liquid chromatography-mass spectrometry instrument (LC-MS, purchased from Waters Inc., model: LCT Premier) was used for molecular weight determination. The found value was consistent with the calculated value, indicating that the synthesized conjugates 5-23 have the target designed double-stranded nucleic acid sequences. The siRNA conjugates have a structure represented by formula (403), and the siRNAs contained in the siRNA conjugates have the siRNA sequences corresponding to conjugates 5-23 in Table 3.

Table 3. siRNA sequences in siRNA conjugates

| Preparation Example No. | siRNA conjugate No. | Sequence (5'-3') | | SEQ ID NO |
|---|---|---|---|---|
| Preparation Example 5 | Conjugate 5 | Sense strand | CmsCmsUmUmGmAmGfGfCfAmUmAmCmUmUmCmAmAmAm | 61 |
| | | Antisense strand | VPUmsUmsTmoeGmAmAfGmUmAmUmGmCmCmUfCmAfAmGmGmsUmsUm | 62 |
| Preparation Example 6 | Conjugate 6 | Sense strand | CmsCmsUmUmGmAmGfGfCfAmUmAmCmUmUmCmAmAmAm | 63 |
| | | Antisense strand | VPUmsUmsUmGmAmoeAfGmUmAmUmGmCmCmUfCmAfAmGmGmsUmsUm | 64 |
| Preparation Example 7 | Conjugate 7 | Sense strand | CmsCmsUmUmGmAmGfGfCfAmUmAmCmUmUmCmAmAmAm | 65 |
| | | Antisense strand | VPUmsUmsTmoeGmAmoeAfGmUmAmUmGmCmCmUfCmAfAmGmGmsUmsUm | 66 |
| Preparation Example 8 | Conjugate 8 | Sense strand | CmsCmsUmUmGmAmGfGfCfAmUmAmCmUmUmCmAmAmAm | 67 |
| | | Antisense strand | VPUmsUmsUmGmAmoeAfGmoeUmAmUmGmCmCmUfCmAfAmGmGmsUmsUm | 68 |
| Preparation Example 9 | Conjugate 9 | Sense strand | UmsGmsCmUmAmUmGfCfCfUmCmAmUmCmUmUmCmUmAm | 69 |
| | | Antisense strand | VPUmsAfsGmoeAmAmGfAmUmGmAmGmGmCmAfUmAfGmCmAmsUmsUm | 70 |
| Preparation Example 10 | Conjugate 10 | Sense strand | UmsGmsCmUmAmUmGfCfCfUmCmAmUmCmUmUmCmUmAm | 71 |
| | | Antisense strand | VPUmsAfsGmAmAmoeGfAmUmGmAmGmGmCmAfUmAfGmCmAmsUmsUm | 72 |
| Preparation Example 11 | Conjugate 11 | Sense strand | GmsGmsAmCmAmGmUfAfUfUmCmUmCmAmGmUmGmCmUm | 73 |
| | | Antisense strand | PAmsGfsCmoeAmCmUfGmAmGmAmAmUmAmCfUmGfUmCmCmsCmsUm | 74 |
| Preparation Example 12 | Conjugate 12 | Sense strand | GmsGmsAmCmAmGmUfAfUfUmCmUmCmAmGmUmGmCmUm | 75 |
| | | Antisense strand | PAmsGfsCmAmCmoeUfGmAmGmAmAmUmAmCfUmGfUmCmCmsCmsUm | 76 |

(continued)

| Preparation Example No. | siRNA conjugate No. | Sequence (5'-3') | | SEQ ID NO |
|---|---|---|---|---|
| Preparation Example 13 | Conjugate 13 | Sense strand | UmsUmsAmAmAmAmGfGfGfAmCmAmGmUmAmUmUmCmUm | 77 |
| | | Antisense strand | PAmsGfsAmoeAmUmAfCmUmGmUmCmCmCmUfUmUfUmAmAmsGmsCm | 78 |
| Preparation Example 14 | Conjugate 14 | Sense strand | UmsUmsAmAmAmAmGfGfGfAmCmAmGmUmAmUmUmCmUm | 79 |
| | | Antisense strand | PAmsGfsAmAmTmoeAfCmUmGmUmCmCmCmUfUmUfUmAmAmsGmsCm | 80 |
| Preparation Example 15 | Conjugate 15 | Sense strand | CmsAmsAmUmAmAmAfGfCfUmGmGmAmCmAmAmGmAmAm | 81 |
| | | Antisense strand | PUmsUfsCmoeUmUmGfUmCmCmAmGmCmUmUfUmAfUmUmGmsGmsGm | 82 |
| Preparation Example 16 | Conjugate 16 | Sense strand | CmsAmsAmUmAmAmAfGfCfUmGmGmAmCmAmAmGmAmAm | 83 |
| | | Antisense strand | PUmsUfsCmUmTmoeGfUmCmCmAmGmCmUmUfUmAfUmUmGmsGmsGm | 84 |
| Preparation Example 17 | Conjugate 17 | Sense strand | UmsUmsAmAmAmAmGfGfGfAmCmAmGmUmAmUmUmCmUm | 85 |
| | | Antisense strand | AmsGfsAmoeAmUmAfCmUmGmUmCmCmCmUfUmUfUmAmAmsUmsUm | 86 |
| Preparation Example 18 | Conjugate 18 | Sense strand | CmsCmsAmAmGmAmGfCfAfCmCmAmAmGmAmAmCmUmAm | 87 |
| | | Antisense strand | PUmsAfsGmoeUmUmCfUmUmGmGmUmGmCmUfCmUfUmGmGmsCmsUm | 88 |
| Preparation Example 19 | Conjugate 19 | Sense strand | CmsCmsAmAmGmAmGfCfAfCmCmAmAmGmAmAmCmUmAm | 89 |
| | | Antisense strand | PUmsAfsGmUmTmoeCfUmUmGmGmUmGmCmUfCmUfUmGmGmsCmsUm | 90 |
| Preparation Example 20 | Conjugate 20 | Sense strand | CmsCmsAmAmGmAmGfCfAfCmCmAmAmGmAmAmCmUmAm | 91 |
| | | Antisense strand | PUmsAfsGmTmoeTmoeCfUmUmGmGmUmGmCmUfCmUfUmGmGmsCmsUm | 92 |
| Preparation Example 21 | Conjugate 21 | Sense strand | CmsCmsAmAmGmAmGfCfAfCmCmAmAmGmAmAmCmUmAm | 93 |
| | | Antisense strand | PUmsAfsGmTmoeUmCfUmUmGmGmUmGmCmUfCmUfUmGmGmsCmsUm | 94 |
| Preparation Example 22 | Conjugate 22 | Sense strand | CmsCmsUmUmGmAmGfGfCfAmUmAmCmUmUmCmAmAmAm | 95 |
| | | Antisense strand | VPUmsUmsTmoeGmAmAfGmUmAmoeUmGmCmCmUfCmAfAmGmGmsUmsUm | 96 |

(continued)

| Preparation Example No. | siRNA conjugate No. | Sequence (5'-3') | | SEQ ID NO |
|---|---|---|---|---|
| Preparation Example 23 | Conjugate 23 | Sense strand | CmsCmsUmUmGmAmGfGfCfAmUmAmCmUmUmCmAmAmAm | 97 |
| | | Antisense strand | VPUmsUmsUmGmAmoeAfGmUmAmoeUmGmCmCmUfCmAfAmGmGmsUmsUm | 98 |
| Comparative Preparation Example 4 | Reference Conjugate 4 | Sense strand | CmsCmsUmUmGmAmGfGfCfAmUmAmCmUmUmCmAmAmAm | 99 |
| | | Antisense strand | VPUmsUmsUmGmAmAfGmUmAmUmGmCmCmUfCmAfAmGmGmsUmsUm | 100 |
| Comparative Preparation Example 5 | Reference Conjugate 5 | Sense strand | UmsGmsCmUmAmUmGfCfCfUmCmAmUmCmUmUmCmUmAm | 101 |
| | | Antisense strand | VPUmsAfsGmAmAmGfAmUmGmAmGmGmCmAfUmAfGmCmAmsUmsUm | 102 |
| Comparative Preparation Example 6 | Reference Conjugate 6 | Sense strand | GmsGmsAmCmAmGmUfAfUfUmCmUmCmAmGmUmGmCmUm | 103 |
| | | Antisense strand | PAmsGfsCmAmCmUfGmAmGmAmAmUmAmCfUmGfUmCmCmsCmsUm | 104 |
| Comparative Preparation Example 7 | Reference Conjugate 7 | Sense strand | UmsUmsAmAmAmAmGfGfGfAmCmAmGmUmAmUmUmCmUm | 105 |
| | | Antisense strand | PAmsGfsAmAmUmAfCmUmGmUmCmCmCmUfUmUfUmAmAmsGmsCm | 106 |
| Comparative Preparation Example 8 | Reference Conjugate 8 | Sense strand | CmsAmsAmUmAmAmAfGfCfUmGmGmAmCmAmAmGmAmAm | 107 |
| | | Antisense strand | PUmsUfsCmUmUmGfUmCmCmAmGmCmUmUfUmAfUmUmGmsGmsGm | 108 |
| Comparative Preparation Example 9 | Reference Conjugate 9 | Sense strand | CmsCmsAmAmGmAmGfCfAfCmCmAmAmGmAmAmCmUmAm | 109 |
| | | Antisense strand | PUmsAfsGmUmUmCfUmUmGmGmUmGmCmUfCmUfUmGmGmsCmsUm | 110 |
| Comparative Preparation Example 10 | Reference Conjugate 10 | Sense strand | CmsCmsUmUmGmAmGfGfCfAmUmAmCmUmUmCmAmAmAm | 111 |
| | | Antisense strand | VPUmsUmsUGmAmAfGmUmAmUmGmCmCmUfCmAfAmGmGmsUmsUm | 112 |
| Comparative Preparation Example 11 | Reference Conjugate 11 | Sense strand | CmsCmsAmAmGmAmGfCfAfCmCmAmAmGmAmAmCmUmAm | 113 |
| | | Antisense strand | PUmsAfsGmUmUCfUmUmGmGmUmGmCmUfCmUfUmGmGmsCmsUm | 114 |
| Comparative Preparation Example 12 | Reference Conjugate 12 | Sense strand | CmsCmsUmUmGmAmGfGfCfAmUmAmCmUmUmCmAmAmAm | 115 |
| | | Antisense strand | VPUmsUmsTmoeGmAmAfGmUmAmUmGmCmCmTmoeCmAfAmGmGmsUmsUm | 116 |

(continued)

| Preparation Example No. | siRNA conjugate No. | Sequence (5'-3') | | SEQ ID NO |
|---|---|---|---|---|
| Comparative Preparation Example 13 | Reference Conjugate 13 | Sense strand | CmsCmsUmUmGmAmGfGfCfAmUmAmCmUmUmCmAmAmAm | 117 |
| | | Antisense strand | VPUmsUmsUmGmAmoeAfGmUmAmUmGmCmCmTmoeCmAfAmGmGmsUmsUm | 118 |
| Comparative Preparation Example 14 | Reference Conjugate 14 | Sense strand | CmsCmsUmUmGmAmGfGfCfAmUmAmCmUmUmCmAmAmAm | 119 |
| | | Antisense strand | VPUmsUmsUmGmAmAfGmoeUmAmUmGmCmoeCmUfCmAfAmGmGmsUmsUm | 120 |
| Comparative Preparation Example 15 | Reference Conjugate 15 | Sense strand | UmsGmsCmUmAmUmGfCfCfUmCmAmUmCmUmUmCmUmAm | 121 |
| | | Antisense strand | VPUmsAfsGmAmAmGfAmoeUmGmAmGmGmCmAfUmAfGmCmAmsUmsUm | 122 |

The uppercase letters C, G, U, A, and T represent the base composition of nucleotides; the lowercase letter m indicates that the nucleotide adjacent to the letter m on the left side is a methoxy-modified nucleotide; the lowercase letter f indicates that the nucleotide adjacent to the letter f on the left side is a fluoro-modified nucleotide; the letter combination moe underlined indicates that the nucleotide adjacent to the letter combination moe on the left side is a ribose 2'-O-methoxyethyl-modified nucleotide; the lowercase letter s indicates that the two nucleotides on the left and right sides of the letter s are linked by a phosphorothioate group; VP indicates that the nucleotide adjacent to the letter VP on the right side is a 5'-vinylphosphate-modified nucleotide; P indicates that the nucleotide adjacent to the letter P on the right side is a 5'-phosphate nucleotide.

Comparative Preparation Examples 4-15. Synthesis of reference siRNA conjugates

According to the preparation method described in Preparation Example 1 of CN110959011A, the following reference conjugates designated reference conjugates 4-15 in Table 3 were prepared, the only difference being that the sense strand and antisense strand of the siRNA contained in each reference siRNA conjugate were as shown in Table 3. According to the nucleic acid sequences of the siRNAs designated conjugates 4-15 in Table 3, the sense strands and the antisense strands of the siRNAs were synthesized. Ultrapure water (Milli-Q ultrapure water instrument, resistivity 18.2 M92*cm (25 °C)) was used to dilute each reference siRNA conjugate to a concentration of 0.2 mg/mL (on a siRNA basis), and then a liquid chromatography-mass spectrometry instrument (LC-MS, purchased from Waters Inc., model: LCT Premier) was used for molecular weight determination. The found value was consistent with the calculated value, indicating that the synthesized reference conjugates 4-15 have the target designed double-stranded nucleic acid sequences. The reference siRNA conjugates have a structure represented by formula (403), and the siRNAs contained have the siRNA sequences corresponding to reference conjugates 4-15 in Table 3.

Experimental Example 1. Determination of double-strand melting temperature Tm

Each of the prepared conjugate 5, conjugate 19, reference conjugate 10 and reference conjugate 11 was prepared with 1× PBS buffer into a 0.02 mg/mL solution as a test sample solution, respectively. The test solution was added into a 10 mm path length quartz cuvette on an Agilent cary300 UV spectrophotometer equipped with a thermal program. A temperature-absorbance curve at a wavelength of 260 nm was recorded, wherein the heating rate was 0.5 °C/min, and the temperature was raised from 20.0 °C to 95 °C. The double-strand melting temperature Tm was obtained by calculating the first derivative of the temperature-absorbance curve according to the spectrophotometer manual. The Tm values and ΔTm values are shown in Table 4:

Table 4. Double-strand melting temperature Tm

| Conjugate | Tm (°C) | *ΔTm* (°C) |
|---|---|---|
| Reference conjugate 10 | 65.02 | 0 |
| Conjugate 5 | 66.07 | 1.05 |
| Reference conjugate 11 | 67.27 | 0 |
| Conjugate 19 | 71.22 | 3.95 |

For conjugate 5 and reference conjugate 10,

$$\Delta Tm \text{ value (conjugate to be tested)} = Tm \text{ (conjugate to be tested)} - Tm \text{ (reference conjugate 10)};$$

For conjugate 19 and reference conjugate 11,

$$\Delta Tm \text{ value (conjugate to be tested)} = Tm \text{ (conjugate to be tested)} - Tm \text{ (reference conjugate 11)}.$$

According to the results in Table 4, compared with the case where an unmodified nucleotide is present at the same position, the double-stranded oligonucleotide of the present disclosure comprising a stabilizing modified nucleotide and the conjugate thereof have a higher double-strand melting temperature.

Experimental Example 2. Inhibition activity of siRNAs in the *in vitro* siCHECK system

According to the method described in Kumico Ui-Tei et al., Functional dissection of siRNA sequence by systematic DNA substitution: modified siRNA with a DNA seed arm is a powerful tool for mammalian gene silencing with significantly reduced off-target effect. Nucleic Acids Research, 2008.36(7), 2136-2151, a detection plasmid was constructed, and HEK293A cells were co-transfected with the detection plasmid and siRNAs to be tested. The target sequence inhibition activity of the siRNAs was reflected by the expression level of the dual luciferase reporter gene. Specific steps are as follows:

[1] Construction of detection plasmid

A detection plasmid was constructed using the psiCHECK™-2 (Promega™) plasmid, and the plasmid contained a target sequence 1, i.e., the siRNA target sequence. For the siRNAs to be tested, target sequence 1 is as follows: GACCTTGAGGCATACTTCAAA (SEQ ID NO: 123)

Target sequence 1 is a sequence completely complementary to the antisense strands of the siRNAs tested, so the inhibition effect of each siRNA on target sequence 1 can reflect the abilities of the tested siRNAs to inhibit target gene expression. Target sequence 1 and the complementary sequence thereof were cloned into the Xho I/Not I site of the psiCHECK™-2 plasmid.

[2] Transfection

HEK293A cells (purchased from Nanjing CoBioer Biosciences Co., Ltd.) were cultured at 37 °C in DMEM complete medium (Hyclone Inc.) supplemented with 20% fetal bovine serum (FBS, Hyclone Inc.) and 0.2 vol% of penicillin-streptomycin (Gibco, Invitrogen Inc.) in an incubator containing 5% $CO_2$/95% air.

HEK293A cells were seeded at 8 × $10^3$ cells/well in 96-well plates. When the cell growth density reached 70-80% after 16 h, H-DMEM complete medium was aspirated off the wells, and 80 μL of Opti-MEM medium (GIBCO ) was added to each well for an additional 1.5 h.

The detection plasmid was diluted into 200 ng/μL detection plasmid working solution by DEPC water; each of the following siRNAs was formulated with DEPC water into siRNA working solutions at the following 11 different concentrations: 4000 nM, 1000 nM, 250 nM, 62.5 nM, 15.625 nM, 3.91 nM, 0.977 nM, 0.244 nM, 0.061 nM, 0.0153 nM and 0.0038 nM, respectively, the siRNA used being siRNA1, siRNA2, siRNA3, siRNA4, reference siRNA1 and reference siRNA2 prepared as described above.

For each siRNA, 2A1-2A11 solutions were prepared, and each 2A1-2A11 solution contained 1 μL of the siRNA working solution at the 11 concentrations above, 0.05 μL of detection plasmid working solution (containing 10 ng of the

detection plasmid) and 10 μL of Opti-MEM media.

2B solutions were prepared, each 2B solution containing 0.2 μL of Lipofectamine™ 2000 and 10 μL of Opti-MEM media.

2C solutions were prepared, each 2C solution containing 0.05 μL of the detection plasmid working solution (containing 10 ng of the detection plasmid) and 10 μL of Opti-MEM media.

An aliquot of the 2B solution was mixed with the resulting aliquot of 2A1-2A11 solution for each siRNA, and incubated at room temperature for 20 min, respectively, to obtain transfection complexes 2X1-2X11 for each siRNA.

An aliquot of the 2B solution was mixed with an aliquot of the 2C solution, and incubated at room temperature for 20 min, respectively, to obtain a transfection complex 2X12.

In the culture wells, transfection complexes 2X1-2X11 for each siRNA were added, respectively, and mixed uniformly in an amount of 20 μL/well to obtain transfection complexes for each siRNA at final concentrations of about 40 nM, 10 nM, 2.5 nM, 0.625 nM, 0.15625 nM, 0.0391 nM, 0.00977 nM, 0.00244 nM, 0.00061 nM, 0.000153 nM and 0.000038 nM, respectively. The transfection complexes 2X1-2X11 for each siRNA were transfected into 3 culture wells, respectively, to obtain a co-transfection mixture containing siRNAs, which was designated as a test group.

For each siRNA, transfection complex 2X12 was added into another 3 culture wells in an amount of 20 μL/well to obtain a transfection mixture without siRNA, which was designated as a blank control group.

The co-transfection mixture with siRNA and the co-transfection mixture without siRNA were respectively transfected in culture wells for 4 h, and 100 μL of H-DMEM complete medium containing 20% FBS was added to each well. The 96-well plate was placed in a $CO_2$ incubator and incubated for another 24 h.

[3] Detection

The culture media in the culture wells were aspirated, and 150 μL of Dual-Glo® Luciferase reagents and H-DMEM mixed solution (the volume ratio was 1: 1) were added into each well, mixed fully and uniformly, and incubated at room temperature for 10 min; 120 μL of the mixed solution was transferred onto a 96-well microplate, and the chemiluminescence values (Fir) of Firefly in each culture well on the 96-well microplate was read by using a Synergy II multifunctional microplate reader (BioTek company); 60 μL of Dual-Glo® Stop & Glo® reagent was added into every well of the 96-well microplate again, mixed fully and uniformly, and incubated at room temperature for 10 min; and the chemical luminescence value (Ren) of Renilla in each culture well of the 96-well microplate was read with a microplate reader according to the arrangement of reading Fir.

The luminescence ratio Ratio = Ren / Fir of each well on the 96-well microplate was calculated, the luminescence ratio Ratio (test) or Ratio (control) of each test group or control group being the average value of Ratio of three culture wells; with the luminescence ratio of the control group as the baseline, the luminescence ratio of each test group was normalized to obtain the ratio R of Ratio (test) / Ratio (control), which represented the relative expression level of the *Renilla* reporter gene, that is, the residual activity. Inhibition rate of siRNA to target sequence = (1 - R) × 100%.

According to the relative residual activity of *Renilla* in HEK293A cells transfected with different concentrations of siRNA to be tested, the dose-effect curve of log (inhibitor) vs. response-Variable slope (four parameters) was fitted using the nonlinear regression analysis function of Graphpad 5.0 software.

According to the function corresponding to the fitted dose-effect curve, the $IC_{50}$ value of the target sequence targeted by the siRNA to be tested was calculated, with the function shown as follows:

$$Y = \mathrm{Bot} + \frac{\mathrm{Top} - \mathrm{Bot}}{1 + 10^{(X' - X) \times \mathrm{HillSlope}}}$$

where:

Y is the ratio R, the relative residual activity of *Renilla,*

X is the logarithmic value of transfection siRNA concentration,

Bot is the Y value at the bottom of the steady state period,

Top is the Y value at the top of the steady state period, and

X' is the corresponding X value when Y is halfway between the bottom and the top, and HillSlope is the slope of the curve at X'.

From the dose-effect curve and the corresponding function, the corresponding $X_{50}$ value when Y = 50% was determined, the $IC_{50}$ value of each siRNA was calculated by $IC_{50}$ = 10^$X_{50}$ (nM), and the $IC_{50}$ values are summarized in Table 5.

Table 5. $IC_{50}$ for siRNAs

| siRNA No. | $IC_{50}$ |
|---|---|
| siRNA1 | 0.0029 nM |
| siRNA2 | 0.0086 nM |
| siRNA3 | 0.0022 nM |
| siRNA4 | 0.0022 nM |
| Reference siRNA1 | 0.0036 nM |
| Reference siRNA2 | 0.2851 nM |

According to the results in Table 5, the siRNAs provided by the present disclosure have very high target sequence inhibition activity in the *in vitro* siCHECK system, with $IC_{50}$ of 0.0022-0.0086 nM. Meanwhile, their target sequence inhibition activity was similar to that of reference siRNA1, which contained no stabilizing modified nucleotides. In particular, the $IC_{50}$ values of siRNA1, siRNA3 and siRNA4 were lower than that of reference siRNA1, indicating higher target sequence inhibition effects. In contrast, in the direction from the 5' end to the 3' end, the target sequence inhibition activity of reference siRNA2, which contained a stabilizing modified nucleotide only at the 2nd position of the antisense strand, was greatly reduced, and its $IC_{50}$ value was about 33 times, or even more than 100 times that of reference siRNA1 and the siRNAs of the present disclosure.

Experimental Example 3. Off-target sequence inhibition activity of siRNAs in the *in vitro* siCHECK system

According to the method in Experimental Example 2, the off-target sequence inhibition activity of siRNA3 and reference siRNA1 in the *in vitro* siCHECK system was detected. The only difference was that the target sequence used was target sequence 2 as shown below:
TCAAGGTCTTACGACTTCAAA (SEQ ID NO: 124)

Only part of target sequence 2 is complementary to the antisense strand of the siRNA to be tested, so the inhibition effect of each siRNA on target sequence 2 can reflect the degree of off-target effect. That is, the higher the inhibition effect, the more likely the siRNA is off-target.

From the dose-effect curve and the corresponding function, the corresponding $X_{75}$ value when Y = 75% was determined, and the off-target $IC_{25}$ value = 10^$X_{75}$ (nM) of each siRNA was calculated. After measurement and calculation, the IC25 value of the off-target effect of reference siRNA1 was 1.6255 nM. However, the siRNA3 was not off-target in all concentration ranges detected, and Y was always higher than 75%. Hence, the siRNA of the present disclosure exhibited a significantly lower off-target effect than the reference siRNA.

Experimental Example 4. Target mRNA inhibition activity of siRNAs in mouse primary hepatocytes

HBV transgenic mice C57BL/6J-Tg(A1b1HBV)44Bri/J were purchased from the Department of Experimental Animal Science, Peking University Health Science Center. Before the experiment, mice with S/COV>10 were selected for the experiment, hereinafter referred to as 44Bri mice.

Mouse primary hepatocytes were extracted from fresh liver tissue of 44Bri mice, and the density of the mouse primary hepatocytes was adjusted to $2 \times 10^5$ cells/ mL in Opti-MEM (1X) medium (GIBCO, Cat. No. 31985-070) to obtain the mouse liver primary cell suspension. Then, the obtained mouse liver primary cell suspensions were added to different culture wells of the 12-well plate, and the mouse liver primary cells were inoculated into the culture wells. The volume of the mouse primary hepatocyte suspension added was 0.5 mL/well, and the number of mouse primary hepatocytes was $1 \times 10^5$ cells/well.

Each siRNA in the following siRNAs was prepared into 20 $\mu$M siRNA working solution with DEPC water, and the siRNAs used were siRNA1, siRNA2, siRNA3, siRNA4, reference siRNA1, reference siRNA2 and reference siRNANC.

4A solutions were prepared. For each siRNA, a 4A solution was prepared. Each of the 4A solutions contains 1.5 $\mu$L of the above siRNA working solution and 50 $\mu$L of Opti-MEM media in order.

4B solutions were prepared, each 4B solution containing 1 $\mu$L of Lipofectamine™ 2000 and 50 $\mu$L of Opti-MEM media.

An aliquot of the 4B solution was mixed with the resulting aliquot of 4A solution for each siRNA, and incubated at room temperature for 20 min, respectively, to obtain a transfection complex 4X for each siRNA.

An aliquot of the 4B solution was mixed with 50 μL of Opti-MEM medium and incubated at room temperature for 20 min to obtain a transfection complex 4X'.

In the culture wells, the transfection complexes 4X for each siRNA were added, respectively, and mixed uniformly in an amount of 98 μL/well to obtain transfection complexes for each siRNA at final concentrations of about 50 nM. The transfection complexes 4X for each siRNA were transfected into 3 culture wells, respectively, to obtain a co-transfection mixture containing siRNAs, which was designated as a test group.

The transfection complexes 4X' were added into another 3 culture wells in an amount of 98 μL/well to obtain a transfection mixture without siRNA, which was designated as a blank control group.

The co-transfection mixture containing siRNA and the co-transfection mixture without siRNA were respectively transfected in different culture wells for 4 h, and 1 mL of H-DMEM complete medium containing 20% FBS was added to each well. The 24-well plate was placed in a $CO_2$ incubator and cultured at 37 °C for another 24 h.

Subsequently, TRIZOL (purchased from SIGMA, Cat. No. T9424) was used to extract the total RNA in the cells in each well according to the method described in the instructions to obtain a total RNA aqueous solution.

For each well of cells, the total RNA aqueous solution containing 1 μg of total RNA was taken respectively, and the reagents provided by the reverse transcription kit Goldenstar™ RT6 cDNA Synthesis Kit (purchased from Beijing TsingKe Biotech Co., Ltd., Cat. No. TSK301M) were used, wherein Goldenstar™ Oligo $(dT)_{17}$ was used as a primer, and 20 μL of a reverse transcription reaction system was configured according to the reverse transcription operation steps in the instruction of the kit, and the total RNA of cells in each well was reverse transcribed. The reverse transcription conditions are: for each reverse transcription reaction system, the reverse transcription reaction system was incubated at 50 °C for 50 min, then incubated at 85 °C for 5 min, and finally incubated at 4 °C for 5 min, and 80 μL of DEPC water was added to the system to obtain a solution containing cDNA.

For each reverse transcription reaction system, with 5 μL of the cDNA-containing solution as a template, the reagents provided by NovoStart® SYBR qPCR SuperMix Plus kit (purchased from Novoprotein Scientific Inc., Cat. No. E096-01B) were used to prepare 15 μL of qPCR reaction system, wherein the PCR primer sequences used to amplify the target gene HBV X and the internal reference gene mGAPDH are shown in Table 6, and the final concentration of each primer was 0.25 μM. Each qPCR reaction system was placed on an ABI StepOnePlus Real-Time PCR instrument, and amplified using a three-step method. The amplification program was pre-denaturation at 95 °C for 10 min, followed by denaturation at 95 °C for 30s, annealing at 60 °C for 30 s, and extension at 72 °C for 30 s. After repeating the denaturation, annealing, and extension processes for a total of 40 times, a product W containing amplified target gene HBV X and internal reference gene mGAPDH was obtained. The product W was then incubated at 95 °C for 1 min, 55 °C for 30 s, and 95 °C for 30 s. The real-time fluorescent quantitative PCR instrument collected the melting curves of the target gene HBV X and the internal reference gene mGAPDH in the product W respectively, and obtained the Ct values of the target gene HBV X and the internal reference gene mGAPDH.

Table 6. Primer sequence information

| Gene name | Primer | Nucleotide sequence (5'→3') | SEQ ID NO |
|---|---|---|---|
| HBVX | Forward primer | CCGTCTGTGCCTTCTCATCT | 125 |
| | Reverse primer | TAATCTCCTCCCCCAACTCC | 126 |
| mGAPDH | Forward primer | TGCACCACCAACTGCTTAG | 127 |
| | Reverse primer | GGATGCAGGGATGATGTTC | 128 |

The comparative Ct (ΔΔCt) method was used to carry out relative quantitative calculation of the relative expression level and inhibition rate of the target gene HBV in each test group, and the calculation method was as follows:

ΔCt (test group) = Ct (target gene of test group) - Ct (internal reference gene of test group)

ΔCt (control group) = Ct (target gene of control group) - Ct (internal reference gene of control group)

ΔΔCt (test group) = ΔCt (test group) - ΔCt (average of control group)

ΔΔCt (control group) = ΔCt (control group) - ΔCt (average of control group)

wherein ΔCt (average of control group) is the arithmetic average value of ΔCt (control group) of the three culture

wells of the control group. Therefore, each culture well of the test group and the control group corresponds to a ΔΔCt value.

Taking the control group as a benchmark, the expression level of HBV mRNA in the test group was normalized, and the expression level of HBV mRNA in the blank control group was defined as 100%.

$$\text{HBV mRNA relative expression level of test group} = 2^{-\Delta\Delta Ct(\text{test group})} \times 100\%$$

HBV mRNA inhibition rate of test group = (1 - HBV mRNA relative expression level of test group) × 100%

FIG. 1 is a bar chart showing the relative expression levels of HBV mRNA in primary hepatocytes of 44Bri mice after transfection with siRNA1, siRNA2, siRNA3, siRNA4, reference siRNA1, reference siRNA2 and reference siRNA NC. Further, the inhibition rates of the siRNAs against HBV mRNA are summarized in Table 7.

Table 7. Inhibition of HBV mRNA in mouse primary hepatocytes

| siRNA No. | HBV mRNA inhibition rate (%) |
|---|---|
| siRNA1 | 73.92 |
| siRNA2 | 77.58 |
| siRNA3 | 74.83 |
| siRNA4 | 77.29 |
| Reference siRNA1 | 77.26 |
| Reference siRNA2 | 36.31 |
| Reference siRNA NC | -3.62 |

According to the results in FIG. 1 and Table 7, the siRNAs of the present disclosure showed excellent HBV mRNA inhibition activity in the primary hepatocytes of 44Bri mice. At a siRNA concentration of 50 nM, the HBV mRNA inhibition rate was at least 73.92% and could reach up to 77.58%, and the siRNAs showed similar HBV mRNA inhibition activity to the corresponding reference siRNA1, which contained no stabilizing modified nucleotides. In contrast, in the direction from the 5' end to the 3' end, the target sequence inhibition activity of reference siRNA2, which contained a stabilizing modified nucleotide only at the 2nd position of the antisense strand, was greatly reduced, and the HBV mRNA inhibition rate was only 36.31%.

Experimental Example 5. Target mRNA inhibition activity of siRNA conjugates in mouse primary hepatocytes

HBV transgenic mice C57BL/6J-Tg(A1b1HBV)44Bri/J were purchased from the Department of Experimental Animal Science, Peking University Health Science Center. Before the experiment, mice with S/COV>10 were selected for the experiment, hereinafter referred to as 44Bri mice.

Mouse primary hepatocytes were extracted from fresh liver tissue of 44Bri mice, and the density of the mouse primary hepatocytes was adjusted to 1 × $10^5$ cells/ mL in Opti-MEM (1X) medium (GIBCO, Cat. No. 31985-070) to obtain the mouse primary hepatocyte suspension. Then, the obtained mouse primary hepatocyte suspensions were added to different culture wells of the 12-well plate, and the mouse primary hepatocytes were inoculated into the culture wells. The volume of the mouse primary hepatocyte suspension added was 1 mL/well, and the number of mouse primary hepatocytes was 1 × $10^5$ cells/well.

Each of the following siRNA conjugates was prepared into a 4 μM (based on siRNA) siRNA conjugate working solution with DEPC water, the siRNA conjugates used being conjugate 5, conjugate 6 or reference conjugate 4. The reference siRNA NC was prepared into a 4 μM reference siRNA NC working solution.

The siRNA conjugate working solution or the reference siRNA NC working solution of each conjugate was added to the culture wells of different mouse primary hepatocyte suspensions, and mixed evenly, with adding amount of 2.5 μL/well. Each siRNA conjugate or reference siRNA NC was transfected into 3 culture wells to obtain a transfection

mixture containing siRNA (based on siRNA, with a final concentration of 10 nM), which was designated as the test group. The mouse primary hepatocyte suspension in the other 3 culture wells was designated as the blank control group.

Each transfection mixture containing siRNA and the blank control group were placed in an incubator containing 5% $CO_2$, and incubated at 37 °C for 24 h.

Subsequently, TRIZOL (purchased from SIGMA, Cat. No. T9424) was used to extract the total RNA in the cells in each well according to the method described in the instructions, to obtain a total RNA aqueous solution.

For each well of cells, the total RNA aqueous solution containing 1 μg of total RNA was taken respectively, and the reagents provided by the reverse transcription kit Goldenstar™ RT6 cDNA Synthesis Kit (purchased from Beijing TsingKe Biotech Co., Ltd., Cat. No. TSK301M) were used, wherein Goldenstar™ Oligo $(dT)_{17}$ was used as a primer, and 20 μL of a reverse transcription reaction system was configured according to the reverse transcription operation steps in the instruction of the kit, and the total RNA of cells in each well was reverse transcribed. The reverse transcription conditions are: for each reverse transcription reaction system, the reverse transcription reaction system was incubated at 50 °C for 50 min, then incubated at 85 °C for 5 min, and finally incubated at 4 °C for 5 min, and 80 μL of DEPC water was added to the system to obtain a solution containing cDNA.

For each reverse transcription reaction system, with 5 μL of the cDNA-containing solution as a template, and the reagents provided by NovoStart® SYBR qPCR SuperMix Plus kit (purchased from Novoprotein Scientific Inc., Cat. No. E096-01B) were used to prepare 15 μL of qPCR reaction system, wherein the PCR primer sequences used to amplify the target gene HBV X and the internal reference gene GAPDH are shown in Table 6, and the final concentration of each primer was 0.25 μM. Each qPCR reaction system was placed on an ABI StepOnePlus Real-Time PCR instrument, and amplified using a three-step method. The amplification program was pre-denaturation at 95 °C for 10 min, followed by denaturation at 95 °C for 30 s, annealing at 60 °C for 25 s, and extension at 72 °C for 25 s. After repeating the denaturation, annealing, and extension processes for a total of 40 times, a product W containing amplified target gene HBV X and internal reference gene GAPDH was obtained. The product W was then incubated at 95 °C for 1 min, 55 °C for 30 s, and 95 °C for 30 s. The real-time fluorescent quantitative PCR instrument collected the melting curves of the target gene HBV X and the internal reference gene GAPDH in the product W respectively, and obtained the Ct values of the target gene HBV X and the internal reference gene GAPDH.

According to the method described in Experimental Example 4, the relative expression level and inhibition rate of HBV mRNAin mouse primary hepatocytes were calculated from the Ct value after free intake of each siRNA conjugate. The results are shown in FIG. 2.

FIG. 2 is a bar chart showing the relative expression levels of HBV mRNA in primary hepatocytes of 44Bri mice after free intake of conjugate 5, conjugate 6 or reference conjugate 4 and reference siRNA NC. Further, the inhibition rates of the siRNA conjugates or reference siRNA NC against HBV mRNA are summarized in Table 8.

Table 8. Inhibition of HBV mRNAin mouse primary hepatocytes

| Conjugate No. (siRNA No.) | HBV mRNA inhibition rate (%) |
|---|---|
| Conjugate 5 | 93.06 |
| Conjugate 6 | 91.77 |
| Reference conjugate 4 | 95.93 |
| Reference siRNANC | 6.56 |

According to the results in FIG. 2 and Table 8, the siRNA conjugates provided by the present disclosure showed excellent HBV mRNA inhibition activity in 44Bri mouse primary hepatocytes. At a siRNA concentration of 10 nM, the HBV mRNA inhibition rate was at least 91.77%. In particular, the HBV mRNAinhibition activity of conjugate 5 in mouse primary hepatocytes could reach 93.06%, indicating that conjugate 5 showed similar HBV mRNA inhibition activity to the corresponding reference conjugate 4, which contains no stabilizing modified nucleotides.

Experimental Example 6. Inhibition of HBV mRNA by siRNA conjugates in mice *(in vivo)*

Hepatitis B virus surface antigen diagnostic kit (enzyme-linked immunoassay) (Shanghai Kehua BioEngineering Co., Ltd.) was used to detect the serum HbsAg content of 44Bri mice according to the method recorded in the instructions, and mice with S/COV > 10 were selected, randomly divided into groups, 5 (all were male) per group, and numbered. Each of the mice was subcutaneously injected with conjugate 5, conjugate 6 or reference conjugate 4 at a dose of 1 mg/kg or 0.1 mg/kg mouse body weight (based on siRNA); the siRNA conjugates were provided in the form of solutions in 1× PBS containing 0.2 mg/mL or 0.02 mg/mL (based on siRNA) of siRNA conjugate, and the administration volumes were 5 ml/kg; each of the other two groups of mice was administered with 1 × PBS, and the administration volume was

5 ml/kg, serving as a blank control group.

Taking the administration time point as day 1, the animals were sacrificed on day 8, and the liver tissues of each mouse were collected and preserved in RNA later (Sigma Aldrich); 1 mL of Trizol (Sigma) was added to each liver tissue, crushed in a Tissuelyset II automatic tissue homogenizer for 3 times, each time for 30 s, to obtain liver tissue homogenate, to which 0.2 mL of chloroform was added, and left to stand for 3 min. The mixture was centrifuged at 12000 rpm at 4 °C for 10 min, and 0.4 mL of supernatant was collected. The 0.5 mL of isopropyl alcohol was added to the supernatant, and the mixture was allowed to stand at room temperature for 10 min. The mixture was centrifuged at 12000 rpm at 4 °C for 10 min and the supernatant was discarded. The precipitate was washed with 1 mL of ethanol, centrifuged at 12000 rpm at 4 °C for 5 min, and the supernatant was discarded. To the precipitate, 70 μL of DEPC water was added to obtain a total RNA solution.

For the total RNA of the liver tissue of each mouse, 10.5 μL of the total RNA aqueous solution containing 1 μg of total RNA was prepared into 20 μL of reverse transcription reaction system by using the reverse transcription kit Reverse Transcription System (purchased from Promega, Cat. No. A3500) according to the reverse transcription steps in the instructions of the kit, and the total RNA was reverse transcribed. The reverse transcription conditions are: for each reverse transcription reaction system, the reverse transcription reaction system was incubated at 42°C for 30 min, then incubated at 95°C for 5 min, and finally incubated at 4°C for 5 min, and 80 μL of DEPC water was added to the system to obtain a solution containing cDNA.

For each reverse transcription reaction system, with 5 μL of the cDNA-containing solution as a template, the reagents provided by SYBR select Master Mix kit (purchased from Applied biosystem Inc.) were used to prepare 20 μL of qPCR reaction system, wherein the PCR primer sequences used to amplify the target gene HBV X and the internal reference gene GAPDH are shown in Table 6, and the final concentration of each primer was 0.25 μM. Each qPCR reaction system was placed on an ABI StepOnePlus Real-Time PCR instrument, and amplified using a three-step method. The amplification program was pre-denaturation at 95 °C for 10 min, followed by denaturation at 95 °C for 30 s, annealing at 60 °C for 30 s, and extension at 72 °C for 30 s. After repeating the denaturation, annealing, and extension processes for a total of 40 times, a product W containing amplified target gene HBV X and internal reference gene GAPDH was obtained. The product W was then incubated at 95 °C for 1 min, 55 °C for 30 s, and 95 °C for 30 s. The real-time fluorescent quantitative PCR instrument collected the melting curves of the target gene HBV X and the internal reference gene GAPDH in the product W respectively, and obtained the Ct values of the target gene HBV X and the internal reference gene GAPDH.

According to the method described in Experimental Example 4, the relative expression level and inhibition rate of HBV mRNA in the liver tissue of mouse were calculated from the Ct value after administration of each siRNA conjugate. The results are shown in FIGs. 3A and 3B.

FIGs. 3A and 3B are scatter plots showing the relative expression levels of HBV mRNA in the livers of 44Bri mice after administration of 1 mg/kg or 0.1 mg/kg (based on siRNA) conjugate 5, conjugate 6 or reference conjugate 4 and PBS. In FIGs. 3A and 3B, PBS represents a blank control group. Further, the inhibition rates of the siRNA conjugates against HBV mRNA are summarized in Table 9.

Table 9. Inhibition of HBV mRNA in mice

| Conjugate No. | Dose (based on siRNA, mg/kg) | HBV mRNA inhibition rate (%) |
|---|---|---|
| Conjugate 5 | 1 | 96.31 |
| | 0.1 | 63.18 |
| Conjugate 6 | 1 | 91.83 |
| | 0.1 | 67.57 |
| Reference conjugate 4 | 1 | 97.42 |
| | 0.1 | 73.90 |

As shown in FIGs. 3A, 3B and Table 9, the siRNA conjugates of the present disclosure exhibited excellent HBV mRNA inhibition effects in mice. At a dose of 0.1 mg/kg, the HBV mRNA inhibition rate was at least 63.18%. At a dose of 1 mg/kg, the HBV mRNA inhibition rate could even reach up to 96.31%. In addition, the siRNA conjugates showed similar HBV mRNA inhibition activity to the corresponding reference conjugate 4, which contained no stabilizing modified nucleotides.

Experimental Example 7. Toxic effects of siRNA conjugates in rats

Conjugate 5, conjugate 6, reference conjugate 4 and reference conjugate 14 were dissolved in PBS to obtain a 6 mg/ml solution (based on siRNA conjugate). SD rats (all male, weighing 0.22-0.28 kg, 5-7 weeks old, purchased from Charles River Inc.) were randomly divided into groups, with 5 rats in each group, and numbered respectively. In the manner of subcutaneous injection on the back of the neck, the siRNA conjugate solution was administered to each rat respectively at an administration volume of 5 mL/kg, as a test group. In addition, PBS was administered to each of one group of rats at an administration volume of 5 mL/kg, as a blank control group.

Taking the administration time point as day 1, each rat in the test group and the blank control group was sacrificed on day 15 and dissected, and the livers thereof were weighed and normalized on the basis of the blank control group. The livers were preserved in 10% neutral buffered formalin fixative and pathological sections were made. The results of gross autopsy and liver weight results are shown in Table 10. In the pathological sections, the severity of hepatic steatosis and inflammation were evaluated and graded and compared.

Table 10. Toxic effects of siRNA conjugates in rats

| Experimental group | Blank control | Conjugate 5 | Conjugate 6 | Reference conjugate 4 | Reference conjugate 14 |
|---|---|---|---|---|---|
| Dose | - | 30mg/kg | 30mg/kg | 30mg/kg | 30mg/kg |
| Gross dissection | No abnormalities | No abnormalities | No abnormalities | Liver yellowing and hepatomegaly | Severe liver yellowing and hepatomegaly |
| Liver weight | Normalized 1 | ↑5.43% | ↑3.45% | ↑81.31% | ↑84.03% |

In Table 10, % and the preceding numbers represent the percentage difference in the corresponding index when compared with the reference blank control group. "↑" represents increase. For example, ↑5.43% means that the liver weight is increased by 5.43% compared with the blank control group.

According to the results in Table 10, the rats administered reference conjugate 4 and reference conjugate 14, which contained no conjugates, the increases were up to 81.31% and 84.03%, respectively, showing significant liver weight increases. Further, significant liver yellowing and hepatomegaly were shown in the gross autopsy. However, the liver weights of the rats administered conjugate 5 and conjugate 6 were increased by only 5.45% and 3.45%, respectively, and no significant abnormality was found in the gross autopsy compared with the blank control group.

The results of pathological sections showed that 3 cases of the rats administered reference conjugate 4 had severe hepatic steatosis and mild or moderate liver inflammation, specifically manifested as that extensive and severe fatty degeneration occurred to the hepatocytes in the tissue, cellular round vacuoles of varying numbers and sizes can be seen in the cytoplasm, a small number of hepatocytes were severely swollen, the cytoplasm was lightly stained, and a small amount of inflammatory cell infiltration may be seen around the vein. All rats administered reference conjugate 14 containing a stabilizing modified nucleotide only at position 7 in the 5'-3' direction of the antisense strand showed very severe hepatic steatosis and mild or moderate liver inflammation, specifically manifested as that extensive and severe fatty degeneration occurred to the hepatocytes in the tissue, cellular round vacuoles of varying numbers and sizes can be seen in the cytoplasm, multifocal inflammatory cell infiltrates were visible in the lobules or occasional inflammatory cell infiltrates were visible around portal vein, severe swelling and pale cytoplasmic staining occurred to a small number of hepatocytes.

Among the rats administered conjugate 5, only mild fatty degeneration was found in 4 rats, manifested as tight arrangement of hepatocytes, clear boundaries, mild fatty degeneration of a small number of hepatocytes, and tiny round vacuoles in the cytoplasm. However, no steatosis was found in the other rat, and no significant liver inflammation was found in all rats. Among the rats administered conjugate 6, the degree of steatosis in 2 rats was mild, specifically manifested as that the hepatocytes were closely arranged, the boundary was clear, some hepatocytes had mild fatty degeneration, and round vacuoles may be seen in the cytoplasm. The degree of steatosis of 3 rats was moderate, specifically manifested as that the hepatocytes were closely arranged, the boundary was clear, mild steatosis occurred to many hepatocytes, round vacuoles can be seen in the cytoplasm, and no significant liver inflammation was found in all rats.

The above results show that compared with the reference conjugates, the siRNA conjugates of the present disclosure can effectively reduce the hepatotoxicity caused by off-target effects and therefore show significantly higher safety in the preparation of medicaments for treating and/or preventing HBV diseases or symptoms, having excellent development prospects.

Experimental Example 8. Off-target sequence inhibition activity of siRNA conjugates in the *in vitro* siCHECK system

According to the method described in Experimental Example 2, the off-target sequence inhibition activity of siRNA conjugates in the *in vitro* siCHECK system was determined, the only difference being that conjugate 5, conjugate 6, conjugate 7, conjugate 8, conjugate 22, conjugate 23, reference conjugate 4, reference conjugate 10, reference conjugate 12 or reference conjugate 13 was determined instead of the tested siRNA; and, the target sequence used was target sequence 3 as shown below:

GGCCGCATTGAAGTTACTGATCCTTCCAAATTGAAGTTACTGATCCTTCCAAATTGAAGTTACTGATCCTTCCAAATTGAAGTTACTGATCCTTCCAAATTGAAGTTACTGATCCTTC (SEQ ID NO: 129)

Multiple sequences in target sequence 3 are complementary to the siRNA antisense strand of the siRNA conjugate to be tested, so the inhibition effect of each siRNA conjugate on target sequence 3 can reflect the degree of off-target effect. That is, the higher the inhibition effect, the more likely the siRNA conjugate is off-target.

From the dose-effect curve and the corresponding function, the corresponding $X_{50}$ value when Y = 50% was determined, and the off-target $IC_{50}$ value = $10^{\wedge}X_{50}$ (nM) of each siRNA conjugate was calculated.

As a result, the off-target $IC_{50}$ value of reference conjugate 4 was 218.085pM, and the off-target $IC_{50}$ value of reference conjugate 10 was 202.581pM, that is, when the two reference siRNA conjugates were at concentrations above the off-target IC, the same were off-target significantly. Compared with this, the off-target $IC_{50}$ value of conjugate 22 was 555.240pM, which was significantly higher than that of the reference siRNA conjugate. In particular, the relative residual activities of Renilla values of the other siRNA conjugates in the entire test concentration range was always higher than 50%, among which, the relative residual activities of Renilla of conjugate 5, conjugate 6, conjugate 7, conjugate 8 and conjugate 23 were 55.65%, 71.42%, and 71.38%, 65.47% and 67.84%, respectively, that is, none of the siRNA conjugates was off-target. Hence, each of the siRNA conjugates containing stabilizing modified nucleotides showed significantly lower off-target effects than reference conjugate 4 and reference conjugate 10, which contained no stabilizing modified nucleotides.

Experimental Example 9. Target mRNA inhibition activity of siRNA conjugates in mouse primary hepatocytes

According to the method described in Experimental Example 5, the target mRNA inhibition activity of siRNA conjugates in mouse primary hepatocytes was determined, the only difference being that conjugate 5, conjugate 6, conjugate 22, conjugate 23, reference conjugate 4, reference conjugate 10, reference conjugate 12, reference conjugate 13, or reference siRNA NC was subjected to determination in place of the tested siRNA conjugates, and each conjugate, the reference conjugate or reference siRNA NC was tested in 2 culture wells. The results are shown in FIG. 4.

FIG. 4 is a bar chart showing the relative expression levels of HBV mRNA in primary hepatocytes of 44Bri mice after free intake of conjugate 5, conjugate 6, conjugate 22, conjugate 23, reference conjugate 4, reference conjugate 10, reference conjugate 12, reference conjugate 13 or reference siRNANC. Further, the inhibition rates of the siRNA conjugates or reference siRNANC against HBV mRNA are summarized in Table 11.

Table 11. Inhibition of HBV mRNA in mouse primary hepatocytes

| Conjugate No. (siRNA No.) | HBV mRNA inhibition rate (%) |
|---|---|
| Conjugate 5 | 82.94 |
| Conjugate 6 | 82.93 |
| Conjugate 22 | 78.47 |
| Conjugate 23 | 85.97 |
| Reference conjugate 4 | 86.69 |
| Reference conjugate 10 | 62.98 |
| Reference conjugate 12 | -6.81 |
| Reference conjugate 13 | 32.27 |
| Reference siRNANC | -10.97 |

According to the results in FIG. 4 and Table 11, siRNA conjugates 5, 6, 22 and 23 of the present disclosure exhibited excellent HBV mRNA inhibition activity in primary hepatocytes of 44Bri mice. At a siRNA concentration of 10 nM, the HBV mRNA inhibition rate was at least 78.47% and could reach up to 85.97%. Their HBV mRNA inhibition activity was comparable to that of reference conjugate 4, and was significantly higher than that of reference conjugates 10, 12 and 13. Among them, a non-stabilizing modified nucleotide is at the corresponding position in reference conjugate 4, an unmodified nucleotide is at the corresponding position in reference conjugate 10, and reference conjugates 12 and 13 further comprise stabilizing modified nucleotides at the positions other than 3rd-9th positions of the antisense strand in the direction from the 5' end to the 3' end.

Experimental Example 10. Inhibition of HBV mRNA by siRNA conjugates in mice *(in vivo)*

According to the method described in Experimental Example 6, the inhibition effect of siRNA conjugates on HBV mRNA in mice was tested, the only difference being that conjugate 10 or reference conjugate 5 were tested instead of the siRNA conjugate used. The results are shown in FIG. 5.

FIG. 5 is a scatter plot showing the relative expression levels of HBV mRNA in the livers of 44Bri mice after administration of 1 mg/kg or 0.1 mg/kg (based on siRNA) conjugate 10 or reference conjugate 5 and PBS. In the figure, PBS represents a blank control group. Further, the inhibition rates of the siRNA conjugates against HBV mRNA are summarized in Table 12.

Table 12. Inhibition of HBV mRNA in mice

| Conjugate No. | Dose (based on siRNA, mg/kg) | HBV mRNA inhibition rate (%) |
|---|---|---|
| Conjugate 10 | 1 | 84.25 |
| | 0.1 | 46.26 |
| Reference conjugate 5 | 1 | 89.17 |
| | 0.1 | 60.43 |

As shown in FIG. 5 and Table 12, the siRNA conjugate of the present disclosure exhibited an excellent HBV mRNA inhibition effect in mice. At a dose of 0.1 mg/kg, the HBV mRNA inhibition rate was at least 46.26%. In particular, at a dose of 1 mg/kg, the HBV mRNA inhibition rate of conjugate 10 in mice could even reach up to 84.25%. At the same concentration, the conjugate showed similar HBV mRNA inhibition activity to the corresponding reference conjugate 5, which contained no stabilizing modified nucleotides.

Experimental Example 11. Toxic effects of siRNA conjugates in mice

Conjugate 9, conjugate 10, reference conjugate 5 and reference conjugate 15 were dissolved in PBS to obtain a 3 mg/ml solution (based on siRNA conjugate). ICR mice (half were male and half female, weighing 18-22 g, 5-6 weeks old, purchased from SiPeiFu Inc.) were randomly divided into groups, 6 (half were male and half female) per group, and numbered. In the manner of subcutaneous injection on the back of the neck, the siRNA conjugate solution was administered to each mouse respectively at an administration volume of 10 mL/kg, as a test group. In addition, PBS was administered to each of one group of mice at an administration volume of 10 mL/kg, as a blank control group.

Taking the administration time point as day 1, on day 15, each mouse in the test group and the blank control group was subjected to orbital blood collection. The blood collection volume was 0.6 mL. The blood was incubated at 37 °C for 60 min and then centrifuged at 3000 rpm at 4 °C for 15 min to obtain serum. The concentrations of alanine aminotransferase (ALT) and aspartate aminotransferase (AST) in serum were further detected by PM1P000/3 automatic serum biochemical analyzer (SABA, Italy). The results are shown in FIG. 6A and FIG. 6B. In these two figures, PBS represents a blank control group.

FIGs. 6A and 6B are scatter plots showing the ALT and AST concentrations in mouse serum after administration of conjugate 9, conjugate 10, reference conjugate 5, reference conjugate 15 or PBS at 30 mg/kg. As shown in FIGs. 6A and 6B, compared with the blank control group, after administration of reference conjugate 5, which contained no stabilizing modified nucleotides, the serum ALT and AST concentrations increased; after administration of the siRNA conjugates of the present disclosure, the serum ALT and AST concentrations were comparable to that of the blank control group, indicating that the siRNA conjugates of the present disclosure have low hepatotoxicity. In another aspect, after administration of reference conjugate 15, which contained a stabilizing modified nucleotide only at the 7th position of the antisense strand in the direction from the 5' end to the 3' end, the ALT and AST concentrations in mouse serum significantly increased, indicating that this reference conjugate may produce higher hepatotoxicity.

Further, after blood collection on day 15, the mice were sacrificed and dissected, preserved in 10% neutral buffered formalin fixative, and pathological sections were made. In the pathological sections, the severity of hepatic steatosis was evaluated and graded and compared.

The results of pathological sections showed that compared with the blank control group, among the mice administered reference conjugate 5, which contained no stabilizing modified nucleotides, 4 mice exhibited severe hepatocyte degeneration, specifically manifested that the cytoplasm of the hepatocytes in many tissues was loose, and ballooning degeneration occurred to many hepatocytes, the cells were swollen, and the cytoplasm was vacuolated. One mouse showed moderate hepatocyte degeneration, specifically manifested that many hepatocyte cytoplasms were loose and stained, vacuolar degeneration occurred to some hepatocytes, tiny round vacuoles were visible in the cytoplasms, a small number of local hepatocytes were necrotic and the nuclei were condensed or fragmented. One mouse showed mild hepatocyte degeneration, specifically manifested that many hepatocyte cytoplasms were loose and stained, which showed more severe hepatic steatosis than the blank control group.

Compared with the blank control, 2 of the mice administered reference conjugate 15 containing a stabilizing modified nucleotide only at the 7th position of the antisense strand in the direction from the 5' end to the 3' end showed severe hepatic steatosis, four showed moderate hepatic steatosis and also showed more severe hepatic steatosis than the mice in the blank control group.

Among the mice administered siRNA conjugate 9 of the present disclosure, 2 mice showed moderate hepatic steatosis, 3 mice showed mild hepatic steatosis, and no severe or worse hepatocyte degeneration was observed. Among the mice administered siRNA conjugate 10 of the present disclosure, 3 mice showed moderate hepatic steatosis, 2 mice showed mild hepatic steatosis, and no severe or worse hepatocyte degeneration was observed. Mice administered the conjugates of the present disclosure showed a lower degree of hepatic steatosis compared with the reference conjugate.

The above results show that compared with the reference conjugates, the siRNA conjugates of the present disclosure can effectively reduce the hepatotoxicity caused by off-target effects and therefore show significantly higher safety in the preparation of medicaments for treating and/or preventing HBV diseases or symptoms, having excellent development prospects.

Experimental Example 12. Inhibition activity of siRNA conjugates in the *in vitro* siCHECK system In this Experimental Example, the *in vitro* siCHECK system was used to detect the inhibition activity of conjugate 11, conjugate 12, conjugate 13, conjugate 14, conjugate 15, conjugate 16, reference conjugate 6, reference conjugate 7, reference conjugate 8 or reference siRNA NC on the target sequence in the *in vitro* siCHECK system.

[1] Construction of detection plasmid

A detection plasmid was constructed using the psiCHECK™-2 (Promega™) plasmid, and the plasmid contained a target sequence 4, i.e., the siRNA target sequence. For the siRNA conjugate to be tested, target sequence 4 is as follows:

TGCTCAGTTCATCCCTAGAGGCAGCTGCTCCAGGAACAGAGGTGCCATGCAGCCCCGGG
TACTCCTTGTTGTTGCCCTCCTGGCGCTCCTGGCCTCTGCCCGAGCTTCAGAGGCCGAG
GATGCCTCCCTTCTCAGCTTCATGCAGGGTTACATGAAGCACGCCACCAAGACCGCCAA
GGATGCACTGAGCAGCGTGCAGGAGTCCCAGGTGGCCCAGCAGGCCAGGGGCTGGGT
GACCGATGGCTTCAGTTCCCTGAAAGACTACTGGAGCACCGTTAAGGACAAGTTCTCTG
AGTTCTGGGATTTGGACCCTGAGGTCAGACCAACTTCAGCCGTGGCTGCCTGAGACCTC
AATACCCCAAGTCCACCTGCCTATCCATCCTGCGAGCTCCTTGGGTCCTGCAATCTCCAG
GGCTGCCCCTGTAGGTTGCTTAAAAGGGACAGTATTCTCAGTGCTCTCCTACCCCACCTC
ATGCCTGGCCCCCCTCCAGGCATGCTGGCCTCCCAATAAAGCTGGACAAGAAGCTGCTA
TG (SEQ ID NO: 130)

Target sequence 4 is a nucleotide sequence in the mRNA expressed by the human ApoC3 gene targeted by the tested siRNA, so the inhibition effect of each siRNA conjugate on the target sequence 4 may reflect the abilities of the tested siRNA conjugate to inhibit target gene expression. Target sequence 4 and the complementary sequence thereof were cloned into the Xho I/Not I site of the psiCHECK™-2 plasmid.

[2] Transfection

HEK293A cells (purchased from Nanjing CoBioer Biosciences Co., Ltd.) were cultured at 37 °C in DMEM complete medium (Hyclone) supplemented with 20% fetal bovine serum (FBS, Hyclone) and 0.2% by volume of penicillin-streptomycin (Gibco, Invitrogen) in an incubator containing 5% $CO_2$/95% air.

HEK293A cells were seeded at 8 $\times$ $10^3$ cells/well in 96-well plates. When the cell growth density reached 70-80% after 16 h, H-DMEM complete medium was aspirated off the wells, and 80 μL of Opti-MEM medium (GIBCO) was added to each well for an additional 1.5 h.

DEPC water was used to dilute the detection plasmid into 200 ng/μL detection plasmid working solution; DEPC water was used to prepare each of the following siRNA conjugates or reference siRNA NC into three kinds of siRNA conjugate working solution or reference siRNA NC working solution with three different concentrations (based on siRNA): 10 nM, 3 nM, and 1 nM. The siRNA conjugates used were conjugate 11, conjugate 12, conjugate 13, conjugate 14, conjugate 15, conjugate 16, reference conjugate 6, reference conjugate 7 and reference conjugate 8 prepared.

For each siRNA conjugate or reference siRNA NC, 12A1-12A3 solutions were prepared, and each 12A1-12A3 solution contained 1 μL of the 3 concentrations of siRNA conjugate working solution or reference siRNA NC working solution, 0.05 μL of detection plasmid working solution (containing 10 ng of the detection plasmid) and 10 μL of Opti-MEM medium.

12B solutions were prepared, each 12B solution containing 0.2 μL of Lipofectamine™ 2000 and 10 μL of Opti-MEM media.

12C solutions were prepared, each 12C solution containing 0.05 μL of the detection plasmid working solution (containing 10 ng of the detection plasmid) and 10 μL of Opti-MEM media.

An aliquot of the 12B solution was mixed with an aliquot of the resulting 12A1-12A3 solutions of each siRNA conjugate or reference siRNA NC, and incubated at room temperature for 20 min to obtain transfection complexes 12X1-12X3 for each siRNA conjugate or reference siRNA NC.

An aliquot of the 12B solution was mixed with an aliquot of the 12C solution, and incubated at room temperature for 20 min, respectively, to obtain a blank transfection complex 12X4.

Transfection complexes 12X1-12X3 for each siRNA conjugate or reference siRNA NC were added to the culture wells in an amount of 20 μL/well, respectively, and mixed uniformly to obtain transfection complexes for each siRNA conjugate or reference siRNA NC at final concentrations of about 0.1 nM, 0.03 nM and 0.01 nM (based on siRNA). The transfection complexes 12X1-12X3 for each siRNA conjugate or reference siRNA NC were transfected into 3 culture wells, respectively, to obtain a co-transfection mixture containing siRNA conjugates or reference siRNA NC, which was designated as a test group.

For each siRNA conjugate or reference siRNA NC, the transfection complex 12X4 was added into another 3 culture wells in an amount of 20 μL/well to obtain a transfection mixture without siRNA, which was designated as a blank control group.

The co-transfection mixture with siRNA and the co-transfection mixture without siRNA were respectively transfected in culture wells for 4 h, and 100 μL of H-DMEM complete medium containing 20% FBS was added to each well. The 96-well plate was placed in a $CO_2$ incubator and incubated for another 24 h.

[3] Detection

The culture media in the culture wells were aspirated, and 150 μL of Dual-Glo® Luciferase reagents and H-DMEM mixed solution (the volume ratio was 1: 1) were added into each well, mixed fully and uniformly, and incubated at room temperature for 10 min; 120 μL of the mixed solution was transferred onto a 96-well microplate, and the chemiluminescence values (Fir) of Firefly in each culture well on the 96-well microplate was read by using a Synergy II multifunctional microplate reader (BioTek company); 60 μL of Dual-Glo® Stop & Glo® reagent was added into every well of the 96-well microplate again, mixed fully and uniformly, and incubated at room temperature for 10 min; and the chemical luminescence value (Ren) of Renilla in each culture well of the 96-well microplate was read with a microplate reader according to the arrangement of reading Fir.

The luminescence ratio Ratio = Ren / Fir of each well on the 96-well microplate was calculated, the luminescence ratio Ratio (test) or Ratio (control) of each test group or control group being the average value of Ratio of three culture wells; with the luminescence ratio of the control group as the baseline, the luminescence ratio of each test group was normalized to obtain the ratio R of Ratio (test) / Ratio (control), which represented the relative expression level of the *Renilla* reporter gene, that is, the residual activity. Inhibition rate of each siRNA conjugate or reference siRNA NC against target sequence 4 = (1 - R) $\times$ 100%.

The inhibition effect of each siRNA conjugate or reference siRNA NC on target sequence 4 is shown in FIG. 7. FIG. 7 is a bar chart showing the relative expression levels of target sequence 4 in the *in vitro* siCHECK system after co-transfection with plasmids containing target sequence 4 and siRNA conjugates to be tested or reference siRNANC.

Further, the expression inhibition rates of the siRNA conjugates or reference siRNA NC against target sequence 4 are summarized in Table 13.

Table 13. Target sequence 4 expression inhibition rates in the *in vitro* siCHECK system

| siRNA conjugate No. (reference siRNA No.) | Target sequence 4 expression inhibition ratio (%) | | |
|---|---|---|---|
| | 0.1 nM | 0.03 nM | 0.01 nM |
| Conjugate 11 | 85.17 | 66.64 | 38.92 |
| Conjugate 12 | 88.32 | 74.61 | 52.08 |
| Reference conjugate 6 | 88.80 | 73.81 | 45.12 |
| Conjugate 13 | 88.72 | 77.90 | 55.52 |
| Conjugate 14 | 88.50 | 77.85 | 52.61 |
| Reference conjugate 7 | 91.04 | 82.47 | 64.65 |
| Conjugate 15 | 89.35 | 83.33 | 67.54 |
| Conjugate 16 | 84.73 | 74.36 | 50.13 |
| Reference conjugate 8 | 86.20 | 78.95 | 59.69 |
| Reference siRNA NC | 3.15 | -11.56 | 8.68 |

According to the results in FIG. 7 and Table 13, the siRNA conjugates provided by the present disclosure have very high target sequence inhibition activity in the *in vitro* siCHECK system. At a low concentration of 0.01 nM, the target sequence 4 expression inhibition rate was at least 38.92 nM and could reach up to 67.54%. At a concentration of 0.1 nM, the target sequence 4 expression inhibition rate was at least 84.73% and could reach up to 89.35%. Meanwhile, the siRNA conjugates have similar target sequence inhibition activity to reference conjugate 6, reference conjugate 7 or reference conjugate 8, which contained no stabilizing modified nucleotides.

Experimental Example 13. Inhibition activity of siRNA conjugates in the *in vitro* siCHECK system According to the method in Experimental Example 2, the inhibition activity of conjugate 11, conjugate 12 and reference conjugate 6 in the *in vitro* siCHECK system was tested, the only difference being that conjugate 11, conjugate 12 or reference conjugate 6 was used tested instead of the tested siRNA. The target sequence used in the detection plasmid was the following target sequence 5; and DEPC water was used to prepare each siRNA conjugate into siRNA conjugate working solutions at the following 11 different concentrations (all based on siRNA): 1.00 μM, 0.330 μM, 0.110 μM, 0.0370 μM, 0.0123 μM, 0.00412 μM, 0.00137 μM, 0.000457 μM, 0.000152 μM, 0.0000508 μM and 0.0000169 μM.

Target sequence 5:

TTGCTTAAAAGGGACAGTATTCTCAGTGCTCTCCTACC (SEQ ID NO: 131)

Target sequence 5 is the exact complement of the antisense strand of the siRNA in each conjugate tested.

The measured $IC_{50}$ values are summarized in Table 14.

Table 14. $IC_{50}$ of siRNA conjugates in the pscheck system

| Conjugate No. | $IC_{50}$ |
|---|---|
| Conjugate 11 | 8.55 pM |
| Conjugate 12 | 6.89 pM |
| Reference conjugate 6 | 6.68 pM |

According to the results in Table 14, the siRNA conjugates of the present disclosure have very high target sequence inhibition activity in the *in vitro* siCHECK system, with $IC_{50}$ of 6.89-8.55 pM. Meanwhile, the siRNA conjugates have similar target sequence inhibition activity to reference conjugate 6, which contained the same sequence but no stabilizing modified nucleotides.

Experimental Example 14. Lowering effects of siRNA conjugates on blood lipid in mice (*in vivo*)

Human APOC3 transgenic mice Tg(APOC3)3707Bres (purchased from Jackson Laboratory, USA) with serum TG content > 2 mmol/L were selected and randomly divided into groups, 6 mice per group, half were male and half female. Conjugate 12, reference conjugate 6 and PBS blank control were administered to each group of mice respectively. All animals were dosed according to their body weight, and administered in a single subcutaneous injection. The dose of each siRNA conjugate (based on the amount of siRNA) was 3 mg/kg and 1 mg/kg mouse body weight, and the administration volume was 5 ml/kg. Each siRNA conjugate was provided in PBS aqueous solution, and the concentration that the conjugate should be prepared was calculated according to the dose and volume of administration. Each of the mice in the other group was administered 1× PBS with a volume of 5 ml/kg, serving as a blank control group.

The time point of administration was recorded as day 1, and blood was collected from the orbital venous plexus of the mice on days 1, 9, 15, 22, 29, 36, and 50, with 100 μL each time. The collected blood was left at room temperature for 30 min, and then centrifuged at 3000 rpm at 4 °C for 15 min to obtain serum. PM1P000/3 automatic serum biochemical analyzer (SABA, Italy) was then used to detect the contents of total cholesterol (CHO) and triglyceride (TG) in serum.

Standardized blood lipid level = (blood lipid content of test group after administration/blood lipid content of test group before administration) × 100%.

Inhibition rate of blood lipid level = (1 - blood lipid content of test group after administration/blood lipid content of test group before administration) × 100%.

Blood lipid refers to total cholesterol (CHO) or triglyceride (TG).

FIGs. 8A and 8B are line graphs showing changes in the level of serum TG or serum CHO over time after administration of a siRNA conjugate of the present disclosure, a reference siRNA conjugate or PBS. Further, the serum TG inhibition rates and serum CHO inhibition rates at these time points are summarized in Table 15Aand Table 15B:

Table 15A. Serum TG inhibition rate of siRNA conjugates in transgenic mice

| Group | Dose mg/kg | TG level inhibition rate (%) | | | | | |
|---|---|---|---|---|---|---|---|
| | | D9 | D15 | D22 | D29 | D36 | D50 |
| Blank control | / | 8.2 | 19.2 | -3.6 | 3.5 | 3.7 | -16.3 |
| Reference conjugate 6 | 3 | 88.2 | 89.0 | 88.6 | 85.9 | 86.9 | 85.4 |
| | 1 | 86.1 | 86.5 | 83.7 | 78.1 | 80.5 | 70.0 |
| Conjugate 12 | 3 | 90.2 | 88.6 | 88.2 | 86.5 | 86.1 | 82.7 |
| | 1 | 83.9 | 80.8 | 76.5 | 74.3 | 67.0 | 54.0 |

Table 15B. Serum CHO inhibition rates of siRNA conjugates in transgenic mice

| Group | Dose mg/kg | CHO level inhibition rate (%) | | | | | |
|---|---|---|---|---|---|---|---|
| | | D9 | D15 | D22 | D29 | D36 | D50 |
| Blank control | / | -1.9 | 9.5 | 2.8 | 2.1 | -1.5 | -1.3 |
| Reference conjugate 6 | 3 | 52.2 | 54.8 | 56.3 | 53.2 | 50.8 | 53.2 |
| | 1 | 47.5 | 44.2 | 39.5 | 42.8 | 44.6 | 41.5 |
| Conjugate 12 | 3 | 51.4 | 43.8 | 53.2 | 47.6 | 46.0 | 47.3 |
| | 1 | 51.8 | 45.8 | 51.2 | 45.5 | 40.7 | 39.1 |

According to the results in FIGs. 8A and 8B and Tables 15A and 15B, at different time points after administration, conjugate 12 can significantly reduce the levels of TG and CHO in mouse serum, and showed a similar blood lipid level lowering effect to the corresponding reference conjugate 6, which contained no stabilizing modified nucleotides. In particular, at a dose of 3 mg/kg, conjugate 12 has always shown a very high blood lipid TG lowering effect throughout the administration period of up to 50 days, and the highest inhibition rate can reach up to 90.2%.

Experimental Example 15. Toxic effects of siRNA conjugates in mice

Conjugate 11, conjugate 12, and reference conjugate 6 were dissolved in PBS to obtain a 10 mg/mL solution and a 30 mg/mL solution (based on siRNA conjugate). ICR mice (half were male and half female, weighing 18-22 g, 5-6 weeks old, purchased from SiPeiFu Inc.) were randomly divided into groups and numbered. For each conjugate at each concentration, the animals were divided into a 2-week group and a 4-week group, with 6 mice in each group (half were male and half female). In the manner of subcutaneous injection on the back of the neck, the siRNA conjugate solution was administered to each mouse at an administration volume of 10 mL/kg, as a test group. In addition, PBS was administered to each one in the two groups of mice at an administration volume of 10 mL/kg, as the 2-week group and 4-week group in the blank control group.

Taking the administration time point as day 1, 6 mice in the 2-week group in the test group and the blank control group were subjected to orbital blood collection on day 15, and 6 mice in the 4-week group in the test group and the blank control group were subjected to orbital blood collection on day 29. The blood collection volume was 0.6 mL, and the collected blood was incubated at 37 °C for 60 min and then centrifuged at 3000 rpm at 4 °C for 15 min to obtain serum. The concentrations of alanine aminotransferase (ALT) and aspartate aminotransferase (AST) in serum was further detected by PM1P000/3 automatic serum biochemical analyzer (SABA, Italy), and compared to that of the blank control group, with results shown in Table 16. Further, after blood collection on day 15, the 6 mice administered 300 mg/kg conjugate from the 2-week group were sacrificed and dissected, and preserved in 10% neutral buffered formalin fixative, and pathological sections were made. The severity of inflammatory cell infiltration and hepatocyte necrosis in the pathological sections was assessed, graded and compared.

Among them, ALT: M: 37% means that the average value of alanine aminotransferase in male mouse serum is 37% higher than that of the blank control group; (M: grade 1 1/3) means that one of all 3 male mice has a mild response, and (F: grade 3 3/3) means that all 3 female mice show a severe response, and so on. "-" means no significant abnormality, and "N/A" means no test.

Table 16. Blood biochemical and liver histopathological results of mice administered siRNA conjugates

| Experimental group | | Reference conjugate 6 | | Conjugate 11 | | Conjugate 12 | |
|---|---|---|---|---|---|---|---|
| Dose | | 100mg/kg | 300mg/kg | 100mg/kg | 300mg/kg | 100mg/kg | 300mg/kg |
| ALT | D29 | - | F:212% | - | - | - | F:130% |
| AST | D29 | - | F:36% | - | - | - | F:32% |

In Table 16, F represents a female mouse, and % and the preceding figures represent the difference between the concentration of alanine aminotransferase (ALT) or aspartate aminotransferase (AST) in the mouse serum and the concentration in the serum of the blank control group, the percentage of the concentration in the serum of the blank control group. For example, F: 212% in Table 16 means that the concentration of alanine aminotransferase in female mice administered reference conjugate 6 at 300 mg/Kg was 212% higher than that in the blank control group.

According to the results in Table 16, compared with the blank control, mice administered reference conjugate 6, which contained no stabilizing modified nucleotides, showed significant changes in blood biochemical indicators. At a high dose of 300 mg/Kg, the concentrations of ALT and AST in female mice were increased by 212% and 36%, respectively. At the same dose, the concentrations of ALT and AST in female mice administered conjugate 12 of the present disclosure were increased by only 130% and 32%, respectively. Compared with reference conjugate 6, the concentration of ALT decreased significantly. No increase was found in the concentrations of ALT and AST in the mice administered conjugate 11 of the present disclosure, and the degree of change in the blood biochemical indicators was significantly reduced compared with reference conjugate 6.

The results of pathological sections showed that compared with the blank control, the mice administered reference conjugate 6, which contained no stabilizing modified nucleotides, showed significant liver inflammation, and all 6 mice showed inflammatory cell infiltration. Whereas only 3 mice among the mice administered conjugate 11 showed inflammatory cell infiltration. Only 3 of the mice administered conjugate 12 showed inflammatory cell infiltration. Compared with the reference conjugate, there was also a significant reduction in the number of mice suffering from inflammatory cell infiltration after being administered the conjugates of the present disclosure.

The above results show that compared with the reference conjugates, the siRNA conjugates of the present disclosure can effectively reduce the hepatotoxicity caused by off-target effects and therefore show significantly higher safety in the preparation of medicaments for treating and/or preventing dyslipidemia-related diseases or symptoms, having excellent development prospects.

Experimental Example 16. Inhibition activity of siRNA conjugates in the *in vitro* siCHECK system The off-target

sequence inhibition activity of conjugate 11 and conjugate 13 in the *in vitro* siCHECK system was detected according to the method in Experimental Example 13. The only difference is that conjugate 11 or conjugate 13 was tested instead of the tested siRNA conjugate. For conjugate 11, the target sequence used was target sequence 5, or target sequences 6-8 as shown below were used instead of target sequence 5. For conjugate 13, the target sequence used was target sequence 5, or target sequences 9-11 as shown below were used instead of target sequence 5, respectively:

Target sequence 6:
TAGGCCCCTTTCAAGTATTCT (SEQ ID NO: 132)

Target sequence 7:
AGAATACTGTCCCTTTTAAGC (SEQ ID NO: 133)

Target sequence 8:
CTCCGCAGTGAAATTTTAAGC (SEQ ID NO: 134)

Target sequence 9:
CTTTCACTGCGGATCAGTGCT(SEQ ID NO: 135)

Target sequence 10:
AGCACTGAGAATACTGTCCCT (SEQ ID NO: 136)

Target sequence 11:
CTACAGTCTCCGCCTGTCCCT (SEQ ID NO: 137)

Among them, target sequence 5 contains a sequence that is completely complementary to the antisense strands of the siRNAs in conjugate 11 and conjugate 13, so the inhibition effect of conjugate 11 or 13 on target sequence 5 can reflect the ApoC3 mRNA inhibition activity of conjugate 11 or 13. Target sequence 6 contains a sequence that is partially complementary to the antisense strand of the siRNA in conjugate 11, target sequence 7 contains a sequence that is completely complementary to the sense strand of the siRNA in conjugate 11, and target sequence 8 contains a sequence that is partially complementary to the sense strand of the siRNA in conjugate 11. Therefore, the inhibition effect of conjugate 11 on target sequence 6, 7 or 8 may reflect the degree of off-target effect. That is, the higher the inhibition effect, the more likely conjugate 11 is off-target. Similarly, target sequence 9 contains a sequence that is partially complementary to the antisense strand of siRNA in conjugate 13, target sequence 10 contains a sequence that is completely complementary to the sense strand of siRNA in conjugate 13, and target sequence 11 contains a sequence that is partially complementary to the sense strand of siRNA in conjugate 13. Therefore, the inhibition effect of conjugate 13 on target sequence 9, 10 or 11 may reflect the degree of off-target effect. That is, the higher the inhibition effect, the more likely conjugate 13 is off-target.

As a result, in the *in vitro* siCHECK system, the inhibitory $IC_{50}$ of conjugate 11 on target sequence 5 was 11.3 pM, and the inhibition rate on target sequence 6, 7 or 8 was less than 50% within the concentration ranges of all tested siRNAs, that is, none of them was off the target. The inhibitory $IC_{50}$ of conjugate 13 on target sequence 5 was 4.50 pM, and the inhibition rate on target sequence 9, 10 or 11 was less than 50% within the concentration ranges of all tested siRNAs, that is, none was off the target.

Hence, in the *in vitro* siCHECK system, the siRNA conjugates of the present disclosure exhibited excellent target sequence on-target inhibition activity, with an $IC_{50}$ value of 4.50 pM-11.3 pM. Meanwhile, the siRNA conjugates of the present disclosure have low off-target effects.

Experimental Example 17. Lowering effects of siRNA conjugates on blood lipid in mice (*in vivo*)

According to the method in Experimental Example 14, the blood lipid-lowering effect of the siRNA conjugate in mice was tested, the only difference being that the siRNA conjugate used was conjugate 13 or conjugate 14. The results are shown in FIGs. 9A and 9B.

FIGs. 9A and 9B are line graphs showing changes in the level of serum TG or serum CHO over time after administration of siRNA conjugates of the present disclosure or PBS. Further, the serum TG inhibition rates and serum CHO inhibition rates at these time points are summarized in Table 17A and Table 17B:

Table 17A. Serum TG inhibition rate of siRNA conjugates in transgenic mice

| Group | Dose mg/kg | TG level inhibition rate (%) | | | | | |
|---|---|---|---|---|---|---|---|
| | | D9 | D15 | D22 | D29 | D36 | D50 |
| Blank control | / | 8.2 | 19.2 | -3.6 | 3.5 | 3.7 | -16.3 |
| Conjugate 13 | 3 | 87.5 | 87.1 | 85.3 | 77.8 | 63.2 | 55.9 |
| | 1 | 81.7 | 82.2 | 80.8 | 65.3 | 58.8 | 31.2 |
| Conjugate 14 | 3 | 90.5 | 92.0 | 90.3 | 82.5 | 81.3 | 66.0 |
| | 1 | 85.2 | 89.5 | 80.1 | 69.2 | 57.2 | 53.0 |

Table 17B. Serum CHO inhibition rates of siRNA conjugates in transgenic mice

| Group | Dose mg/kg | CHO level inhibition rate (%) | | | | | |
|---|---|---|---|---|---|---|---|
| | | D9 | D15 | D22 | D29 | D36 | D50 |
| Blank control | / | -1.9 | 9.5 | 2.8 | 2.1 | -1.5 | -1.3 |
| Conjugate 13 | 3 | 53.4 | 48.5 | 53.6 | 45.7 | 47.6 | 40.6 |
| | 1 | 50.7 | 48.9 | 50.7 | 37.6 | 40.9 | 24.9 |
| Conjugate 14 | 3 | 47.1 | 43.7 | 51.1 | 45.2 | 53.2 | 42.0 |
| | 1 | 53.0 | 48.3 | 48.1 | 42.2 | 40.1 | 39.8 |

The results in FIG. 9A, FIG. 9B and Tables 17A and 17B show that at different time points after administration, conjugate 13 and conjugate 14 can significantly reduce the levels of TG and CHO in mouse serum. In particular, at doses of 3 mg/kg and 1 mg/kg, conjugate 14 has always shown a very high blood lipid TG lowering effect throughout the administration period of up to 50 days, and the highest inhibition rate can reach 92.0%.

Experimental Example 18. Toxic effects of siRNA conjugates in mice

According to the method in Experimental Example 15, the toxicity effect of the siRNA conjugate in mice was verified, the only difference being that the experiment was carried out with conjugate 13 and reference conjugate 7, and each conjugate was respectively dissolved in PBS to obtain a 10 mg/mL solution (based on siRNA conjugate). The 2-week group and 4-week group (marked as D15 group and D29 group in Table 18) each had 3 mice, all of which were male. That is, the toxic effects of conjugate 13 and reference conjugate 7 in mice were tested at a dose of 100 mg/kg. The results are shown in Table 18.

Table 18. Blood biochemical results of mice administered siRNA conjugates

| Experimental group | | Reference conjugate 7 | Conjugate 13 |
|---|---|---|---|
| Dose | | 100mg/kg | 100mg/kg |
| ALT | D15 | 71% | - |
| | D29 | 118% | - |
| AST | D15 | 54% | - |
| | D29 | 94% | - |

In Table 18, % and the preceding figures represent the difference between the concentration of alanine aminotransferase (ALT) or aspartate aminotransferase (AST) in the mouse serum and the concentration in the serum of the blank control group, the percentage of the concentration in the serum of the blank control group.

According to the results in Table 18, compared with the blank control, the mice administered reference conjugate 7, which contained no stabilizing modified nucleotides, showed significant changes in blood biochemical indicators. On

day 15 after the administration, the concentrations of ALT and AST in serum were increased by 71% and 54%, respectively, and on day 29 after administration, the concentrations of ALT and AST in serum were increased by 118% and 94%, respectively. However, no increase was observed in the serum ALT and AST concentrations in mice administered the same dose of conjugate 13. Conjugate 13 of the present disclosure showed significantly lower blood biochemical indicators.

The above results show that compared with the reference conjugates, the siRNA conjugates of the present disclosure can effectively reduce the hepatotoxicity caused by off-target effects and therefore show significantly higher safety in the preparation of medicaments for treating and/or preventing dyslipidemia-related diseases or symptoms, having excellent development prospects.

Experimental Example 19. Lowering effects of siRNA conjugates on blood lipid in mice *(in vivo)*

According to the method in Experimental Example 14, the lowering effect of siRNA conjugates on blood lipids in mice was detected, the only difference being that the siRNA conjugates used were conjugate 13, and the dose of each siRNA conjugate (based on a siRNA) was 9 mg/kg, 3 mg/kg, 1 mg/kg, 0.5 mg/kg, 0.25 mg/kg, 0.1 mg/kg or 0.05 mg/kg mouse body weight, and the administration volume was 5 ml/kg. Each siRNA conjugate was provided in PBS aqueous solution, and the concentration that the conjugate should be prepared was calculated according to the dose and volume of administration. The administration time point was recorded as day 1, and blood was collected from the orbital venous plexus of mice on days 1, 8, 15, 22, 29, 36, 43, 50, 57 and 64 to detect the level of TG in serum. The results are shown in FIG. 10.

FIG. 10 is a line graph showing changes in the serum TG level over time after administration of different concentrations of conjugate 13 or PBS. Further, the serum TG inhibition rates at these time points are summarized in Table 19:

Table 19. Serum TG inhibition rates of siRNA conjugates in transgenic mice

| Group | Dose mg/kg | TG level inhibition rate (%) | | | | | | | | |
|---|---|---|---|---|---|---|---|---|---|---|
| | | D8 | D15 | D22 | D29 | D36 | D43 | D50 | D57 | D64 |
| Blank control | / | 27.1 | 13.1 | 4.0 | 8.2 | 22.7 | 15.3 | 17.4 | -0.3 | 17.4 |
| Conjugate 13 | 9 | 89.5 | 88.5 | 89.0 | 87.9 | 88.3 | 86.6 | 71.3 | 59.7 | 54.7 |
| | 3 | 88.3 | 86.1 | 87.4 | 79.8 | 70.7 | 63.1 | 45.2 | 30.6 | 24.5 |
| | 1 | 76.3 | 68.2 | 54.4 | 63.1 | 57.9 | 39.8 | 15.5 | 5.0 | 7.0 |
| | 0.5 | 67.4 | 51.9 | 45.3 | 37.7 | 30.3 | 13.1 | 11.7 | 0.8 | 16.1 |
| | 0.25 | 32.6 | 18.3 | 32.1 | 34.7 | 25.0 | 15.2 | 7.3 | 5.3 | 5.3 |
| | 0.1 | 39.1 | 39.4 | 23.6 | 35.1 | 27.4 | 13.5 | 19.9 | 1.7 | 10.9 |
| | 0.05 | 47.8 | 38.7 | 5.6 | 17.0 | 14.6 | -3.7 | -21.6 | -13.2 | -12.5 |

The results in FIG. 10 and Table 19 show that at different time points after administration, different concentrations of conjugate 13 can all reduce the TG level in mouse serum. Especially, at a dose of 9 mg/kg, the siRNA conjugate of the present disclosure, after only one administration, can maintain a TG level inhibition rate of greater than 50% for as long as 64 days, and the inhibition rate can reach up to 89.5%, showing an excellent blood lipid inhibition ability.

Experimental Example 20. Inhibition activity of siRNA conjugates in the *in vitro* siCHECK system According to the method in Experimental Example 13, the inhibition activity of conjugate 17 in the *in vitro* siCHECK system was tested, the only difference being that conjugate 17 was tested instead of the tested siRNA conjugate. As a result, conjugate 17 showed high target sequence inhibition activity in the *in vitro* siCHECK system, with an $IC_{50}$ value of 49.8 pM.

Experimental Example 21. Lowering effects of siRNA conjugates on blood lipid in mice *(in vivo)*

Human APOC3 transgenic mice Tg(APOC3)3707Bres (purchased from Jackson Laboratory, USA) with serum TG content > 2 mmol/L were selected and randomly divided into groups, 8 mice per group, half were male and half female. Conjugate 13, reference conjugate 17 and PBS blank control were administered to each group of mice respectively. All animals were dosed according to their body weight, and administered in a single subcutaneous injection. The dose of each siRNA conjugate (based on the amount of siRNA) was 3 mg/kg and 1 mg/kg mouse body weight, and the administration volume was 5 ml/kg. Each siRNA conjugate was provided in PBS aqueous solution, and the concentration

that the conjugate should be prepared was calculated according to the dosage and volume of administration. Each of the mice in the other group was administered 1× PBS with a volume of 5 ml/kg, serving as a blank control group.

The time point of administration was recorded as day 1, and blood was collected from the orbital venous plexus of the mice on days 1, 8, 15, 22, 29, 36, and 43, with 100 μL each time. The collected blood was left at room temperature for 30 min, and then centrifuged at 3000 rpm at 4 °C for 15 min to obtain serum. PM1P000/3 automatic serum biochemical analyzer (SABA, Italy) was then used to detect the contents of total cholesterol (CHO) and triglyceride (TG) in serum.

Standardized blood lipid level = (blood lipid content of test group after administration/blood lipid content of test group before administration) × 100%.

Inhibition rate of blood lipid level =(1 - blood lipid content of test group after adiministration/blood lipid content of test group before administration) × 100%.

Blood lipid refers to total cholesterol (CHO) or triglyceride (TG).

FIGs. 11A and 11B are line graphs showing changes in the level of serum TG or serum CHO over time after administration of siRNA conjugates of the present disclosure or PBS. Further, the serum TG inhibition rates and serum CHO inhibition rates at these time points after administration of the siRNA conjugates of the present disclosure are summarized in Table 20A and Table 20B:

Table 20A. Serum TG inhibition rate of siRNA conjugates in transgenic mice

| Group | Dose mg/kg | TG level inhibition rate (%) | | | | | |
|---|---|---|---|---|---|---|---|
| | | D8 | D15 | D22 | D29 | D36 | D43 |
| Conjugate 13 | 3 | 88.26 | 84.22 | 89.33 | 88.22 | 81.60 | 64.87 |
| | 1 | 80.65 | 75.07 | 70.78 | 56.35 | 41.94 | 44.23 |
| Conjugate 17 | 3 | 88.57 | 86.99 | 87.59 | 88.61 | 80.18 | 60.13 |
| | 1 | 86.09 | 83.67 | 70.63 | 45.51 | 35.31 | 12.17 |

Table 20B. Serum CHO inhibition rates of siRNA conjugates in transgenic mice

| Group | Dose mg/kg | CHO level inhibition rate (%) | | | | | |
|---|---|---|---|---|---|---|---|
| | | D8 | D15 | D22 | D29 | D36 | D43 |
| Conjugate 13 | 3 | 47.92 | 44.15 | 49.14 | 51.02 | 51.18 | 40.19 |
| | 1 | 41.83 | 36.74 | 39.42 | 37.94 | 22.04 | 29.08 |
| Conjugate 17 | 3 | 52.95 | 53.46 | 55.17 | 57.41 | 47.98 | 39.96 |
| | 1 | 45.03 | 44.23 | 38.27 | 29.18 | 29.08 | 10.06 |

According to the results in FIG. 11A, FIG. 11B and Tables 20A and 20B, at different time points after administration, conjugate 13 and conjugate 17 can significantly reduce the levels of TG and CHO in mouse serum. In addition, within the 43 days of the experiment, the inhibition effect was always kept high. Especially, at a dose of 3 mg/kg, both conjugate 13 and conjugate 17 showed excellent inhibition effects on blood lipids in mice, their maximum serum TG inhibition rates were both higher than 88%, and their maximum serum CHO inhibition rates were 51.18% and 57.41%, respectively. The above results show that the siRNA conjugates of the present disclosure can effectively reduce blood lipid levels for a long time, and show excellent development prospects in the preparation of medicaments for treating and/or preventing dyslipidemia-related diseases or symptoms.

Experimental Example 22. Toxic effects of siRNA conjugates in mice

Conjugate 13 and conjugate 17 were dissolved in PBS to obtain a 30 mg/mL solution (based on siRNA conjugate). ICR mice (half were male and half female, weighing 18-22 g, 5-6 weeks old, purchased from SiPeiFu Inc.) were randomly divided into groups, 10 mice per group (half were male and half female), and numbered. In the manner of subcutaneous injection on the back of the neck, the siRNA conjugate solution was administered to each mouse respectively at an administration volume of 10 mL/kg, as a test group. In addition, PBS was administered to each of a group of mice at an administration volume of 10 mL/kg, as a blank control group.

Taking the time point of the first administration as day 1, each mouse was repeatedly administered on day 8 and day 15, and the concentration and administration volume of the siRNA conjugate solution (or PBS) used were the same as those of the first administration. Each mouse in the test group and the blank control group was subjected to orbital blood collection on day 16. The blood collection volume was 0.6 mL, and the collected blood was incubated at 37 °C for 60 min and then centrifuged at 3000 rpm at 4 °C for 15 min to obtain serum. The concentrations of alanine aminotransferase (ALT) and aspartate aminotransferase (AST) in serum were further detected by PM1P000/3 automatic serum biochemical analyzer (SABA, Italy). The results are shown in FIG. 12A and FIG. 12B.

FIGs. 12A and 12B are scatter plots showing ALT and AST concentrations in mouse serum after weekly administration of 300 mg/kg of siRNA conjugates of the present disclosure or PBS for three consecutive weeks. As shown in FIGs. 12A and 12B, compared with the blank control group, after administration of the conjugates of the present disclosure, the serum ALT and AST concentrations were comparable to those of the blank control group, indicating that the siRNA conjugates of the present disclosure have very low hepatotoxicity.

Further, after blood collection, the mice were sacrificed and dissected, and preserved in 10% neutral buffered formalin fixative, and pathological sections were made. The pathological sections showed that the mice administered siRNA conjugate 3 or conjugate 5 of the present disclosure showed a similar response to the blank control group in terms of hepatic steatosis and inflammation, without significant abnormalities. It also indicates that the siRNA conjugates of the present disclosure have very low hepatotoxicity. It also indicates that the siRNA conjugates of the present disclosure have very low hepatotoxicity.

The above results show that the siRNA conjugates of the present disclosure can effectively reduce the hepatotoxicity caused by off-target effects and therefore show significantly higher safety in the preparation of medicaments for treating and/or preventing dyslipidemia-related diseases or symptoms, having excellent development prospects.

Experimental Example 23. Lowering effects of siRNA conjugates on blood lipid in mice *(in vivo)*

Human APOC3 transgenic mice Tg(APOC3)3707Bres (purchased from Jackson Laboratory, USA) with serum TG content > 2 mmol/L were selected and randomly divided into groups, 6 mice per group, half of which were male and half female. Conjugate 15, conjugate 16, reference conjugate 8 and PBS blank control were administered to each group of mice respectively. All animals were dosed according to their body weight, and administered in a single subcutaneous injection. The dose of each siRNA conjugate (based on the amount of siRNA) was 3 mg/kg and 1 mg/kg mouse body weight, and the administration volume was 5 ml/kg. Each siRNA conjugate was provided in PBS aqueous solution, and the concentration that the conjugate should be prepared was calculated according to the dosage and volume of administration. Each of the mice in the other group was administered 1× PBS with a volume of 5 ml/kg, serving as a blank control group.

The time point of administration was recorded as day 1, and blood was collected from the orbital venous plexus of the mice on days 1, 8, 15 and 22, 100 μL each time. The collected blood was left at room temperature for 30 min, and then centrifuged at 3000 rpm at 4 °C for 15 min to obtain serum. PM1P000/3 automatic serum biochemical analyzer (SABA, Italy) was then used to detect the contents of total cholesterol (CHO) and triglyceride (TG) in serum.

Standardized blood lipid level = (blood lipid content of test group after administration/blood lipid content of test group before administration) × 100%.

Inhibition rate of blood lipid level = (1 - blood lipid content of test group after administration/blood lipid content of test group before administration) × 100%.

Blood lipid refers to total cholesterol (CHO) or triglyceride (TG).

FIGs. 13A and 13B are line graphs showing changes in the level of serum TG or serum CHO over time after administration of a siRNA conjugate of the present disclosure, a reference siRNA conjugate or PBS. Further, the serum TG inhibition rates and serum CHO inhibition rates at these time points after administration of the siRNA conjugates of the present disclosure are summarized in Table 21A and Table 21B:

Table 21A. Serum TG inhibition rates of siRNA conjugates in transgenic mice

| Group | Dose mg/kg | TG level inhibition rate (%) | | |
|---|---|---|---|---|
| | | D9 | D15 | D22 |
| Blank control | / | 17.3 | -3.8 | -3.2 |
| Reference conjugate 8 | 3 | 92.2 | 90.6 | 85.7 |
| | 1 | 84.7 | 80.6 | 80.6 |
| Conjugate 15 | 3 | 92.0 | 92.3 | 90.0 |
| | 1 | 76.1 | 74.6 | 67.6 |
| Conjugate 16 | 3 | 92.9 | 92.3 | 89.9 |
| | 1 | 82.8 | 79.3 | 82.3 |

Table 21B. Serum CHO inhibition rates of siRNA conjugates in transgenic mice

| Group | Dose mg/kg | CHO level inhibition rate (%) | | |
|---|---|---|---|---|
| | | D9 | D15 | D22 |
| Blank control | / | 19.4 | -2.6 | 1.7 |
| Reference conjugate 8 | 3 | 51.6 | 44.3 | 42.7 |
| | 1 | 52.1 | 45.5 | 50.8 |
| Conjugate 15 | 3 | 57.5 | 57.3 | 47.2 |
| | 1 | 49.3 | 43.9 | 42.4 |
| Conjugate 16 | 3 | 54.9 | 50.0 | 55.4 |
| | 1 | 50.9 | 44.0 | 46.7 |

The results in FIG. 13A, FIG. 13B and Tables 21A and 21B show that at different time points after administration, conjugate 15 and conjugate 16 can significantly reduce the levels of TG and CHO in mouse serum, and maintained high inhibition effects throughout the 22 days of the experiment. In addition, the conjugates showed similar blood lipid level lowering effects to the corresponding reference conjugate 8, which contained no stabilizing modified nucleotides.

Especially, at a dose of 3 mg/kg, both conjugate 15 and conjugate 16 showed excellent inhibition effects on blood lipids in mice, their maximum serum TG inhibition rates were both higher than 92%, and their maximum serum CHO inhibition rates were 57.5% and 54.9%, respectively. The above results show that the siRNA conjugates of the present disclosure can effectively reduce blood lipid levels for a long time, and show excellent development prospects in the preparation of medicaments for treating and/or preventing dyslipidemia-related diseases or symptoms.

Experimental Example 24. Toxic effects of siRNA conjugates in mice

According to the method in Experimental Example 11, the toxicity effect of the siRNA conjugate in mice was verified, the only difference being that the experiment was carried out with conjugate 15, conjugate 16 and reference conjugate 8, and each conjugate was dissolved in PBS to form a 10 mg/ml solution (based on siRNA conjugate). Serum was collected from all mice on day 8 and the mice were necropsied. That is, the toxic effects of conjugate 15, conjugate 16 and reference conjugate 8 in mice were tested at a dose of 100 mg/kg. The results are shown in Table 22.

Table 22. Blood biochemical results of mice administered siRNA conjugates

| Experimental group | Reference conjugate 8 | Conjugate 15 | Conjugate 16 |
|---|---|---|---|
| Dose | 100 mg/kg | 100 mg/kg | 100 mg/kg |
| ALT | 420% | 116% | 118% |
| AST | 126% | - | 32% |

In Table 22, % and the preceding figures represent the difference between the concentration of alanine aminotransferase (ALT) or aspartate aminotransferase (AST) in the mouse serum and the concentration in the serum of the blank control group and the percentage of the concentration in the serum of the blank control group. For example, 420% in Table 22 means that the concentration of alanine aminotransferase in the mice administered reference conjugate 8 at 100 mg/Kg was 420% higher than that in the blank control group.

According to the results in Table 22, compared with the blank control group, the mice administered reference conjugate 8, which contained no stabilizing modified nucleotides, showed significant changes in blood biochemical indicators: the concentration of ALT was increased by 420%, and the concentration of AST was increased by 126%. In the mice administered siRNA conjugate 15 or 16 of the present disclosure, the concentrations of ALT were increased by only 116% and 118%, respectively, and the concentration of AST was increased by only 32%, showing significantly decreased blood biochemical indicators.

The results of pathological sections showed that compared with the blank control group, one of the 6 mice administered reference conjugate 8, which contained no stabilizing modified nucleotides, had a moderate hepatic inflammatory response, specifically manifested that a small amount of diffuse inflammatory cell infiltration was visible in the hepatic lobules thereof, diffuse proliferation of fibroblasts in hepatic sinusoids. Three mice had mild hepatic inflammatory reaction, focal infiltration of inflammatory cells in local hepatic lobules, and inflammatory necrosis of local hepatic lobule in 1 animal, individual hepatocytes with punctate necrosis. However, among the mice administered conjugate 15 of the present disclosure, only 2 mice showed mild hepatocyte inflammatory response, showing a small amount of inflammatory cell infiltration, and no moderate or severer inflammatory response and necrosis were found. Among the mice administered conjugate 16 of the present disclosure, only one mouse showed mild hepatocyte inflammatory response, and no moderate or severer inflammatory response and necrosis were found. Conjugates 15 and 16 exhibited significantly lower toxicity than reference conjugate 8.

The above results show that compared with the reference conjugates, the siRNA conjugates of the present disclosure can effectively reduce the hepatotoxicity caused by off-target effects and therefore show significantly higher safety in the preparation of medicaments for treating and/or preventing dyslipidemia-related diseases or symptoms, having excellent development prospects.

Experimental Example 25. Inhibition activity of siRNA conjugates in the *in vitro* siCHECK system According to the method in Experimental Example 12, the inhibition activity of the siRNA conjugates in the *in vitro* siCHECK system was tested, the only difference being that conjugate 18, conjugate 19 or reference conjugate 9 were tested instead of the siRNA conjugate tested in Experimental Example 12, and the target sequences used was the following target sequence 12:

Target sequence 12:

AGCCAAGAGCACCAAGAACTA (SEQ ID NO: 138)

Target sequence 12 is a nucleotide sequence in the mRNA expressed by the human AMGPTL3 gene targeted by the tested siRNA, so the inhibition effect of each siRNA conjugate on the target sequence 12 may reflect the abilities of the tested siRNA conjugates to inhibit target gene expression. Target sequence 12 and the complementary sequence thereof were cloned into the Xho I/Not I site of the psiCHECK™-2 plasmid.

The inhibition effect of each siRNA conjugate or reference siRNANC on target sequence 12 is shown in FIG. 14. FIG. 14 is a bar chart showing the relative expression levels of target sequence 12 in the *in vitro* siCHECK system after co-transfection with plasmids containing target sequence 12 and siRNA conjugates to be tested or reference siRNANC. Further, the expression inhibition rates of the siRNA conjugates or reference siRNA NC against target sequence 12 are summarized in Table 23.

Table 23. Target sequence 12 expression inhibition rates in the *in vitro* siCHECK system

| siRNA conjugate No. (reference siRNA No.) | Expression inhibition ratio (%) of target sequence | | |
|---|---|---|---|
| | 0.1 nM | 0.03 nM | 0.01 nM |
| Conjugate 18 | 88.84 | 75.76 | 58.76 |
| Conjugate 19 | 87.92 | 73.04 | 51.56 |
| Reference conjugate 9 | 88.47 | 76.73 | 54.01 |
| Reference siRNA NC | -7.55 | 11.24 | 8.74 |

According to the results in FIG. 14 and Table 23, the siRNA conjugates provided by the present disclosure have very high target sequence inhibition activity in the *in vitro* siCHECK system. At a low concentration of 0.01 nM, the

target sequence expression inhibition rate was at least 51.56 nM and could reach up to 58.76%. At a concentration of 0.1 nM, the target sequence expression inhibition rate may be as high as 87.92-88.84%. Meanwhile, the siRNA conjugate has a target sequence inhibition activity close to that of reference conjugate 9, which contained no stabilizing modified nucleotides.

Experimental Example 26. Off-target sequence inhibition activity of siRNA conjugates in the *in vitro* siCHECK system

According to the method described in Experimental Example 2, the off-target sequence inhibition activity of siRNA conjugates in the *in vitro* siCHECK system was determined, the only difference being that conjugate 18, conjugate 19, conjugate 20, conjugate 21, reference conjugate 9 or reference conjugate 11 was determined instead of the tested siRNA conjugate; and the target sequence used was target sequence 12 or target sequence 13 as shown below:
CTAACCTCTACAAAAGAACTA (SEQ ID NO: 139)

Target sequence 13 comprises a nucleotide sequence that is partially complementary to the antisense strand of the siRNA conjugate to be tested, so the inhibition effect of each siRNA conjugate on target sequence 13 can reflect the degree of off-target effect. That is, the higher the inhibition effect, the more likely the siRNA conjugate is off-target. In another aspect, the ratio of the inhibition effect of each siRNA conjugate on target sequence 12 to the inhibition effect on target sequence 13 can reflect the relative off-target tendency of the siRNA conjugate. The higher the ratio is, the less likely off-target occurs, i.e., the lower the possibility of off-target toxicity when the same level of drug efficacy is obtained.

For target sequence 12, the corresponding $X_{75}$ value when Y=75% was determined from the dose-effect curve and the corresponding function, and the on-target $IC_{25}$ value = $10^{\wedge}X_{25}$ (nM) of each siRNA conjugate was calculated.

For target sequence 13, the corresponding $X_{75}$ value when Y=75% was determined from the dose-effect curve and the corresponding function, and the off-target $IC_{25}$ value = $10^{\wedge}X_{25}$ (nM) of each siRNA conjugate was calculated.

And, the ratio of off-target $IC_{25}$/on-target $IC_{25}$ was calculated for each siRNA conjugate. The above results are summarized in Table 24:

Table 24. Off-target sequence inhibition activity of siRNA conjugates in the siCHECK system

| Conjugate No. | On-target $IC_{25}$ (nM) | Off-target $IC_{25}$ (nM) | Ratio of target $IC_{25}$/off-target $IC_{25}$ |
|---|---|---|---|
| Conjugate 18 | 0.0090 | 4.5995 | 511 |
| Conjugate 19 | 0.0052 | 10.9176 | 2100 |
| Conjugate 20 | 0.0155 | 12.9020 | 832 |
| Conjugate 21 | 0.0044 | 2.1935 | 499 |
| Reference conjugate 9 | 0.0119 | 2.0137 | 169 |
| Reference conjugate 11 | 0.5833 | 16.195 | 28 |

According to the results in Table 24, each of the siRNA conjugates of the present disclosure containing stabilizing modified nucleotides not only has similar or significantly higher inhibition activity against on-target target sequence 12 but also showed significantly lower off-target effects than reference conjugate 9 and reference conjugate 11, which contained no stabilizing modified nucleotides. The ratio of off-target $IC_{25}$/on-target $IC_{25}$ was at least 499 and could even reach up to 2100, indicating that when the same or similar desired drug efficacy is obtained, the off-target effects of the siRNA conjugates of the present disclosure are lower than those of the reference conjugates. Therefore, the siRNA conjugates of the present disclosure show excellent potential in the preparation of medicaments for inhibiting ANGPTL3 that are more effective and have lower toxicity caused by off-target effects.

Experimental Example 27. Lowering effects of siRNA conjugates on ANGPTL3 mRNAin mice (*in vivo*)

C57BL/6 mice (6-8 weeks old, purchased from SiPeifu Inc.) with serum TG content > 2 mmol/L were selected and randomly divided into groups, 5 mice per group, all of which were female. Conjugate 18, conjugate 19, reference conjugate 9 and PBS blank control were administered to each group of mice respectively. All animals were dosed according to their body weight, and administered in a single subcutaneous injection. The dose of each siRNA conjugate (based on the amount of siRNA) was 3 mg/kg mouse body weight, and the administration volume was 5 mL/kg. Each siRNA conjugate was provided in PBS aqueous solution, and the concentration that the conjugate should be prepared was calculated according to the dose and volume of administration. Each of the mice in the other group was administered 1× PBS with a volume of 5 mL/kg, serving as a blank control group.

Taking the administration time point as day 1, the animals were sacrificed on day 15, and the liver tissues of each mouse were collected and preserved in RNA later (Sigma Aldrich); 1 mL of Trizol (Sigma) was added to each liver tissue, and crushed in a Tissuelyset II automatic tissue homogenizer for 3 times, each time for 30 s, to obtain liver tissue homogenate, to which 0.2 mL of chloroform was added; and the mixture was mixed and left to stand for 10 min. The mixture was centrifuged at 12000 rpm at 4 °C for 10 min, and 0.4 mL of supernatant was collected. The 0.5 mL of isopropyl alcohol was added to the supernatant, and the mixture was allowed to stand at room temperature for 10 min. The mixture was centrifuged at 12000 rpm at 4 °C for 10 min and the supernatant was discarded. The precipitate was washed with 1 mL of 75% ethanol, and centrifuged at 12000 rpm at 4 °C for 5 min, and the supernatant was discarded. To the precipitate, 70 μL of DEPC water was added to obtain a total RNA solution. RNA concentration was determined with NANO DROP 2000 (Thermo) according to the method described in the instructions.

For the total RNA of the liver tissue of each mouse, an aqueous total RNA solution containing 1 μg of total RNA was collected (the volume of the solution was 1000 μL/RNA concentration (ng/μL)) and prepared into a 20 μL reverse transcription reaction system by using the reverse transcription kit Reverse Transcription System (purchased from TSINGKE Inc.) according to the reverse transcription steps in the instructions of the kit, and the total RNA was reverse transcribed. The reverse transcription conditions are: for each reverse transcription reaction system, the reverse transcription reaction system was incubated at 42°C for 30 min, then incubated at 95°C for 5 min, and finally incubated at 4°C for 5 min, and 80 μL of DEPC water was added to the system to obtain a solution containing cDNA.

For each reverse transcription reaction system, with 5 μL of the cDNA-containing solution as a template, the reagents provided by 2× Ultra SYBR Mixture (with ROX) kit (purchased from Beijing ComWin Biotech Co., Ltd.) were used to prepare 20 μL of qPCR reaction system, wherein the PCR primer sequences used to amplify the target gene mANGPTL3 and the internal reference gene mGAPDH are shown in Table 25, and the final concentration of each primer was 0.25 μM. Each qPCR reaction system was placed on an ABI StepOnePlus Real-Time PCR instrument, and amplified using a three-step method. The amplification program was pre-denaturation at 95 °C for 10 min, followed by denaturation at 95 °C for 30 s, annealing at 60 °C for 30 s, and extension at 72 °C for 30 s. After repeating the denaturation, annealing, and extension processes for a total of 40 times, a product W containing amplified target gene mANGPTL3 and internal reference gene mGAPDH was obtained. The product W was then incubated at 95 °C for 1 min, 55 °C for 30 s, and 95 °C for 30 s. The real-time fluorescent quantitative PCR instrument collected the melting curves of the target gene mANGPTL3 and the internal reference gene mGAPDH in the product W respectively, and obtained the Ct values of the target gene mANGPTL3 and the internal reference gene mGAPDH.

Table 25. Primer sequence information

| Gene name | Primer type | Nucleotide sequence (5'→3') | SEQ ID NO |
|---|---|---|---|
| mANGPTL3 | Forward primer | GAGGAGCAGCTAACCAACTTAAT | 140 |
| | Reverse primer | TCTGCATGTGCTGTTGACTTAAT | 141 |
| mGAPDH | Forward primer | TGCACCACCAACTGCTTAG | 127 |
| | Reverse primer | GGATGCAGGGATGATGTTC | 128 |

The comparative Ct (ΔΔCt) method was used to carry out relative quantitative calculation of the relative expression level and inhibition rate of the target gene mANGPTL3 in each test group, and the calculation method was as follows:

ΔCt (test group) = Ct (target gene of test group) - Ct (internal reference gene of test group)

ΔCt (control group) = Ct (target gene of control group) - Ct (internal reference gene of control group)

ΔΔCt (test group) = ΔCt (test group) - ΔCt (average of control group)

ΔΔCt (control group) = ΔCt (control group) - ΔCt (average of control group)

wherein ΔCt (average of control group) is the arithmetic average value of ΔCt (control group) of 5 mice of the control group. Therefore, each mouse in the test group and the control group corresponds to a ΔΔCt value.

Taking the control group as a benchmark, the expression level of mANGPTL3 mRNA in the test group was normalized, and the expression level of mANGPTL3 mRNA in the blank control group was defined as 100%.

mANGPTL3 mRNA relative expression level of test group = $2^{-\Delta\Delta Ct(\text{test group})} \times 100\%$

mANGPTL3 mRNA inhibition rate of test group = (1 - mANGPTL3 mRNA relative expression level of test group) × 100%

The results are shown in FIG. 15. FIG. 15 is a scatter plot showing the relative expression levels of mANGPTL3 mRNAin the livers of C57BL/6 mice after administration of 3 mg/kg (based on siRNA) conjugate 18, conjugate 19 or reference conjugate 9 and PBS. Further, the inhibition rates of the siRNA conjugates against mANGPTL3 mRNA are summarized in Table 26.

Table 26. Inhibition of mANGPTL3 mRNA in mice

| Conjugate No. | Dose (based on siRNA, mg/kg) | mANGPTL3 mRNA inhibition rate (%) |
|---|---|---|
| Conjugate 18 | 3 | 70 |
| Conjugate 19 | 3 | 95 |
| Reference conjugate 9 | 3 | 89 |

According to the results in FIG. 15 and Table 26, the siRNA conjugates of the present disclosure exhibited excellent mANGPTL3 mRNA inhibition effects in mice. At a dose of 3 mg/kg, the mANGPTL3 mRNA inhibition rate was at least 70% and could even reach up to 95%, and showed similar or even higher mANGPTL3 mRNA inhibition activity than the corresponding reference conjugate 9, which contained no stabilizing modified nucleotides.

Experimental Example 28. Toxic effects of siRNA conjugates in mice

According to the method in Experimental Example 11, the toxicity effect of the siRNA conjugate in mice was verified, the only difference being that the experiment was carried out with conjugate 18 and reference conjugate 9, and each conjugate was respectively dissolved in PBS to obtain a 10 mg/ml solution (based on siRNA conjugate). That is, the toxic effects of conjugate 18 and reference conjugate 9 in mice were tested at a dose of 100 mg/kg. The results are shown in Table 27.

Table 27. Blood biochemical results of mice administered siRNA conjugates

| Experimental group | Reference conjugate 9 | Conjugate 18 |
|---|---|---|
| Dose | 100 mg/kg | 100 mg/kg |
| ALT (Increase relative to blank control) | M:630%<br>F:840% | M:25.6%<br>F:101% |
| AST (Increase relative to blank control) | M:114%<br>F:145% | M:-10.8%<br>F:37.0% |

In Table 27, % and the preceding figures represent the difference between the concentration of alanine aminotransferase (ALT) or aspartate aminotransferase (AST) in the mouse serum and the concentration in the serum of the blank control group, the percentage of the concentration in the serum of the blank control group.

According to the results in Table 27, compared with the blank control, the concentration of ALT in the serum of male and female mice administered reference conjugate 9, which contained no stabilizing modified nucleotides, increased by 630% and 840%, respectively, and the concentrations of AST increased by 114% and 145%, respectively. However, the concentration of ALT in the serum of male mice and female mice administered conjugate 18 of the present disclosure was increased by only 25.6% and 101%, respectively, and the concentration of AST was increased by only 10.8% and 37.0%, respectively. Compared with reference conjugate 9, significant reductions were shown in blood biochemical indicators.

The results of the pathological section showed that among the mice administered reference conjugate 9, 4 mice showed severe hepatic stestosis, specifically manifested as a large number of ballooning hepatocyte degeneration in the tissue, swelling of the cells, centered nuclei and cytoplasmic vacuolization. Two mice showed severe hepatocyte degeneration, specifically manifested as extensive ballooning hepatic stestosis in the tissue, cell swelling, centered

nuclei and cytoplasmic vacuolation. Among the mice administered conjugate 18 of the present disclosure, 3 mice showed mild hepatocyte degeneration, specifically manifested as complete lobular structure of tissue, tight arrangement of hepatocytes, a small number of hepatocyte cytoplasms were loose, and no severe or very severe hepatocyte degeneration.

The above results show that compared with the reference conjugates, the siRNA conjugates of the present disclosure can effectively reduce the hepatotoxicity caused by off-target effects and therefore show significantly higher safety in the preparation of medicaments for treating and/or preventing dyslipidemia-related diseases or symptoms, having excellent development prospects.

Some embodiments of the present disclosure have been described in detail above, but the present disclosure is not limited to the specific details of the embodiments. Within the scope of the technical concept of the present disclosure, various simple modifications may be made to the technical solutions of the present disclosure. These simple modifications all belong to the protection scope of the present disclosure.

In addition, it should be noted that the various specific technical features described in some embodiments above can be combined in any suitable manner where the features do not contradict each other. In order to avoid unnecessary repetition, such combinations will not be illustrated separately.

In addition, various implementations of the present disclosure may be combined arbitrarily, as long as they do not violate the idea of the present disclosure, and they should also be regarded as the content disclosed in the present disclosure.

## Claims

**1.** A double-stranded oligonucleotide, wherein the double-stranded oligonucleotide comprises a sense strand and an antisense strand; the sense strand comprises a nucleotide sequence I, and the antisense strand comprises a nucleotide sequence II; both the nucleotide sequence I and the nucleotide sequence II consist of 19 nucleotides, and each of the nucleotides in the nucleotide sequence I and the nucleotide sequence II is a modified or unmodified nucleotide; the nucleotide sequence I and the nucleotide sequence II are at least partially reversely complementary to form a double-stranded region, and the nucleotide sequence II is at least partially reversely complementary to a first nucleotide sequence segment; the first nucleotide sequence segment is a nucleotide sequence with 19 nucleotides in length in an mRNA expressed by a target gene; in the direction from the 5' end to the 3' end, at least one of the 3rd-6th nucleotides in the nucleotide sequence II is a stabilizing modified nucleotide, and none of the nucleotides other than the 3rd-9th nucleotides in the nucleotide sequence II are stabilizing modified nucleotides; the stabilizing modified nucleotide refers to a nucleotide whose ribose hydroxyl group at the 2' position is substituted with a stabilizing modification group; compared with a double-stranded oligonucleotide whose nucleotides at the corresponding positions are unmodified nucleotides, the double-stranded oligonucleotide comprising the stabilizing modified nucleotide has increased thermostability, and the steric hindrance of the stabilizing modification group is greater than that of 2'-O-methyl.

**2.** The double-stranded oligonucleotide according to claim 1, wherein in the direction from the 5' end to the 3' end, the 3rd or 5th nucleotide in the nucleotide sequence II is the stabilizing modified nucleotide.

**3.** The double-stranded oligonucleotide according to claim 1 or 2, wherein in the direction from the 5' end to the 3' end, no more than 2 of the 3rd-9th nucleotides in the nucleotide sequence II are the stabilizing modified nucleotides.

**4.** The double-stranded oligonucleotide according to claim 3, wherein in the direction from the 5' end to the 3' end, the 3rd and/or 5th nucleotide(s) in the nucleotide sequence II are/is the stabilizing modified nucleotide(s); optionally, one of the 4th, 7th or 9th nucleotide is also the stabilizing modified nucleotide.

**5.** The double-stranded oligonucleotide according to claim 3, wherein in the direction from the 5' end to the 3' end, the 3rd and 9th nucleotides in the nucleotide sequence II are the stabilizing modified nucleotides; or

in the direction from the 5' end to the 3' end, the 5th and 7th nucleotides in the nucleotide sequence II are the stabilizing modified nucleotides; or
in the direction from the 5' end to the 3' end, the 5th and 9th nucleotides in the nucleotide sequence II are the stabilizing modified nucleotides.

**6.** The double-stranded oligonucleotide according to any one of claims 1-5, wherein the increase in the thermostability of the double-stranded oligonucleotide refers to an increase in the melting temperature Tm of the double-stranded

oligonucleotide.

**7.** The double-stranded oligonucleotide according to claim 6, wherein the increase in the thermostability of the double-stranded oligonucleotide refers to an increase of at least 0.05 °C in the melting temperature Tm of the double-stranded oligonucleotide.

**8.** The double-stranded oligonucleotide according to claim 6, wherein the increase in the thermostability of the double-stranded oligonucleotide refers to an increase of 0.1-6 °C in the melting temperature Tm of the double-stranded oligonucleotide.

**9.** The double-stranded oligonucleotide according to claim 6, wherein the increase in the thermostability of the double-stranded oligonucleotide refers to an increase of 0.5-4 °C in the melting temperature Tm of the double-stranded oligonucleotide.

**10.** The double-stranded oligonucleotide according to any one of claims 1-9, wherein each of the stabilizing modification groups independently has a structure represented by -X-R, wherein X is O, NR', S or $SiR'_2$; R is one of $C_2$-$C_6$ alkyl, substituted $C_2$-$C_6$ alkyl, $C_6$-$C_8$ aryl and substituted $C_6$-$C_8$ aryl; each R' is independently one of H, $C_1$-$C_6$ alkyl, substituted $C_1$-$C_6$ alkyl, $C_6$-$C_8$ aryl and substituted $C_6$-$C_8$ aryl; the substituted $C_2$-$C_6$ alkyl or substituted $C_6$-$C_8$ aryl refers to a group formed by substituting one or more hydrogen atoms on $C_2$-$C_6$ alkyl or $C_6$-$C_8$ aryl with a substituent, and the substituent is each independently selected from one or more of the following substituents: $C_1$-$C_3$ alkyl, $C_6$-$C_8$ aryl, $C_1$-$C_3$ alkoxy, halogen, oxo and sulfanylidene.

**11.** The double-stranded oligonucleotide according to claim 10, wherein each of the stabilizing modification groups is independently selected from one of 2'-O-methoxyethyl, 2'-O-allyl, 2'-C-allyl, 2'-O-2-N-methylamino-2-oxoethyl, 2'-O-2-N,N-dimethylaminoethyl, 2'-O-3-aminopropyl and 2'-O-2,4-dinitrophenyl.

**12.** The double-stranded oligonucleotide according to claim 11, wherein each of the stabilizing modification groups is 2'-O-methoxyethyl.

**13.** The double-stranded oligonucleotide according to any one of claims 1-12, wherein the nucleotide sequence II is substantially reversely complementary, virtually reversely complementary or completely reversely complementary to the first nucleotide sequence segment.

**14.** The double-stranded oligonucleotide according to claim 13, wherein in the direction from the 5' end to the 3' end, the nucleotides at the 2nd-19th positions of the nucleotide sequence II are completely reversely complementary to the nucleotides at the 1 st-18th positions of the first nucleotide sequence segment.

**15.** The double-stranded oligonucleotide according to any one of claims 1-14, wherein the nucleotide sequence II is substantially reversely complementary, virtually reversely complementary or completely reversely complementary to the nucleotide sequence I.

**16.** The double-stranded oligonucleotide according to claim 15, wherein the nucleotide sequence II is completely reversely complementary to the nucleotide sequence I, or there is a base mismatch between the 2nd nucleotide in the direction from the 5' end to the 3' end in the nucleotide sequence II and the 2nd nucleotide in the direction from the 3' end to the 5' end in the nucleotide sequence I.

**17.** The double-stranded oligonucleotide according to any one of claims 1-16, wherein in the direction from the 5' end to the 3' end, the 2nd, 6th, 14th, and 16th nucleotides in the nucleotide sequence II are 2'-fluoro-modified nucleotides, or the 2nd, 14th, and 16th nucleotides in the nucleotide sequence II are 2'-fluoro-modified nucleotides, and the 6th nucleotide in the nucleotide sequence II is a stabilizing modified nucleotide.

**18.** The double-stranded oligonucleotide according to claim 17, wherein all nucleotides in the nucleotide sequence II are modified nucleotides; in the direction from the 5' end to the 3' end, the 2nd, 6th, 14th, and 16th nucleotides in the nucleotide sequence II are 2'-fluoro-modified nucleotides, and the other nucleotides in the nucleotide sequence II are each independently one of non-fluoro-modified nucleotides.

**19.** The double-stranded oligonucleotide according to any one of claims 1-18, wherein in the direction from the 5' end to the 3' end, the 7th-9th nucleotides in the nucleotide sequence I are 2'-fluoro-modified nucleotides.

**20.** The double-stranded oligonucleotide according to claim 19, wherein all nucleotides in the nucleotide sequence I are modified nucleotides; in the direction from the 5' end to the 3' end, the 7th-9th nucleotides in the nucleotide sequence I are 2'-fluoro-modified nucleotides, and the other nucleotides in the nucleotide sequence I are each independently one of non-fluoro-modified nucleotides.

**21.** The double-stranded oligonucleotide according to any one of claims 1-20, wherein the sense strand further comprises a nucleotide sequence III, and the antisense strand further comprises a nucleotide sequence IV; each nucleotide in the nucleotide sequence III and the nucleotide sequence IV is independently one of non-fluoro-modified nucleotides and is not the stabilizing modified nucleotide; the nucleotide sequence III is 1, 2, 3 or 4 nucleotides in length; the nucleotide sequence IV and the nucleotide sequence III are equal in length, and the nucleotide sequence IV is virtually reversely complementary or completely reversely complementary to the nucleotide sequence III; the nucleotide sequence III is linked to the 5' end of the nucleotide sequence I, and the nucleotide sequence IV is linked to the 3' end of the nucleotide sequence II; the nucleotide sequence IV is virtually reversely complementary or completely reversely complementary to a second nucleotide sequence segment, and the second nucleotide sequence segment refers to a nucleotide sequence that is adjacent to the first nucleotide sequence segment in the mRNA expressed by the target gene and is the same length as the nucleotide sequence IV

**22.** The double-stranded oligonucleotide according to any one of claims 1-21, wherein the double-stranded oligonucleotide further comprises a nucleotide sequence V, and each nucleotide in the nucleotide sequence V is independently one of non-fluoro-modified nucleotides and is not the stabilizing modified nucleotide; the nucleotide sequence V is 1 to 3 nucleotides in length and is linked to the 3' end of the antisense strand, thereby constituting the 3' overhang of the antisense strand.

**23.** The double-stranded oligonucleotide according to claim 22, wherein the nucleotide sequence V is 2 nucleotides in length, and in the direction from the 5' end to the 3' end, the nucleotide sequence V is 2 consecutive thymine deoxyribonucleotides, 2 consecutive uracil ribonucleotides, or is completely reversely complementary to a third nucleotide sequence segment; the third nucleotide sequence segment refers to a nucleotide sequence that is adjacent to the first nucleotide sequence segment or the second nucleotide sequence segment in the mRNA expressed by the target gene and is the same length as the nucleotide sequence V.

**24.** The double-stranded oligonucleotide according to any one of claims 1-23, wherein each of the non-fluoro-modified nucleotides is independently selected from one of a nucleotide or nucleotide analog formed by substituting the hydroxyl group at the 2' position of the ribosyl group of a nucleotide with a non-fluorine group.

**25.** The double-stranded oligonucleotide according to any one of claims 1-24, wherein each of the non-fluoro-modified nucleotides is a methoxy-modified nucleotide, and the methoxy-modified nucleotide refers to a nucleotide formed by substituting the 2'-hydroxyl group of the ribosyl group with a methoxy group.

**26.** The double-stranded oligonucleotide according to any one of claims 1-25, wherein at least one of phosphate groups in a phosphate-sugar backbone of at least one single strand of the sense strand and the antisense strand is a phosphate group having a modified group, and the phosphate group having a modified group is present in at least one from the group consisting of the following positions:

between the 1st nucleotide and the 2nd nucleotide at the 5' end of the sense strand;
between the 2nd nucleotide and the 3rd nucleotide at the 5' end of the sense strand;
between the 1st nucleotide and the 2nd nucleotide at the 3' end of the sense strand;
between the 2nd nucleotide and the 3rd nucleotide at the 3' end of the sense strand;
between the 1st nucleotide and the 2nd nucleotide at the 5' end of the antisense strand;
between the 2nd nucleotide and the 3rd nucleotide at the 5' end of the antisense strand;
between the 1st nucleotide and the 2nd nucleotide at the 3' end of the antisense strand; and
between the 2nd nucleotide and the 3rd nucleotide at the 3' end of the antisense strand.

**27.** The double-stranded oligonucleotide according to any one of claims 1-26, wherein a 5' end nucleotide of the antisense strand is a 5'-phosphate nucleotide or a 5'-phosphate analog-modified nucleotide.

**28.** The double-stranded oligonucleotide according to any one of claims 1-27, wherein the double-stranded oligonucleotide is a saRNA or siRNA.

**29.** The double-stranded oligonucleotide according to any one of claims 1-28, wherein the mRNA expressed by the target gene is selected from one of the mRNAs transcribed from the following genes: ACE2, AGT, ANGPTL3, ApoA, ApoB, ApoC, AR, ASK1, C3, C5, Col1A1, CTGF, Ebola, FOXO1, FTO, FVII, FXI, FXII, GCGR, HBV, HCV, HSD, p53, PCSK9, PNP, PLG, PKK, KNG, RAGE, RPTOR, SARS-CoV-2, SCD1, SCNN1A, SOD1, STAT3, TIMP-1, TMPRSS6 and XO.

**30.** The double-stranded oligonucleotide according to any one of claims 1-29, wherein the double-stranded oligonucleotide is a siRNA, and the mRNA expressed by the target gene is selected from an mRNA expressed by the hepatitis B virus gene (HBV), an mRNA expressed by the angiopoietin-like protein 3 (ANGPTL3) gene, or an mRNA expressed by the apolipoprotein C3 (ApoC3) gene.

**31.** The double-stranded oligonucleotide according to claim 30, wherein the double-stranded oligonucleotide is one of the following siRNAs: siRNAa1, siRNAa2, siRNAa3, siRNAa4, siRNAa5, siRNAa6, siRNAa7, siRNAa8, siRNAa9, siRNAa10, siRNAb11, siRNAb12, siRNAb13, siRNAb14, siRNAb15, siRNAb16, siRNAc17, siRNAc18, siRNAc19, siRNAc20, siRNAc21, siRNAa22 and siRNAa23.

**32.** A double-stranded oligonucleotide, wherein the double-stranded oligonucleotide comprises a sense strand and an antisense strand, and each nucleotide of the sense strand and the antisense strand is a modified nucleotide, wherein the sense strand comprises a nucleotide sequence I, and the antisense strand comprises a nucleotide sequence II; both the nucleotide sequence I and the nucleotide sequence II consist of 19 nucleotides; the nucleotide sequence I and the nucleotide sequence II are at least partially reversely complementary to form a double-stranded region, and the nucleotide sequence II is at least partially reversely complementary to a first nucleotide sequence segment; the first nucleotide sequence segment is a nucleotide sequence of 19 nucleotides in an mRNA expressed by a target gene; in the direction from the 5' end to the 3' end, the 7th-9th nucleotides in the nucleotide sequence I are fluoro-modified nucleotides, and each of nucleotides at other positions in the nucleotide sequence I is independently one of non-fluoro-modified nucleotides; in the direction from the 5' end to the 3' end, the 2nd, 6th, 14th, and 16th nucleotides in the nucleotide sequence II are fluoro-modified nucleotides, and each of nucleotides at other positions in the nucleotide sequence II is independently one of non-fluoro-modified nucleotides; and
in the direction from the 5' end to the 3' end, the 3rd and/or 5th nucleotide(s) in the nucleotide sequence II are/is 2'-O-methoxyethyl-modified nucleotide(s).

**33.** The double-stranded oligonucleotide according to claim 32, wherein in the direction from the 5' end to the 3' end, one of the 4th, 7th and 9th nucleotides in the nucleotide sequence II is a 2'-O-methoxyethyl-modified nucleotide, and the steric hindrance of each of the other non-fluoro-modified substituents in the nucleotide sequence II is not greater than that of 2'-O-methyl.

**34.** The double-stranded oligonucleotide according to claim 32 or 33, wherein in the direction from the 5' end to the 3' end, the 3rd and/or 5th nucleotide(s) in the nucleotide sequence II have/has a ribose 2'-O-methoxyethyl modification;

in the direction from the 5' end to the 3' end, the 3rd and 9th nucleotides in the nucleotide sequence II have a ribose 2'-O-methoxyethyl modification;
in the direction from the 5' end to the 3' end, the 5th and 7th nucleotides in the nucleotide sequence II have a ribose 2'-O-methoxyethyl modification; or
in the direction from the 5' end to the 3' end, the 5th and 9th nucleotides in the nucleotide sequence II have a ribose 2'-O-methoxyethyl modification.

**35.** The double-stranded oligonucleotide according to any one of claims 32-34, wherein the nucleotide sequence II is substantially reversely complementary, virtually reversely complementary or completely reversely complementary to the first nucleotide sequence segment.

**36.** The double-stranded oligonucleotide according to claim 35, wherein in the direction from the 5' end to the 3' end, the nucleotides at the 2nd-19th positions of the nucleotide sequence II are completely reversely complementary to the nucleotides at the 1 st-18th positions of the first nucleotide sequence segment.

**37.** The double-stranded oligonucleotide according to any one of claims 32-36, wherein the nucleotide sequence II is substantially reversely complementary, virtually reversely complementary or completely reversely complementary to the nucleotide sequence I.

**38.** The double-stranded oligonucleotide according to claim 37, wherein the nucleotide sequence II is completely reversely complementary to the nucleotide sequence I, or there is a base mismatch between the 2nd nucleotide in the direction from the 5' end to the 3' end in the nucleotide sequence II and the 2nd nucleotide in the direction from the 3' end to the 5' end in the nucleotide sequence I.

**39.** The double-stranded oligonucleotide according to any one of claims 32-38, wherein the sense strand further comprises a nucleotide sequence III, and the antisense strand further comprises a nucleotide sequence IV; each nucleotide in the nucleotide sequence III and the nucleotide sequence IV is independently one of non-fluoro-modified nucleotides and is not the stabilizing modified nucleotide; the nucleotide sequence III is 1, 2, 3 or 4 nucleotides in length; the nucleotide sequence IV and the nucleotide sequence III are equal in length, and the nucleotide sequence IV is virtually reversely complementary or completely reversely complementary to the nucleotide sequence III; the nucleotide sequence III is linked to the 5' end of the nucleotide sequence I, and the nucleotide sequence IV is linked to the 3' end of the nucleotide sequence II; the nucleotide sequence IV is virtually reversely complementary or completely reversely complementary to a second nucleotide sequence segment, and the second nucleotide sequence segment refers to a nucleotide sequence that is adjacent to the first nucleotide sequence segment in the mRNA expressed by the target gene and is the same length as the nucleotide sequence IV

**40.** The double-stranded oligonucleotide according to any one of claims 32-39, wherein the double-stranded oligonucleotide further comprises a nucleotide sequence V, and each nucleotide in the nucleotide sequence V is independently one of non-fluoro-modified nucleotides and is not the stabilizing modified nucleotide; the nucleotide sequence V is 1 to 3 nucleotides in length and is linked to the 3' end of the antisense strand, thereby constituting the 3' overhang of the antisense strand.

**41.** The double-stranded oligonucleotide according to claim 40, wherein the nucleotide sequence V is 2 nucleotides in length, and in the direction from the 5' end to the 3' end, the nucleotide sequence V is 2 consecutive thymine deoxyribonucleotides, 2 consecutive uracil ribonucleotides, or is completely reversely complementary to a third nucleotide sequence segment; the third nucleotide sequence segment refers to a nucleotide sequence that is adjacent to the first nucleotide sequence segment or the second nucleotide sequence segment in the mRNA expressed by the target gene and is the same length as the nucleotide sequence V

**42.** The double-stranded oligonucleotide according to any one of claims 32-41, wherein each of the non-fluoro-modified nucleotides is independently selected from one of a nucleotide or nucleotide analog formed by substituting the hydroxyl group at the 2' position of the ribosyl group of a nucleotide with a non-fluorine group.

**43.** The double-stranded oligonucleotide according to any one of claims 32-42, wherein each of the non-fluoro-modified nucleotides is a methoxy-modified nucleotide, and the methoxy-modified nucleotide refers to a nucleotide formed by substituting the 2'-hydroxyl group of the ribosyl group with a methoxy group.

**44.** The double-stranded oligonucleotide according to any one of claims 32-43, wherein at least one of phosphate groups in a phosphate-sugar backbone of at least one single strand of the sense strand and the antisense strand is a phosphate group having a modified group, and the phosphate group having a modified group is present in at least one from the group consisting of the following positions:

between the 1st nucleotide and the 2nd nucleotide at the 5' end of the sense strand;
between the 2nd nucleotide and the 3rd nucleotide at the 5' end of the sense strand;
between the 1st nucleotide and the 2nd nucleotide at the 3' end of the sense strand;
between the 2nd nucleotide and the 3rd nucleotide at the 3' end of the sense strand;
between the 1st nucleotide and the 2nd nucleotide at the 5' end of the antisense strand;
between the 2nd nucleotide and the 3rd nucleotide at the 5' end of the antisense strand;
between the 1st nucleotide and the 2nd nucleotide at the 3' end of the antisense strand; and
between the 2nd nucleotide and the 3rd nucleotide at the 3' end of the antisense strand.

**45.** The double-stranded oligonucleotide according to any one of claims 32-44, wherein a 5' end nucleotide of the antisense strand is a 5'-phosphate nucleotide or a 5'-phosphate analog-modified nucleotide.

**46.** The double-stranded oligonucleotide according to any one of claims 32-45, wherein the double-stranded oligonucleotide is a saRNA or siRNA.

47. The double-stranded oligonucleotide according to any one of claims 32-46, wherein the mRNA expressed by the target gene is selected from one of the mRNAs transcribed from the following genes: ACE2, AGT, ANGPTL3, ApoA, ApoB, ApoC, AR, ASK1, C3, C5, Col1A1, CTGF, Ebola, FOXO1, FTO, FVII, FXI, FXII, GCGR, HBV, HCV, HSD, p53, PCSK9, PNP, PLG, PKK, KNG, RAGE, RPTOR, SARS-CoV-2, SCD1, SCNN1A, SOD1, STAT3, TIMP-1, TMPRSS6 and XO.

48. The double-stranded oligonucleotide according to any one of claims 32-47, wherein the double-stranded oligonucleotide is a siRNA, and the mRNA expressed by the target gene is selected from an mRNA expressed by the hepatitis B virus gene (HBV), an mRNA expressed by the angiopoietin-like protein 3 (ANGPTL3) gene, or an mRNA expressed by the apolipoprotein C3 (ApoC3) gene.

49. A pharmaceutical composition, wherein the pharmaceutical composition comprises the double-stranded oligonucleotide according to any one of claims 1-48 and a pharmaceutically acceptable carrier.

50. An oligonucleotide conjugate, wherein the oligonucleotide conjugate comprises the double-stranded oligonucleotide according to any one of claims 1-48 and a conjugated group conjugated to the double-stranded oligonucleotide; the conjugated group comprises a linker and pharmaceutically acceptable targeting groups and/or delivery auxiliary groups, and the double-stranded oligonucleotide, the linker and the targeting groups or the delivery auxiliary groups are sequentially linked covalently or non-covalently; each of the targeting groups is selected from a ligand capable of binding to a cell surface receptor, and each of the delivery auxiliary groups is selected from a group capable of increasing the biocompatibility of the oligonucleotide conjugate in a delivery target organ or tissue.

51. Use of the double-stranded oligonucleotide according to any one of claims 1-48 and/or the pharmaceutical composition according to claim 49 and/or the oligonucleotide conjugate according to claim 50 in the preparation of a medicament for treating and/or preventing diseases or symptoms associated with the level of an mRNA expressed by a target gene.

52. The use according to claim 51, wherein the mRNA expressed by the target gene is selected from one of the mRNAs transcribed from the following genes: ACE2, AGT, ANGPTL3, ApoA, ApoB, ApoC, AR, ASK1, C3, C5, Col1A1, CTGF, Ebola, FOXO1, FTO, FVII, FXI, FXII, GCGR, HBV, HCV, HSD, p53, PCSK9, PNP, PLG, PKK, KNG, RAGE, RPTOR, SARS-CoV-2, SCD1, SCNN1A, SOD1, STAT3, TIMP-1, TMPRSS6 and XO.

53. The use according to claim 51 or 52, wherein the mRNA expressed by the target gene is selected from an mRNA expressed by the hepatitis B virus gene (HBV), an mRNA expressed by the angiopoietin-like protein 3 (ANGPTL3) gene, or an mRNA expressed by the apolipoprotein C3 (ApoC3) gene.

54. The use according to any one of claims 51-53, wherein the disease or symptom associated with the level of the mRNA expressed by the target gene is hepatitis B or dyslipidemia.

55. A method for treating and/or preventing diseases or symptoms associated with the level of an mRNA expressed by a target gene, wherein the method comprises: administering to a subject in need thereof the double-stranded oligonucleotide according to any one of claims 1-48 and/or the pharmaceutical composition according to claim 49 and/or the oligonucleotide conjugate according to claim 50.

56. The method according to claim 55, wherein the mRNA expressed by the target gene is selected from one of the mRNAs transcribed from the following genes: ACE2, AGT, ANGPTL3, ApoA, ApoB, ApoC, AR, ASK1, C3, C5, Col1A1, CTGF, Ebola, FOXO1, FTO, FVII, FXI, FXII, GCGR, HBV, HCV, HSD, p53, PCSK9, PNP, PLG, PKK, KNG, RAGE, RPTOR, SARS-CoV-2, SCD1, SCNN1A, SOD1, STAT3, TIMP-1, TMPRSS6 and XO.

57. The method according to claim 55 or 56, wherein the mRNA expressed by the target gene is selected from an mRNA expressed by the hepatitis B virus gene (HBV), an mRNA expressed by the angiopoietin-like protein 3 (ANGPTL3) gene, or an mRNA expressed by the apolipoprotein C3 (ApoC3) gene.

58. The method according to any one of claims 55-57, wherein the disease or symptom associated with the level of the mRNA expressed by the target gene is hepatitis B or dyslipidemia.

59. A method for regulating the expression level of a target gene in a cell, wherein the method comprises: contacting an effective amount of the double-stranded oligonucleotide according to any one of claims 1-48 and/or the pharmaceutical composition according to claim 49 and/or the oligonucleotide conjugate according to claim 50 with the cell.

**60.** The method according to claim 59, wherein the mRNA expressed by the target gene is selected from one of the mRNAs transcribed from the following genes: ACE2, AGT, ANGPTL3, ApoA, ApoB, ApoC, AR, ASK1, C3, C5, Col1A1, CTGF, Ebola, FOXO1, FTO, FVII, FXI, FXII, GCGR, HBV, HCV, HSD, p53, PCSK9, PNP, PLG, PKK, KNG, RAGE, RPTOR, SARS-CoV-2, SCD1, SCNN1A, SOD1, STAT3, TIMP-1, TMPRSS6 and XO.

**61.** The method according to claim 59 or 60, wherein the regulation refers to inhibiting the expression of the target gene in the cell, and the mRNA expressed by the target gene is selected from an mRNA expressed by the hepatitis B virus gene (HBV), an mRNA expressed by the angiopoietin-like protein 3 (ANGPTL3) gene, or an mRNA expressed by the apolipoprotein C3 (ApoC3) gene.

**62.** A kit, wherein the kit comprises the double-stranded oligonucleotide according to any one of claims 1-48 and/or the pharmaceutical composition according to claim 49 and/or the oligonucleotide conjugate according to claim 50.

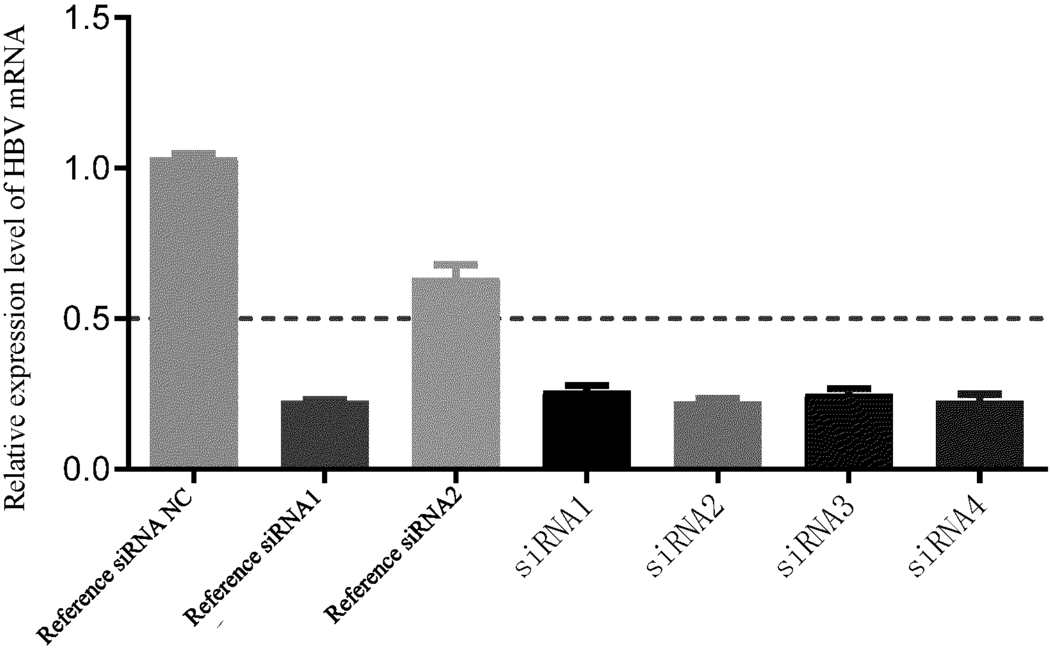
Relative expression level of HBV mRNA
1.5
1.0
0.5
0.0
Reference siRNA NC
Reference siRNA1
Reference siRNA2
siRNA1
siRNA2
siRNA3
siRNA4

FIG. 1

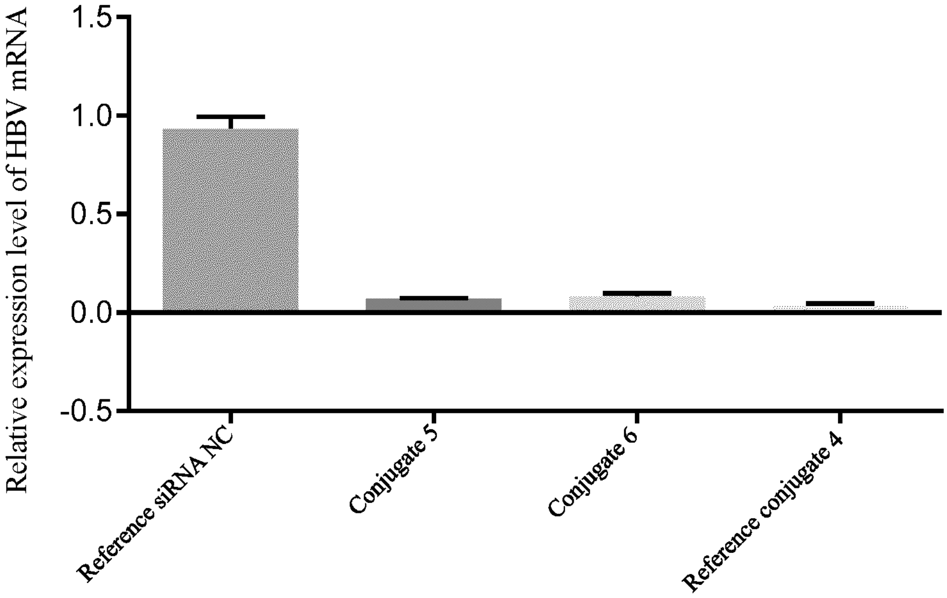
Relative expression level of HBV mRNA
1.5
1.0
0.5
0.0
-0.5
Reference siRNA NC
Conjugate 5
Conjugate 6
Reference conjugate 4

FIG. 2

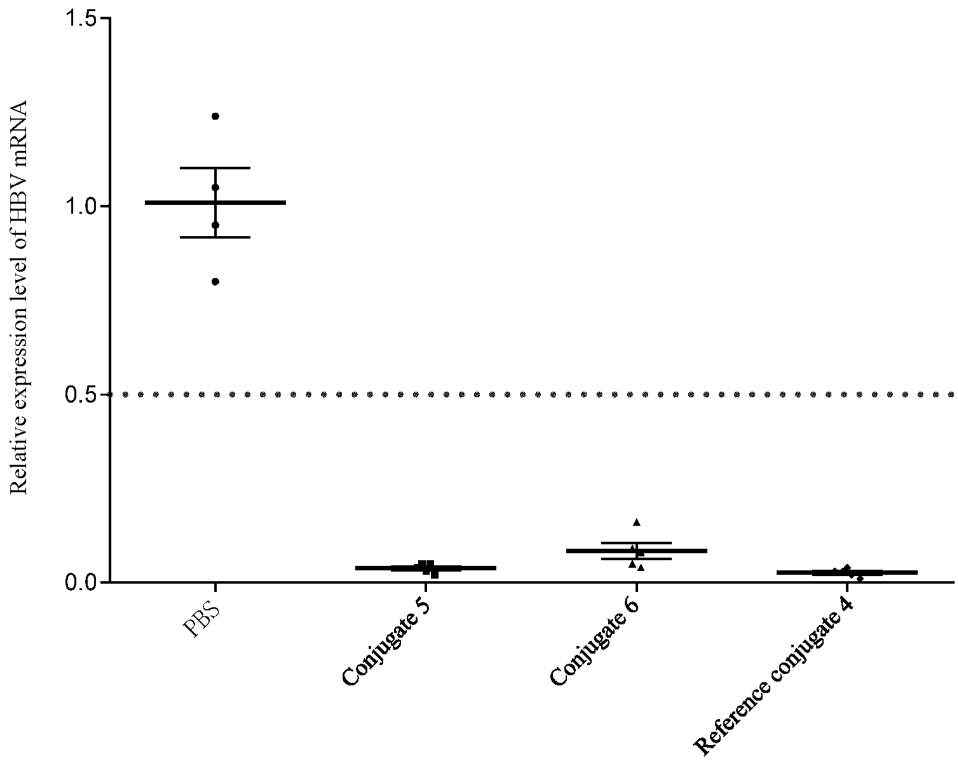
1.5
1.0
0.5
0.0
Relative expression level of HBV mRNA
PBS
Conjugate 5
Conjugate 6
Reference conjugate 4

FIG. 3A

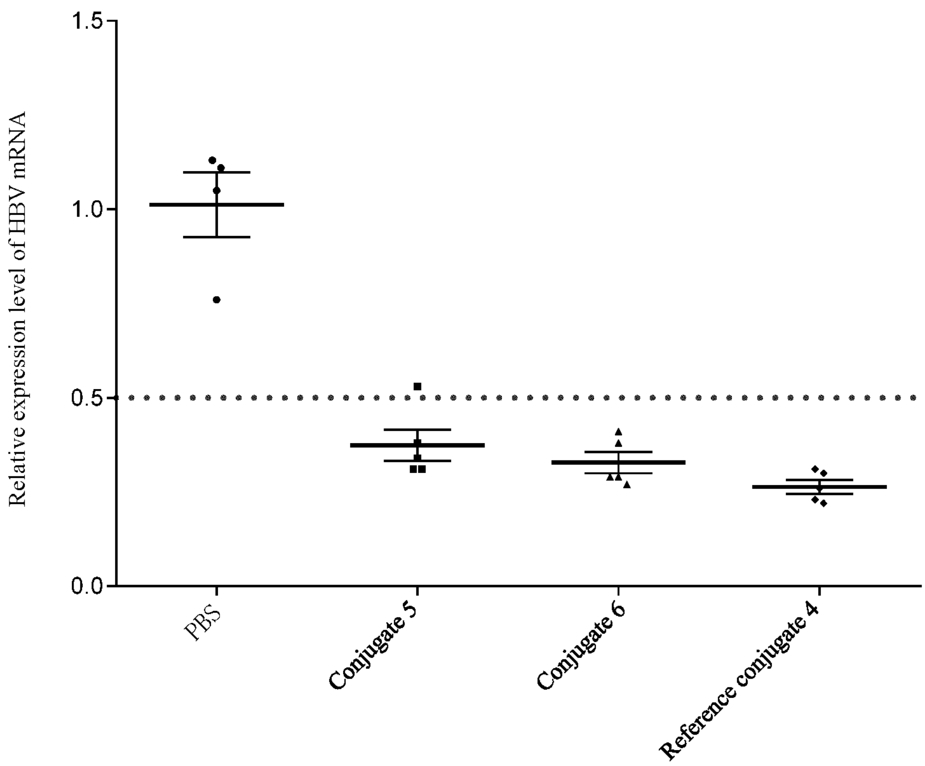
1.5
1.0
0.5
0.0
Relative expression level of HBV mRNA
PBS
Conjugate 5
Conjugate 6
Reference conjugate 4

FIG. 3B

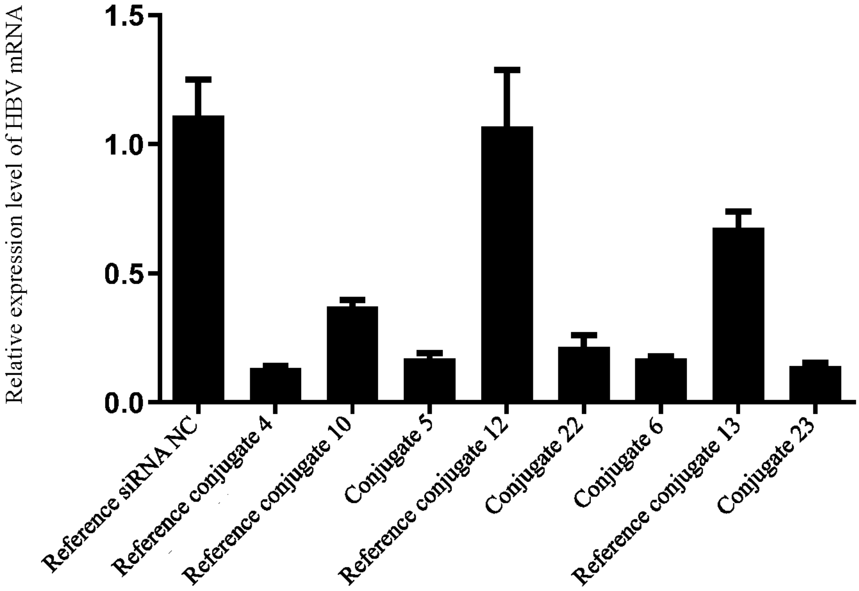
Relative expression level of HBV mRNA
1.5
1.0
0.5
0.0
Reference siRNA NC
Reference conjugate 4
Reference conjugate 10
Conjugate 5
Reference conjugate 12
Conjugate 22
Conjugate 6
Reference conjugate 13
Conjugate 23

FIG. 4

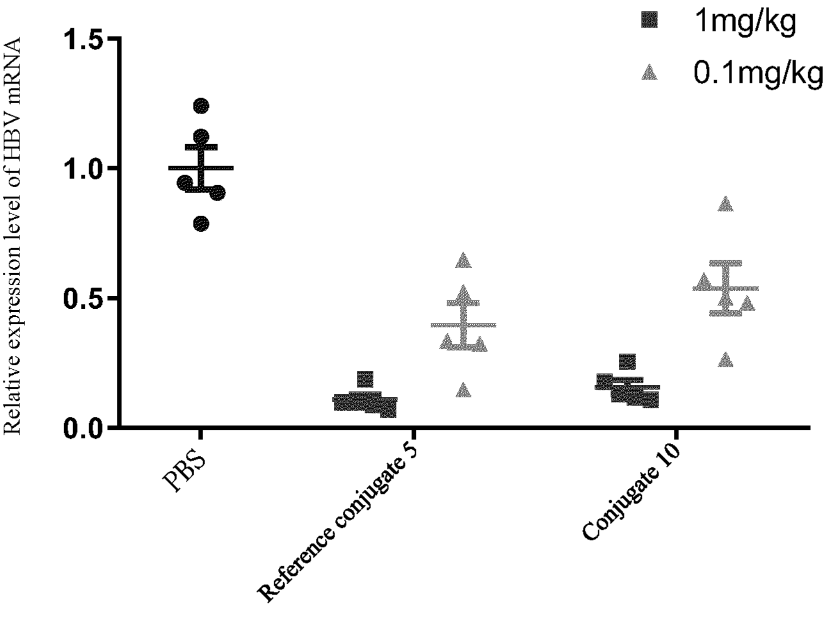
1mg/kg
0.1mg/kg
Relative expression level of HBV mRNA
1.5
1.0
0.5
0.0
PBS
Reference conjugate 5
Conjugate 10

FIG. 5

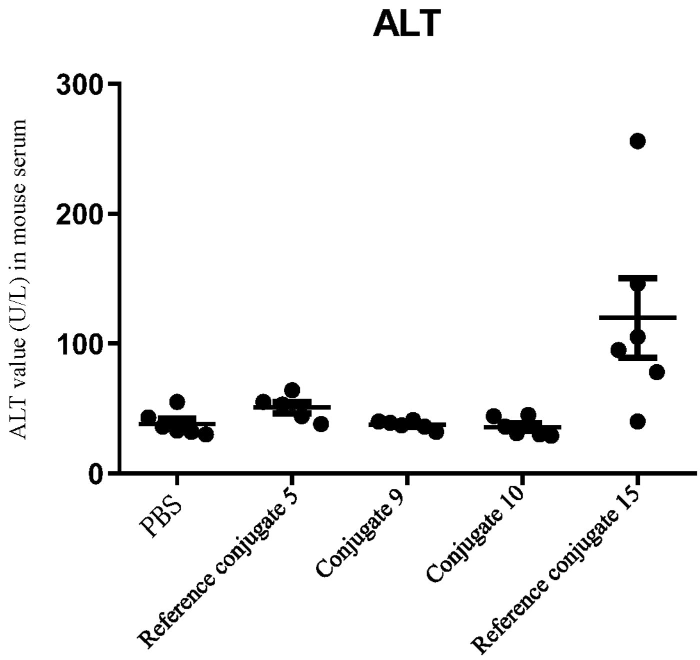
ALT
ALT value (U/L) in mouse serum
300
200
100
0
PBS
Reference conjugate 5
Conjugate 9
Conjugate 10
Reference conjugate 15

FIG. 6A

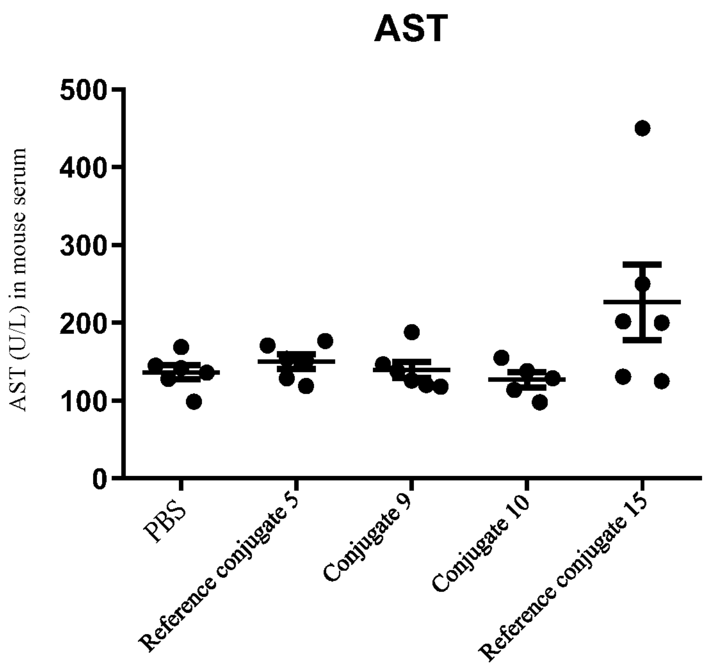
AST
AST (U/L) in mouse serum
500
400
300
200
100
0
PBS
Reference conjugate 5
Conjugate 9
Conjugate 10
Reference conjugate 15

FIG. 6B

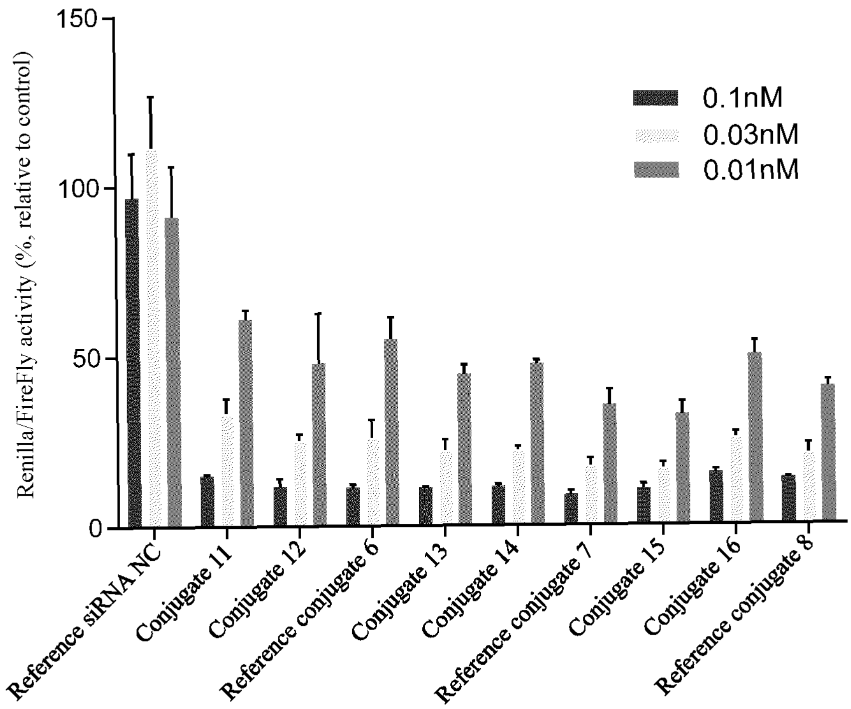
150
100
50
0
Renilla/FireFly activity (%, relative to control)
0.1nM
0.03nM
0.01nM
Reference siRNA NC
Conjugate 11
Conjugate 12
Reference conjugate 6
Conjugate 13
Conjugate 14
Reference conjugate 7
Conjugate 15
Conjugate 16
Reference conjugate 8

FIG. 7

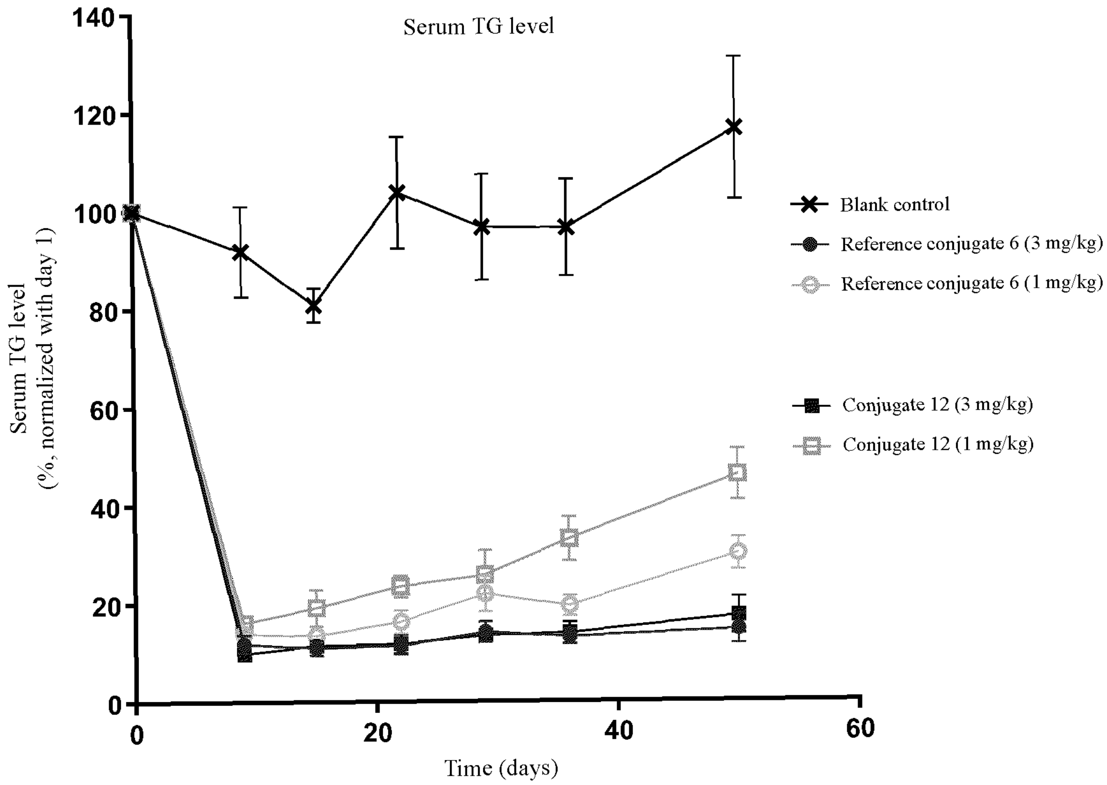
Serum TG level
140
120
100
80
60
40
20
0
Serum TG level
(%, normalized with day 1)
0
20
40
60
Time (days)
Blank control
Reference conjugate 6 (3 mg/kg)
Reference conjugate 6 (1 mg/kg)
Conjugate 12 (3 mg/kg)
Conjugate 12 (1 mg/kg)

FIG. 8A

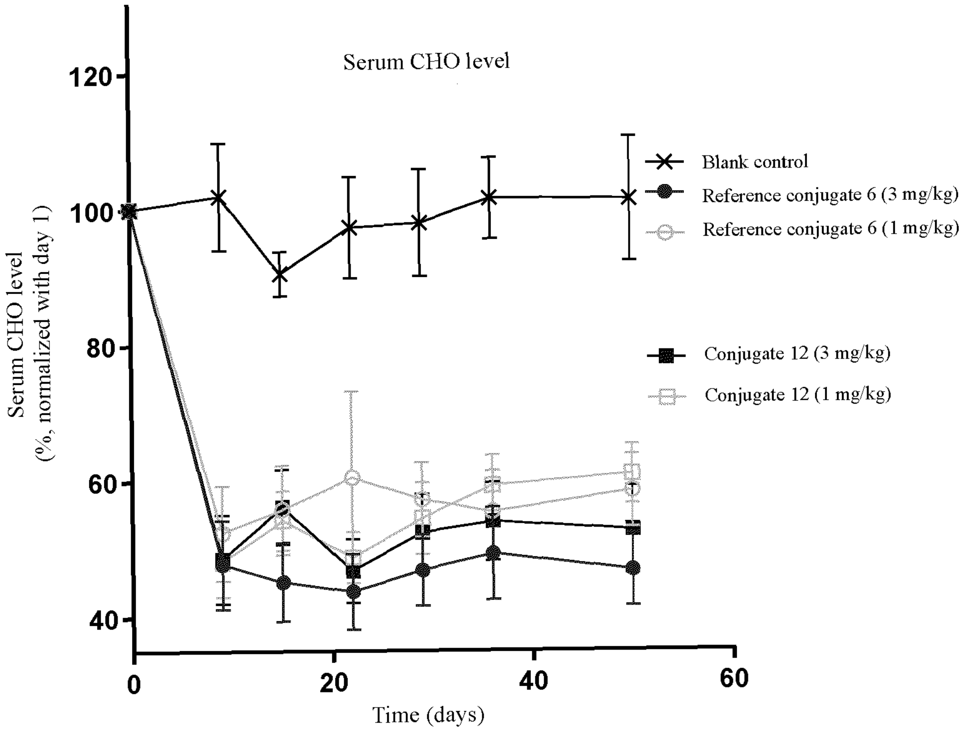
Serum CHO level
120
100
80
60
40
0
20
40
60
Serum CHO level
(%, normalized with day 1)
Time (days)
Blank control
Reference conjugate 6 (3 mg/kg)
Reference conjugate 6 (1 mg/kg)
Conjugate 12 (3 mg/kg)
Conjugate 12 (1 mg/kg)

FIG. 8B

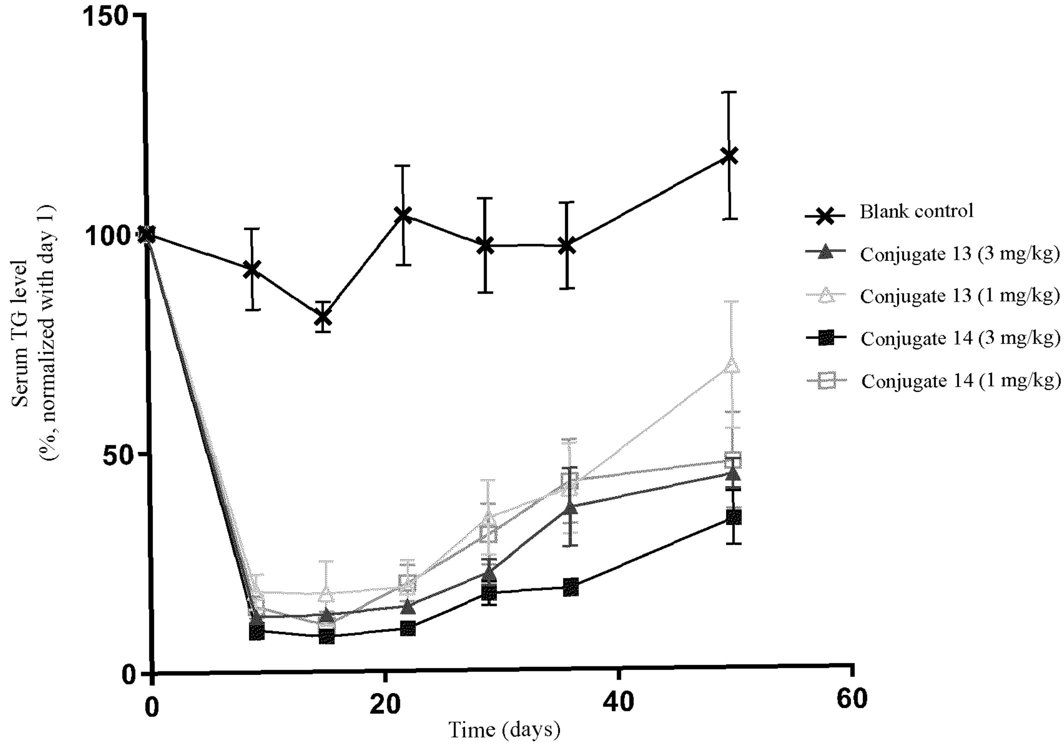
150
100
50
0
0
20
40
60
Serum TG level
(%, normalized with day 1)
Time (days)
Blank control
Conjugate 13 (3 mg/kg)
Conjugate 13 (1 mg/kg)
Conjugate 14 (3 mg/kg)
Conjugate 14 (1 mg/kg)

FIG. 9A

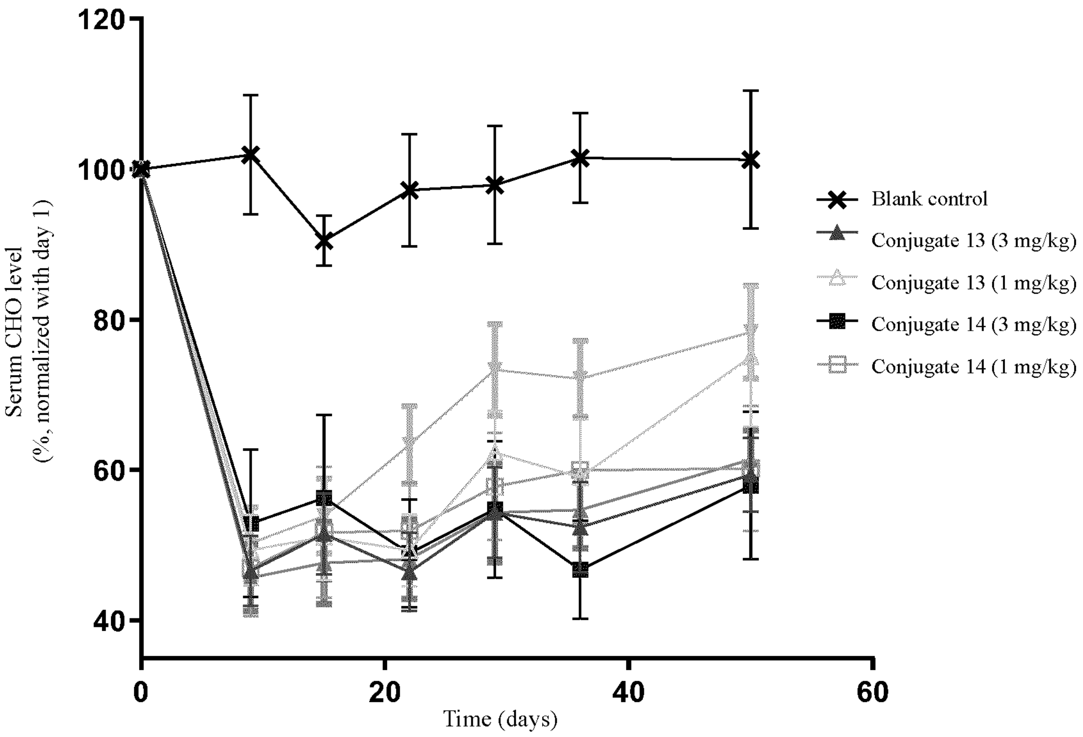
120
100
80
60
40
0
20
40
60
Serum CHO level
(%, normalized with day 1)
Time (days)
Blank control
Conjugate 13 (3 mg/kg)
Conjugate 13 (1 mg/kg)
Conjugate 14 (3 mg/kg)
Conjugate 14 (1 mg/kg)

FIG. 9B

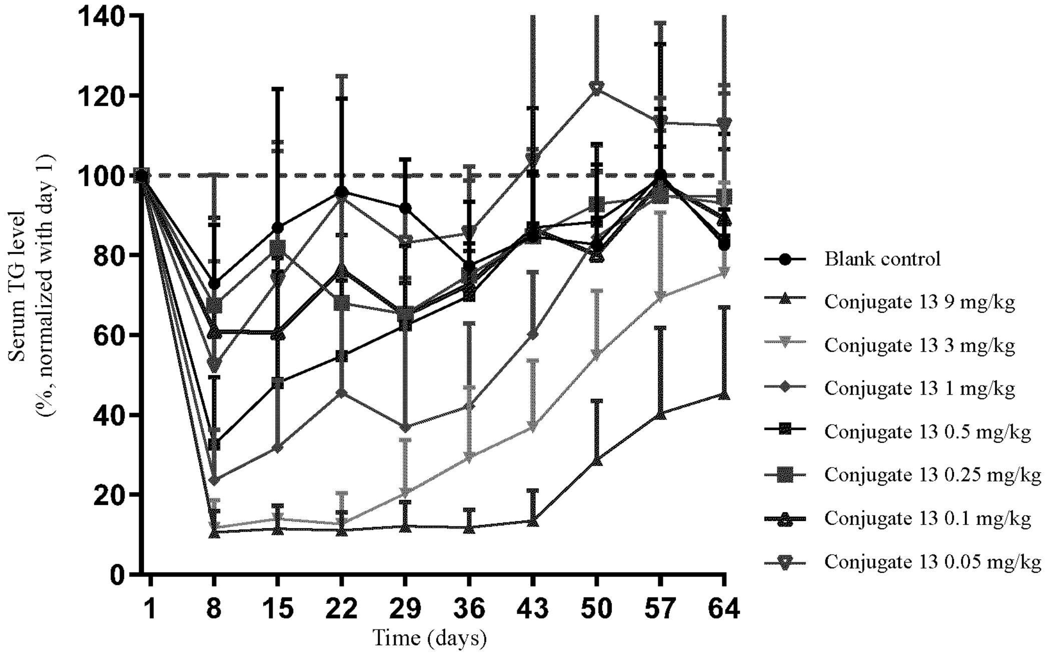
140
120
100
80
60
40
20
0
1
8
15
22
29
36
43
50
57
64
Serum TG level
(%, normalized with day 1)
Time (days)
Blank control
Conjugate 13 9 mg/kg
Conjugate 13 3 mg/kg
Conjugate 13 1 mg/kg
Conjugate 13 0.5 mg/kg
Conjugate 13 0.25 mg/kg
Conjugate 13 0.1 mg/kg
Conjugate 13 0.05 mg/kg

FIG. 10

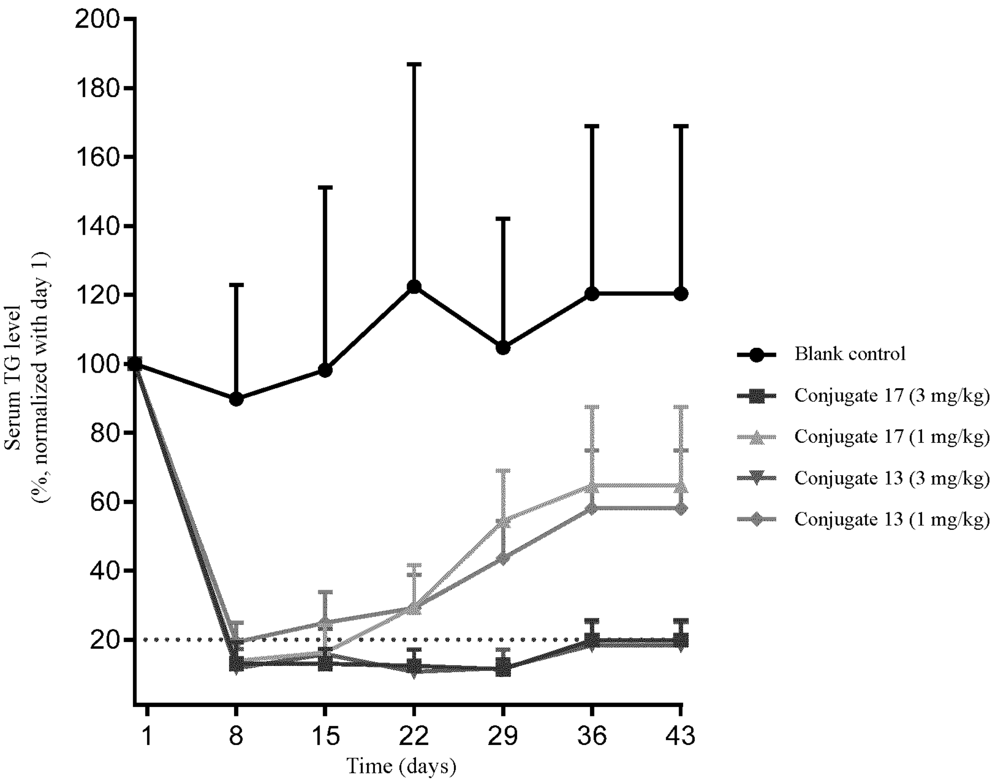


FIG. 11A

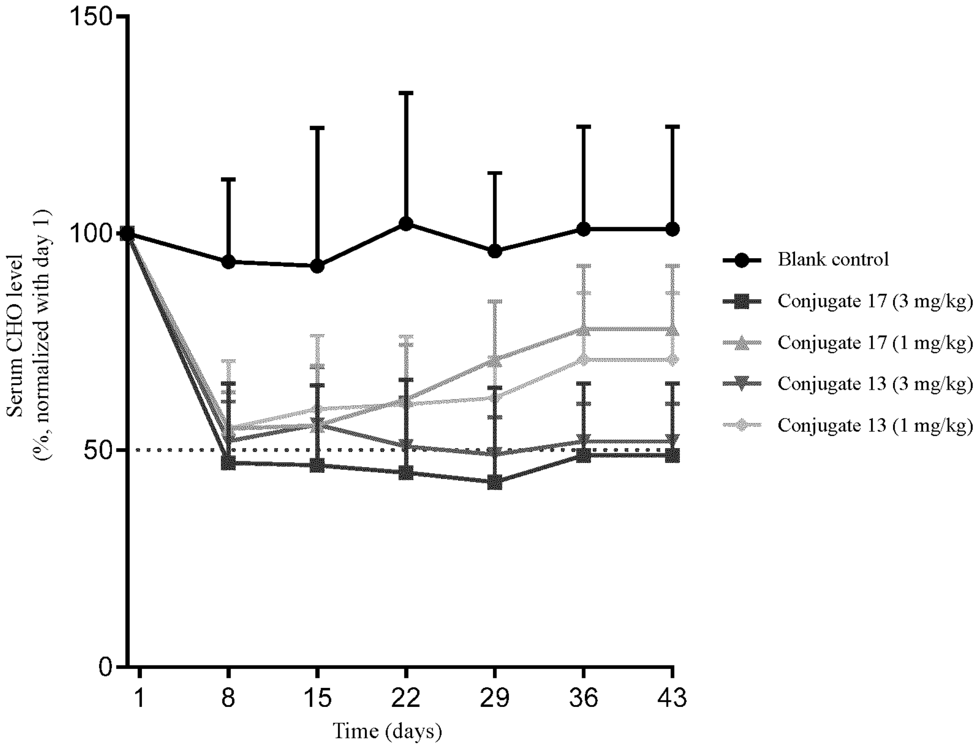


FIG. 11B

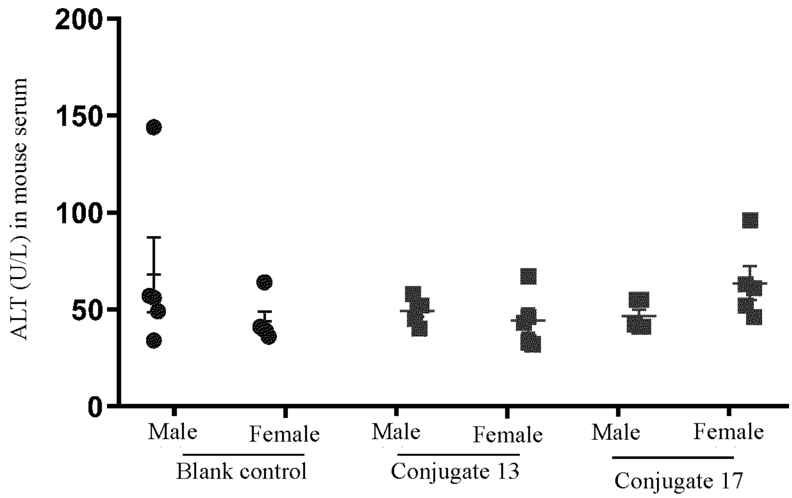


FIG. 12A

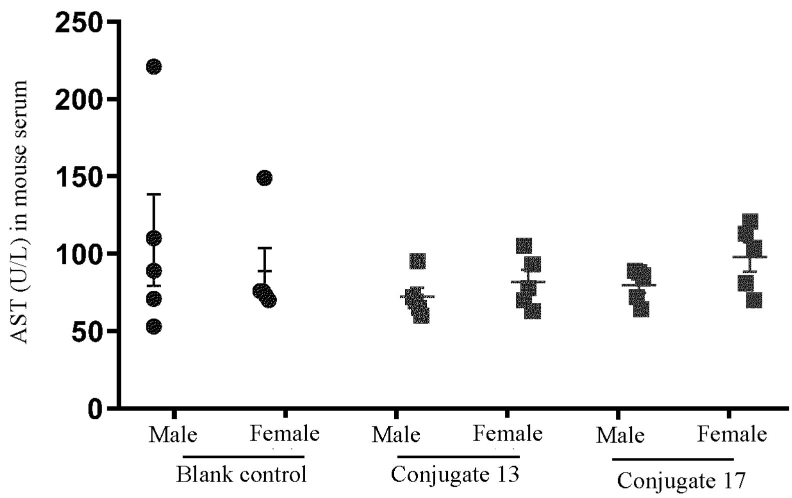


FIG. 12B

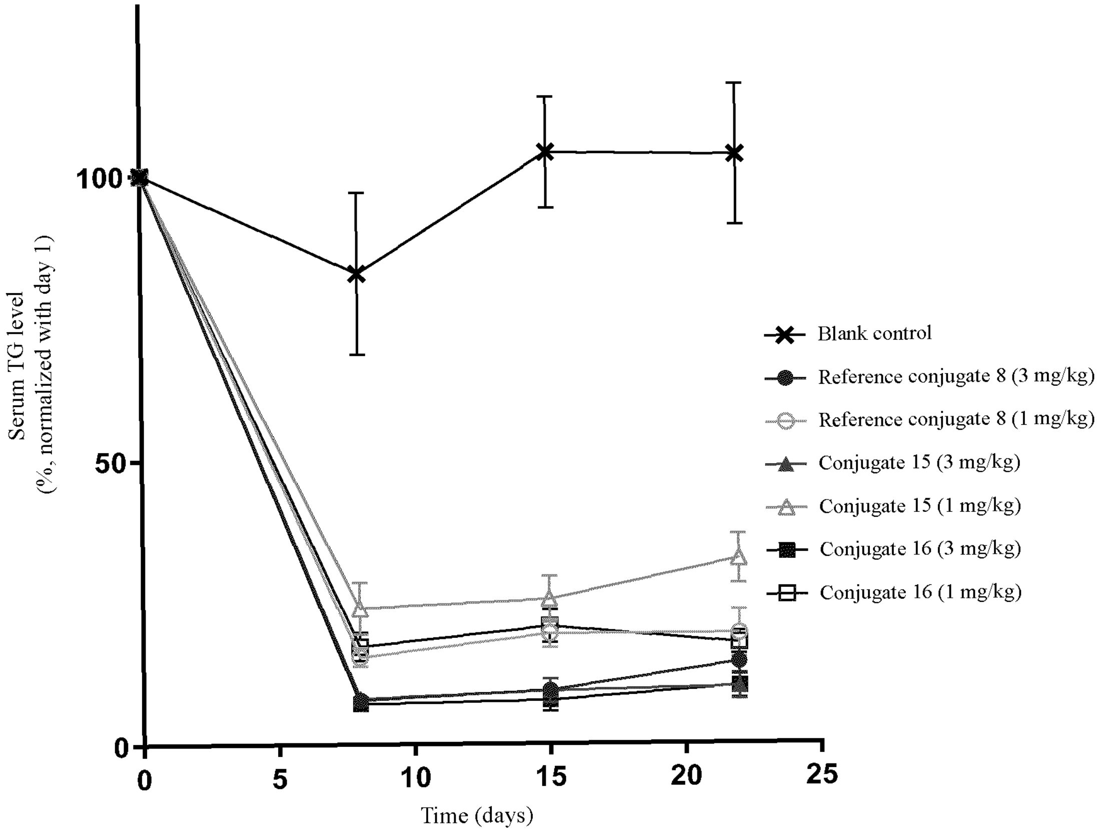


FIG. 13A

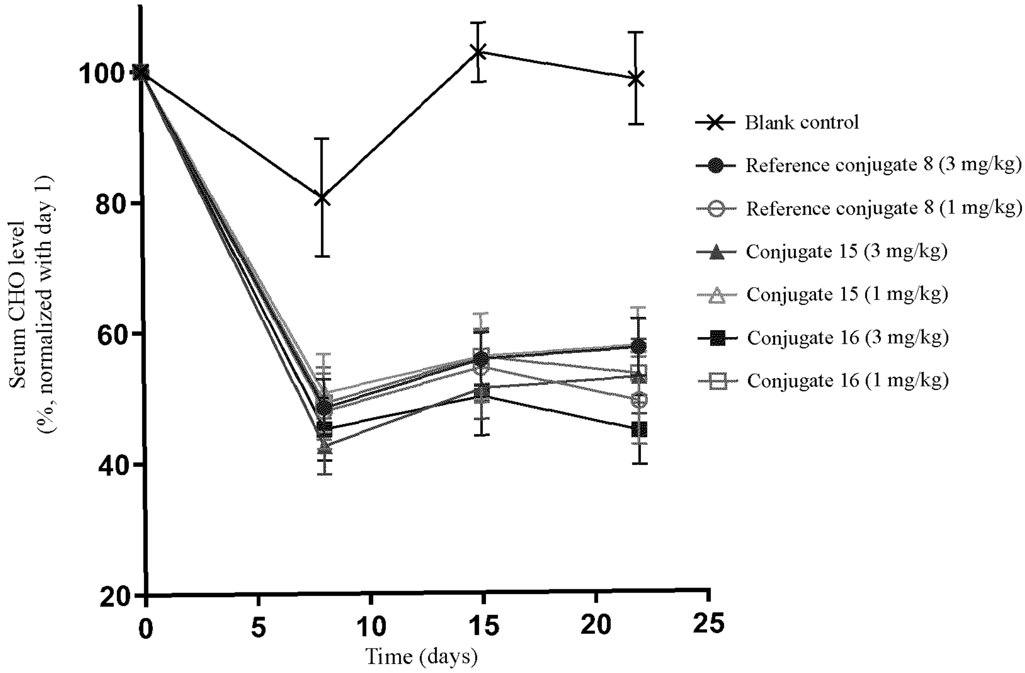


FIG. 13B

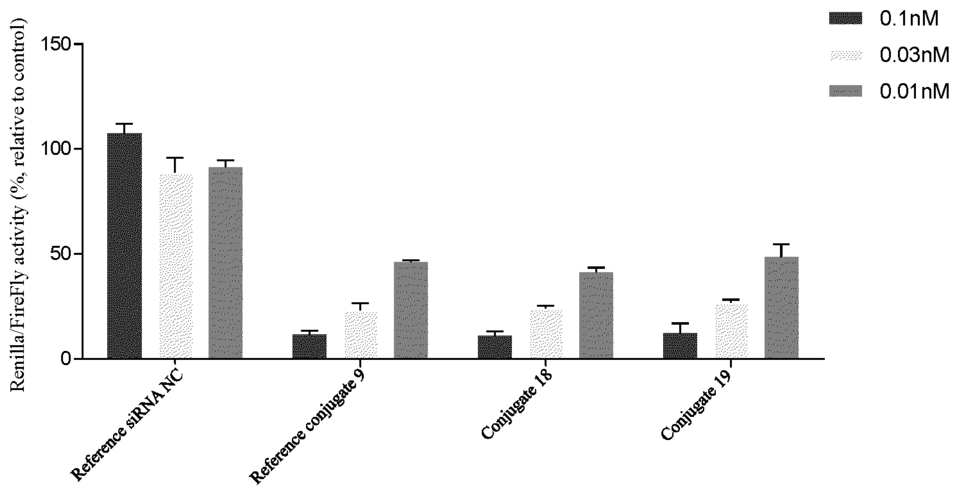

Renilla/FireFly activity (%, relative to control)
150
100
50
0
0.1nM
0.03nM
0.01nM
Reference siRNA NC
Reference conjugate 9
Conjugate 18
Conjugate 19


FIG. 14

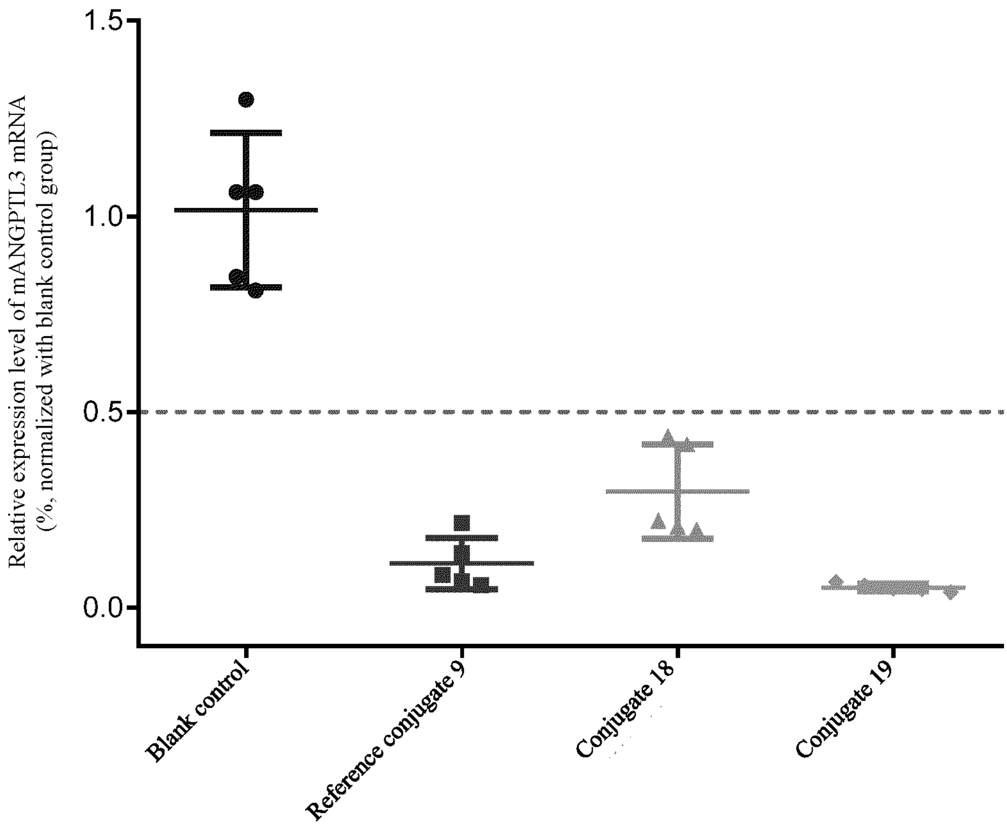

Relative expression level of mANGPTL3 mRNA
(%, normalized with blank control group)
1.5
1.0
0.5
0.0
Blank control
Reference conjugate 9
Conjugate 18
Conjugate 19


FIG. 15

## INTERNATIONAL SEARCH REPORT

International application No.
**PCT/CN2022/105340**

**A. CLASSIFICATION OF SUBJECT MATTER**

C12N 15/113(2010.01)i; A61K 48/00(2006.01)i; A61P 31/00(2006.01)i; A61P 9/00(2006.01)i

According to International Patent Classification (IPC) or to both national classification and IPC

**B. FIELDS SEARCHED**

Minimum documentation searched (classification system followed by classification symbols)

C12N; A61K; A61P

Documentation searched other than minimum documentation to the extent that such documents are included in the fields searched

Electronic data base consulted during the international search (name of data base and, where practicable, search terms used)

CNTXT; ENTXT; ENTXTC; DWPI; CNKI; BAIDU; ISI WEB OF SCIENCES; PUBMED: 甲氧基, 甲氧基乙基, 小干扰RNA, 2'-O-甲基, 2'-氟, 双链寡核苷酸, 稳定性, 脱靶, siRNA, saRNA, 2'-OME, MEO, 2'-F, double stranded nucleic acid, stability, off target

**C. DOCUMENTS CONSIDERED TO BE RELEVANT**

| Category* | Citation of document, with indication, where appropriate, of the relevant passages | Relevant to claim No. |
|---|---|---|
| X | CN 110997919 A (SUZHOU RIBO LIFE SCIENCE CO., LTD.) 10 April 2020 (2020-04-10) description, pages 1 and 6-57 | 1-30, 32-62 |
| Y | CN 110997919 A (SUZHOU RIBO LIFE SCIENCE CO., LTD.) 10 April 2020 (2020-04-10) description, pages 1 and 6-57 | 31, 49-62 |
| Y | CN 110945132 A (SUZHOU RIBO LIFE SCIENCE CO., LTD.) 31 March 2020 (2020-03-31) description, pages 12-14 | 31, 49-62 |
| Y | CN 110944675 A (SUZHOU RIBO LIFE SCIENCE CO., LTD.) 31 March 2020 (2020-03-31) description, pages 10-11 | 31, 49-62 |
| Y | CN 112423794 A (SUZHOU RIBO LIFE SCIENCE CO., LTD.) 26 February 2021 (2021-02-26) description, pages 1-2 | 31, 49-62 |
| Y | CN 110997917 A (SUZHOU RIBO LIFE SCIENCE CO., LTD.) 10 April 2020 (2020-04-10) description, pages 19-20 | 31, 49-62 |
| Y | CN 110945130 A (SUZHOU RIBO LIFE SCIENCE CO., LTD.) 31 March 2020 (2020-03-31) description, pages 13-14 | 31, 49-62 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| | **10 October 2022** |
| Name and mailing address of the ISA/CN<br>**China National Intellectual Property Administration (ISA/CN)**<br>**No. 6, Xitucheng Road, Jimenqiao, Haidian District, Beijing 100088, China** | Authorized officer |
| Facsimile No. **(86-10)62019451** | Telephone No. |

Form PCT/ISA/210 (second sheet) (January 2015)

**INTERNATIONAL SEARCH REPORT**

International application No.
**PCT/CN2022/105340**

**C. DOCUMENTS CONSIDERED TO BE RELEVANT**

| Category* | Citation of document, with indication, where appropriate, of the relevant passages | Relevant to claim No. |
|---|---|---|
| A | CN 102124107 A (SIRNA THERAPEUTICS INC) 13 July 2011 (2011-07-13)<br>entire document | 1-62 |
| A | US 2017081667 A1 (KYOWA HAKKO KIRIN CO., LTD.) 23 March 2017 (2017-03-23)<br>entire document | 1-62 |
| A | US 2010069461 A1 (ALNYLAM PHARMACEUTICALS, INC.) 18 March 2010 (2010-03-18)<br>entire document | 1-62 |
| A | SONG, X. Y. et al. "Site-Specific Modification Using the 20-Methoxyethyl Group Improves the Specificity and Activity of siRNAs"<br>*Molecular Therapy: Nucleic Acids,* Vol. 9, 31 December 2017 (2017-12-31),<br>pages 242-250 | 1-62 |

Form PCT/ISA/210 (second sheet) (January 2015)

**INTERNATIONAL SEARCH REPORT**

International application No.
**PCT/CN2022/105340**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**

International application No.
**PCT/CN2022/105340**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **55-58**
because they relate to subject matter not required to be searched by this Authority, namely:

[1] Claims 55-58 relate to a method for treating and/or preventing, using double-stranded oligonucleotides, diseases or symptoms related to the mRNA expression level of a target gene, which belongs to the subject matter defined in PCT Rule 39.1(iv) that does not warrant a search. The authority conducted a search on the basis of an application of double-stranded oligonucleotides in the preparation of drugs for treating and/or preventing diseases or symptoms related to the mRNA expression level of a target gene.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2022/105340**

| Patent document cited in search report | Publication date (day/month/year) | Patent family member(s) | Publication date (day/month/year) |
|---|---|---|---|
| CN 110997919 A | 10 April 2020 | WO 2019105418 A1 | 06 June 2019 |
| | | KR 20200091414 A | 30 July 2020 |
| | | EP 3719128 A1 | 07 October 2020 |
| | | CA 3083968 A1 | 06 June 2019 |
| | | TW 201925469 A | 01 July 2019 |
| | | US 2022049249 A1 | 17 February 2022 |
| | | JP 2021504415 A | 15 February 2021 |
| | | AU 2018374219 A1 | 21 May 2020 |
| CN 110945132 A | 31 March 2020 | AU 2018377716 A1 | 09 April 2020 |
| | | JP 2021503930 A | 15 February 2021 |
| | | RU 2020118025 A | 04 January 2022 |
| | | CA 3083970 A1 | 06 June 2019 |
| | | TW 201928057 A | 16 July 2019 |
| | | KR 20200095483 A | 10 August 2020 |
| | | WO 2019105437 A1 | 06 June 2019 |
| | | EP 3719125 A1 | 07 October 2020 |
| CN 110944675 A | 31 March 2020 | WO 2019105435 A1 | 06 June 2019 |
| | | TW 201925468 A | 01 July 2019 |
| | | US 2021277400 A1 | 09 September 2021 |
| | | EP 3718572 A1 | 07 October 2020 |
| | | JP 2021503941 A | 15 February 2021 |
| CN 112423794 A | 26 February 2021 | None | |
| CN 110997917 A | 10 April 2020 | US 2020360522 A1 | 19 November 2020 |
| | | TW 201925471 A | 01 July 2019 |
| | | EP 3719127 A1 | 07 October 2020 |
| | | WO 2019105419 A1 | 06 June 2019 |
| | | JP 2021503929 A | 15 February 2021 |
| CN 110945130 A | 31 March 2020 | US 2021032623 A1 | 04 February 2021 |
| | | WO 2019105414 A1 | 06 June 2019 |
| | | JP 2021503940 A | 15 February 2021 |
| | | EP 3719126 A1 | 07 October 2020 |
| | | TW 201925470 A | 01 July 2019 |
| CN 102124107 A | 13 July 2011 | EP 2052079 A2 | 29 April 2009 |
| | | JP 2010503382 A | 04 February 2010 |
| | | WO 2008011431 A2 | 24 January 2008 |
| | | AU 2007275365 A1 | 24 January 2008 |
| | | CA 2658183 A1 | 24 January 2008 |
| US 2017081667 A1 | 23 March 2017 | EP 3118315 A1 | 18 January 2017 |
| | | JP WO2015137459 A1 | 06 April 2017 |
| | | WO 2015137459 A1 | 17 September 2015 |
| US 2010069461 A1 | 18 March 2010 | US 2018127749 A1 | 10 May 2018 |
| | | US 2017121709 A1 | 04 May 2017 |
| | | US 2014206747 A1 | 24 July 2014 |
| | | AT 510545 T | 15 June 2011 |
| | | CA 2626690 A1 | 18 May 2007 |
| | | WO 2007056331 A2 | 18 May 2007 |
| | | AU 2006311730 A1 | 18 May 2007 |
| | | US 2020231968 A1 | 23 July 2020 |
| | | US 2021189393 A1 | 24 June 2021 |

Form PCT/ISA/210 (patent family annex) (January 2015)

**INTERNATIONAL SEARCH REPORT**
**Information on patent family members**

International application No.
**PCT/CN2022/105340**

| Patent document cited in search report | Publication date (day/month/year) | Patent family member(s) | | | Publication date (day/month/year) |
|---|---|---|---|---|---|
| | | AU | 2011200894 | A1 | 24 March 2011 |
| | | EP | 1945270 | A2 | 23 July 2008 |
| | | US | 2011130443 | A1 | 02 June 2011 |
| | | EP | 2363134 | A1 | 07 September 2011 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 102140458 B
- CN 103380113 A
- WO 2015006740 A2
- WO 2009082607 A2
- WO 2014025805 A1
- CN 110959011 A
- WO 2019105418 A
- WO 2019105418 A1


### Non-patent literature cited in the description


- **BEAUCAGE et al.** *Tetrahedron,* 1992, vol. 48, 2223-2311
- **GREENE ; WUTS.** Protective Groups in Organic Synthesis. John Wiley & Sons, 1991
- **J. K. WATTS et al.** Chemically modified siRNA: tools and applications. *Drug Discov Today,* 2008, vol. 13 (19-20), 842-55
- *Nature Biotechnology,* 2017, vol. 35 (3), 238-48
- *ACS Chemical biology,* 2015, vol. 10 (5), 1181-7
- **RAJEEV et al.** *ChemBioChem,* 2015, vol. 16, 903-908
- Molecular Cloning. Cold Spring Harbor LBboratory Press, 1989
- **KUMICO UI-TEI et al.** Functional dissection of siRNA sequence by systematic DNA substitution: modified siRNA with a DNA seed arm is a powerful tool for mammalian gene silencing with significantly reduced off-target effect. *Nucleic Acids Research,* 2008, vol. 36 (7), 2136-2151